# EUROPEAN PATENT APPLICATION

(11) **EP 1 707 204 A1**
(43) Date of publication of application: **04.10.2006**
(21) Application number: 05290728.4
(22) Date of filing: 01.04.2005
(51) Int. Cl.: A61K 31/4453, A61P 25/08

(54) **Treatment of epilepsy with non-imidazole alkylamines histamine H3-receptor ligands**

(71) Applicant: BIOPROJET, 75003 Paris (FR)
(72) Inventor: Schwartz, Jean-Charles, 75014 Paris (FR); Lecomte, Jeanne-Marie, 75003 Paris (FR)
(74) Representative: Colombet, Alain André

(57) **Abstract**

The present invention provides new method of treatment of epilepsy with non-imidazole alkylamine derivatives that constitute antagonists of the H₃-receptors of histamine.

## Description

The present invention relates to the therapeutical application of alkylamines of formula (A) as defined hereafter for the treatment of epilepsy.

Antagonists of histamine H₃-receptor are known especially to increase synthesis and release of cerebral histamine. Through this mechanism, they induce an extended wakefullness, an improvement in cognitive processes, a reduction in food intake and a normalization of vestibular reflexes (Schwartz et al., Physiol. Rev., 1991, 71: 1-51).

Histamine H₃-receptor agonists are known to inhibit the release of several neurotransmitters including histamine, monoamines and neuropeptides and thereby exert sedative and sleep-promoting effects in brain. In peripheral tissues, H₃-receptor agonists exert namely anti-inflammatory, anti-nociceptive, gastro-intestinal, antisecretory smooth muscle decontracting activities.

H₃ receptor antagonist or agonist compounds previously known resemble histamine in possessing an imidazole ring generally monosubstituted in 4(5)-position (Ganellin et al., Ars Pharmaceutica, 1995, 36:3, 455-468; Stark et al., Drug of the Future, 1996, 21 (5), 507-520).

Numerous patents and patent applications are directed to antagonist and/or agonist compounds having such structure, in particular EP 197 840, EP 494 010, WO 93/14070, WO 96/29315, WO 92/15 567, WO 93/20061, WO 93/20062, WO 95/11894, US 5 486 526, WO 93/12107, WO 93/12108, WO 95/14007, WO 95/06037, WO 97/29092, EP 680 960, WO 96/38141, WO 96/38142, WO 96/40126.

In the litterature, Plazzi et al., Eur. J. Med. Chem. 1995, 30, 881, Clitherow et al., Bioorg. & Med. Chem. Lett. 6 (7), 833-838 (1996) Wolin et al., Bioorg. & Med. Chem. Lett; 8, 2157 (1998) can be cited also in this respect.

Nevertheless, such imidazole derivatives may show drawbacks such as poor blood-brain barrier penetration, interaction with cytochrome P-450 proteins and/or some hepatic and ocular toxicities.

Non-imidazole known neuro-active compounds such as betahistine (J-M. Arrang et al., Eur. J. Pharmacol. 1985, 111: 72-84), phencyclidine (J-M. Arrang et al., Eur. J. Pharmacol. 1988, 157: 31-35), dimaprit (J-C Schwartz et al., Agents Actions 1990, 30: 13-23), clozapine (M. Kathmann et al., Psychopharmacology 1994, 116: 464-468), and sesquiterpenes (M. Takigawa et al., JP 06 345 642 (20 Dec 1994)) were suggested to display H₃-receptor antagonism but all these compounds have only very low potency.

These compounds were previously known as therapeutic agent before the discovery and characterization of the histamine H₃-receptor, in particular as neuro-active agents for example as neuroleptic (clozapine) or psychotomimetic (Phencyclidine) agent.

When tested at the H₃-receptor, these compounds were shown to display much lower potency than the imidazole-containing compounds described in patent applications quoted above.

Contrary to previous attempts, the inventors succeeded at developping potent H₃-receptor ligands not containing imidazole ring that reduced the above-mentioned drawbacks. These compounds, their preparation and therapeutical applications thereof have been described in the international patent application WO 00/06254.

The role of brain histamine in convulsive disorders has been evoked for a long time, mainly starting from the observations of seizures as a side-effect of H₁-antihistamines crossing the blood-brain barrier. Another indication was that treatments enhancing brain histamine levels, e.g. administration of L-histidine (the histamine precursor), metoprine (an inhibitor of histamine degradation), or exogenous histamine itself, tend to protect rodents from convulsions (Tuomisto and Tacke Neuropharmacol. 1986, 25, 955-8; Scherkl et al. Epilepsy Res. 1991; 10,111-8) whereas inhibition of histamine synthesis is proconvulsant (Yokoyama et al. Naunyn Schmiedb. Arch. Pharmacol. 1992, 346, 40; CNS Drugs 1996, 5, 321).

However, it remained unclear whether blockade of H₃ receptors could represent a mechanism for a new class of antiepileptic drugs.

Indeed blockade of H₃ auto-receptors enhances histamine release from histaminergic neurons in brain (Arrang et al., Nature 1987, 327, 117) and could thereby protect from convulsions. However, H₃-receptor antagonists may have other actions via H₃ receptors located on other classes of neurons, e.g. catecholaminergic, cholinergic, glutamatergic or peptidergic neurons (Schlicker E et al., Fundam Clin Pharmacol. 1994,8, 128 ). Therefore no one could predict what would be the final outcome of H₃ receptor blockade on convulsions in human patients in which such treatment had never been performed.

Histamine H₃-receptor blockade by drugs like thioperamide, clobenpropit, and AQ0145, was shown to reduce electrically-driven convulsions (Yokoyama et al., ibid) and to prevent pentetrazole-induced convulsions in mice and rats (Zhang et al., Eur J Pharmacol. 2003, 482, 169-75 ; Vohora D et al., Life Sci. 2000, 66, 297-301). However in another study (Scherkl et al., ibid) thioperamide failed to affect seizure susceptibility in mice.

The inventors also assessed the effects of one of the non-imidazole compound they described in WO 00/06254, BF2-649 (3-(4-chlorophenyl)propyl 3-piperidinopropyl ether) at 10 mg/kg on clonic convulsions induced by pentetrazole in mice. This H₃-receptor antagonist was found ineffective in preventing the convulsion (in terms of either latency or duration) and, furthermore, it did not modify (enhance) the anticonvulsive properties of a series of established antiepileptic drugs on this model: carbamazepine (25 mg/kg), sodium valproate (300 mg/kg), phenytoin (25 mg/kg), diazepam (7.5 mg/kg) and Phenobarbital (15 mg/kg).

Finally, in other animal models, probably more relevant to the pathogeny of human epilepsy, i.e. amygdaloid-kindled seizures in rats, thioperamide and various other H₃-receptor antagonists were found ineffective (Yoshida et al., Epilepsy Res. 2000, 40 141-5).

Hence, in view of these conflicting data, it did not appear that histamine H₃-receptor antagonists might represent a new class of anti-epileptic drugs in human pathologic states.

The inventors have now demonstrated that these alkylamines of formula (A), as described below, constitute efficient anti-epileptic drugs.

### Alkylamine histamine H₃-receptor antagonists

Compounds, the structure of which does not contain an imidazole moiety, which are useful as histamine H₃-receptor ligands, are herein described.

These compounds have the following general formula (A): 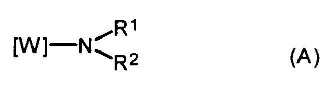 in which:
- W is a residue which imparts antagonistic and/or agonistic activity at histamine H₃-receptors when attached to an imidazole ring in 4(5)-position;
- R¹ and R² may be identical or different and represent each independently
   - a lower alkyl or cycloalkyl,
      or taken together with the nitrogen atom to which they are attached,
   - a saturated nitrogen-containing ring 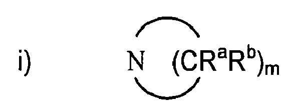 with m ranging from 2 to 8, or
   - a non-aromatic unsaturated nitrogen-containing ring 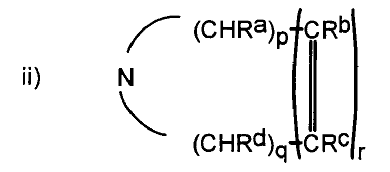 with p and q being from 0 to 3 independently and r being from 0 to 4, provided that p and q are not simulteously 0 and 2 ≤ p + q + r ≤ 8,
      R^{a-d} being independently a hydrogen atom or a lower alkyl, cycloalkyl, or carboalkoxy group, or
   - a morpholino group, or
   - a N-substituted piperazino group: 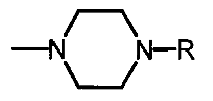 with R being a lower alkyl, cycloalkyl, carboalkoxy, aryl, arylalkyl, an alkanoyl or aroyl group.

Addition salts which the compounds form with pharmaceutically acceptable acids are also described. The pharmaceutically acceptable salts comprise the nontoxic salt of inorganic or organic acids. Examples of these salts include the hydrochloride, the hydrobromide or the hydrogen maleate or hydrogen oxalate.

The present application also describes the hydrates of the compounds, the hydrated salts of these compounds and the polymorphic crystalline structures.

When the compounds can exist in one or a number of isomeric forms according to the number of asymmetric centres in the molecule, the invention relates both to all the optical isomers and to their racemic modifications and the corresponding diastereoisomers. The separation of the diastereoisomers and/or of the optical isomers can be carried out according to methods known per se.

The present application also describes all the possible tautomeric forms of the compounds, whether these tautomers occur in isolated form or in the form of mixtures.

"Lower alkyl" or "cycloalkyl" is intended to mean a linear or branched alkyl group containing from 1 to 6 carbon atoms, or a saturated carbocycle containing 3 to 6 carbon atoms.

Typically examples of lower alkyl are methyl, ethyl, propyl, isopropyl and butyl groups.

A preferred group of compounds comprises those with R¹ and R² representing independently a lower alkyl group, especially an ethyl group.

Preferred compounds are also those of formula (A) in which R¹ and R² taken together with the nitrogen atom to which they are attached, form a saturated nitrogen-containing ring: 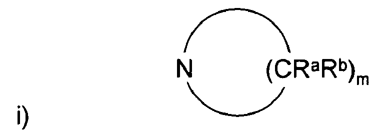 especially with m being 4, 5 or 6, optionally substituted with an alkyl group (R^{a}), preferably a methyl group.

The groups R^{a} and R^{b} are identical or different for each (CR^{a}R^{b}) moiety.

Piperidyl and pyrrolidinyl moieties are especially preferred.

Another preferred group of compounds comprises compounds (A) in which R¹ and R² taken together with the nitrogen atom to which they are attached, form a non-aromatic unsaturated nitrogen-containing ring: 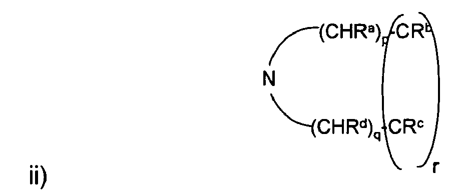 especially with p, q, and r being independently 1 or 2.

In this group, more preferred compounds are those with p being 2 and q and r each being 1.

A sub-class in this group comprises compounds with R^{a-d} being each a hydrogen atom.

When NR¹R² is a nitrogen-containing ring i) or ii) as above-defined, the latter is preferably substituted with one or two lower alkyl group(s), especially a methyl group.

The position for substitution is preferably selected according the following order:

meta>para>ortho.

In this group, for nitrogen-containing ring bearing only one substituent, this latter is preferably in meta position with respect to the nitrogen-atom.

For nitrogen-containing ring bearing two substituents, meta-meta substitution is preferred, especially when these two substituents are in trans-relation.

Piperidyl or pyrrolidinyl moiety substituted in meta or meta-meta position, especially with a methyl group, give particularly preferred compounds.

When NR¹R² represents a N-substituted piperazino group, R may be a lower alkyl e.g. methyl.

Typical examples of group R being an aryl or arylalkyl moiety are phenyl and benzyl.

R may be also an alkanoyl or aroyl group e.g. acetyl or benzoyl.

In all the possible groups for R, the alkyl moiety refers to a linear or branched chain containing from 1 to 6 carbon atoms.

The cycloalkyl group refers to a saturated carbocycle containing 3 to 7 carbon atoms.

When R represents an aryl or arylalkyl group, the aryl moiety is especially a phenyl group optionally substituted with one or more substituents selected from halogen atoms, advantageously selected from fluorine, chlorine and bromine, or a lower alkyl or cycloalkyl, a trifluoromethyl, aryl, alkoxy, aryloxy, nitro, formyl, alkanoyl, aroyl, arylalkanoyl, amino, carboxamido, cyano, alkyloximino, aryloximino, α-hydroxyalkyl, alkenyl, alkynyl, sulphamido, sulfamoyl, carboxamide, carboalkoxy, arylalkyl or oxime group.

R may be also an optionally substituted benzoyl, the substituent being as defined above with reference to the phenyl group.

Typical example of -NR¹R² representing a N-substituted piperazino group is N-acetylpiperazino.

According to one aspect, the compounds have the following general formula (I): 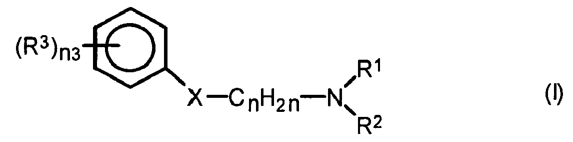 in which:
- CₙH₂n is a linear or branched hydrocarbon chain with n ranging from 2 to 8;
- X is an oxygen or sulfur atom;
- n₃ is an integer from 0 to 5;
- R³ represents each independently
   - a halogen atom,
   - a lower alkyl or cycloalkyl, a trifluoromethyl, aryl, alkoxy, α-alkyloxyalkyl, aryloxy, nitro, formyl, alkanoyl, aroyl, arylalkanoyl, amino, carboxamido, cyano, alkyloximino, alkylalkoximino, aryloximino, α-hydroxyalkyl, alkenyl, alkynyl, sulphamido, sulfamoyl, sulphonamido, carboxamide, carbonylcycloalkyl, alkylcarbonylalkyl, carboalkoxy, arylalkyl or oxime group,
   - or taken together with the carbon atoms of the phenyl ring to which it is fused, a 5- or 6-membered saturated or unsaturated ring or a benzene ring.
- R¹ and R² are as above-defined in formula (A).

A preferred group of compounds is the group composed of compounds of formula (I) in which X is an oxygen atom.

Another preferred group of compounds comprises compounds (I) in which -CₙH₂ₙ- is a linear chain -(CH₂)ₙ- with n being as previously defined.

Preferred compounds are also those with n varying from 3 to 5, and with n being more preferably 3.

A sub-class of compounds according to the invention comprises the compounds of formula (I) with n₃ being zero that is those having an unsubstituted phenyl moiety.

Another group of compounds is composed of compounds containing one or more substituents R³ which may be identical or different. In this group, the compounds having a mono- or di-substituted (n₃ = 1 or 2) phenyl moiety are preferred and those mono-substituted with one group R³ as defined above in para-position are particularly preferred.

Among these compounds, (n₃ being 1) R³ is preferably a halogen atom or a cyano, nitro, alkanoyl, alkyloximino or α-hydroxyalkyl group.

Still more preferred compounds are those with R³ being CN, NO₂, COCH₃, COC₂H₅, H₃C-C=N-OH, H₃C-CH-OH and cycloalkyl-CO like cyclopropyl-CO.

R³ being a halogen atom may be advantageously selected from fluorine, chlorine and bromine.

R³ being an aryl group, may be especially a phenyl group.

In the other substituents R³, the aryl moiety is advantageously a phenyl moiety.

R³ being an aryloxy group may be especially a phenoxy group.

According to the invention, alkanoyl is intended to mean a group containing an alkyl moiety as defined above.

Typical examples of R³ being an alkanoyl, aroyl or arylalkanoyl group are acetyl, butyryl and propionyl groups, benzoyl group or phenylacetyl group.

Typical examples of R³ forming together with the carbon atoms of the phenyl ring to which it is fused, a saturated ring leads to 5,6,7,8-tetrahydronaphthyl or forming a benzene ring leads to a naphthyl moiety.

According to the invention, alkenyl or alkynyl group may contain advantageously from 1 to 8 carbon atoms, in particular from 1 to 6 carbon atoms and preferably 1 to 4 carbon atoms.

In carboalkoxy, carboxyamido, carbonylcycloalkyl, alkylcarbonylalkyl, or carboxamide groups, the hydrocarbon chain is saturated, linear or branched and contains an alkyl moiety as defined above.

In alkoxy, alkylalkoximino, alkyloximino, α-alkyloxyalkyl, arylalkyl or α-hydroxyalkyl group, the alkyl moiety is as previously defined also.

Particularly preferred compounds are:
1-(5-phenoxypentyl)-piperidine
1-(5-phenoxypentyl)-pyrrolidine
N-methyl-N-(5-phenoxypentyl)-ethylamine
1-(5-phenoxypentyl)-morpholine
N-(5-phenoxypentyl)-hexamethyleneimine
N-ethyl-N-(5-phenoxypentyl)-propylamine
1-(5-phenoxypentyl)-2-methyl-piperidine
1-(5-phenoxypentyl)-4-propyl-piperidine
1-(5-phenoxypentyl)-4-methyl-piperidine
1-(5-phenoxypentyl)-3-methyl-piperidine
1-acetyl-4-(5-phenoxypentyl)-piperazine
1-(5-phenoxypentyl)-3,5-trans-dimethyl-piperidine
1-(5-phenoxypentyl)-3,5-cis-dimethyl-piperidine
1-(5-phenoxypentyl)-2,6-cis-dimethyl-piperidine
4-carboethoxy-1-(5-phenoxypentyl)-piperidine
3-carboethoxy-1-(5-phenoxypentyl)-piperidine
1-[3-(4-cyclopropylcarbonylphenoxy) propyl]-piperidine
1-[3-(4-acetylphenoxy)-2-R-methylpropyl] piperidine
1-[3-(4-cyanophenoxy)propyl]-4-methylpiperidine
1-[3-(4-cyanophenoxy)propyl]-3-methylpiperidine
1-[3-(4-acetylphenoxy)-2-S-methylpropyl] piperidine
1-{3-[4-(3-oxobutyl)phenoxy] propyl}piperidine
1-[3-(4-cyano-3-fluorophenoxy)propyl] piperidine
1-[3-(4-nitrophenoxy)propyl]-3-methylpiperidine
1-[3-(4-cyanophenoxy)propyl]-2-methylpiperidine
1-[3-(4-nitrophenoxy)propyl]-2-methylpiperidine
1-[3-(4-nitrophenoxy)propyl]-4-methylpiperidine
1-[3-(4-cyanophenoxy)propyl]-2,6-dimethyfpiperidine
1-[3-(4-propionylphenoxy)propyl]-3-methylpiperidine
1-[3-(4-cyclobutylcarbonylphenoxy)propyl] piperidine
1-[3-(4-cyclopentylcarbonylphenoxy) propyl]piperidine
1-[3-(4-cyanophenoxy)propyl]-cis-2-methyl-5-ethylpiperidine
1-[3-(4-cyanophenoxy)propyl]-trans-2-methyl-5-ethylpiperidine
1-[3-(4-cyanophenoxy)propyl]-cis-3,5-dimethylpiperidine
1-[3-(4-propionylphenoxy)propyl]-4-methylpiperidine
1-[3-(4-propionylphenoxy)propyl]-2-methylpiperidine
1-{3-[4-(1-hydroxypropyl)phenoxy]propyl}-3-methylpiperidine
1-{3-[4-(1-hydroxypropyl)phenoxy]propyl}-4-methylpiperidine
1-[3-(4-propionylphenoxy)propyl]-2-methylpiperidine
1-[3-(4-propionylphenoxy)propyl]-4-methytpiperidine methoxime
1-[3-(4-cyanophenoxy)propyl]-trans-3,5-dimethylpiperidine
1-[3-(4-cyclopropyl carbonyl phenoxy) propyl] -trans-3,5 -dimethylpiperidine
1-[3-(4-cyclopropyl carbonyl phenoxy) propyl] -cis-3,5 -dimethylpiperidine
1-[3-(4-carbomethoxyphenoxy)propyl] piperidine
1-[3-(4-propenylphenoxy)propyl]-2-methyl piperidine
1-[3-(4-propionylphenoxy)propyl]-2-methylpiperidine
1-{3-[4-(1-ethoxypropyl)phenoxy]propyl}-2-methyl piperidine
1-[3-(4-propionylphenoxy)propyl]-4-methylpiperidine
1-[3-(4-bromophenoxy)propyl]piperidine
1-[3-(4-nitrophenoxy)propyl]piperidine
1-[3-(4-N,N-dimethylsulfonamidophenoxy) propyl]piperidine
1-[3-(4-isopropylphenoxy)propyl]piperidine
1-[3-(4-sec-butylphenoxy)propyl]piperidine
1-[3-(4-propylphenoxy)propyl]piperidine
1-[3-(4-ethylphenoxy)propyl]piperidine
1-(5-phenoxypentyl)-1,2,3,6-tetrahydropyridine
1-[5-(4-nitrophenoxy)-pentyl]-pyrrolidine
1-[5-(4-chlorophenoxy)-pentyl]-pyrrolidine
1-[5-(4-methoxyphenoxy)-pentyl]-pyrrolidine
1-[5-(4-methylphenoxy)-pentyl]-pyrrolidine
1-[5-(4-cyanophenoxy)-pentyl]-pyrrolidine
1-[5-(2-naphthyloxy)-pentyl]-pyrrolidine
1-[5-(1-naphthyloxy)-pentyl]-pyrrolidine
1-[5-(3-chlorophenoxy)-pentyl]-pyrrolidine
1-[5-(4-phenylphenoxy)-pentyl]-pyrrolidine
1-{5-[2-(5,6,7,8-tetrahydronaphthyl)-oxy]-pentyl}-pyrrolidine
1-[5-(3-phenylphenoxy)-pentyl]-pyrrolidine
1-(5-phenoxypentyl)-2,5-dihydropyrrole
1-{5-[1-(5,6,7,8-tetrahydronaphthyl)-oxy]-pentyl}-pyrrolidine
1-(4-phenoxybutyl)-pyrrolidine
1-(6-phenoxyhexyl)-pyrrolidine
1-(5-phenylthiopentyl)-pyrrolidine
1-(4-phenylthiobutyl)-pyrrolidine
1-(3-phenoxypropyl)-pyrrolidine
1-[5-(3-nitrophenoxy)-pentyl]-pyrrolidine
1-[5-(4-fluorophenoxy)-pentyl]-pyrrolidine
1-[5-(4-nitrophenoxy)-pentyl]-3-methyl-piperidine
1-[5-(4-acetylphenoxy)-pentyl]-pyrrolidine
1-[5-(4-aminophenoxy)-pentyl]-pyrrolidine
1-[5-(3-cyanophenoxy)-pentyl]-pyrrolidine
N-[3-(4-nitrophenoxy)-propyl]-diethylamine
N-[3-(4-cyanophenoxy)-propyl]-diethylamine
1-[5-(4-benzoylphenoxy)-pentyl]-pyrrolidine
1 -{5-[4-(phenylacetyl)-phenoxy]-pentyl}-pyrrolidine
N-[3-(4-acetylphenoxy)-propyl]-diethylamine
1-[5-(4-acetamidophenoxy)-pentyl]-pyrrolidine
1-[5-(4-phenoxyphenoxy)-pentyl]-pyrrolidine
1-[5-(4-N-benzamidophenoxy)-pentyl]-pyrrolidine
1-{5-[4-(1-hydroxyethyl)-phenoxy]-pentyl}-pyrrolidine
1-[5-(4-cyanophenoxy)-pentyl]-diethylamine
1-[5-(4-cyanophenoxy)-pentyl]-piperidine
N-[5-(4-cyanophenoxy)-pentyl]-dimethylamine
N-[2-(4-cyanophenoxy)-ethyl]-diethylamine
N-[3-(4-cyanophenoxy)-propyl]-dimethylamine
N-[4-(4-cyanophenoxy)-butyl]-diethylamine
N-[5-(4-cyanophenoxy)-pentyl]-dipropylamine
1-[3-(4-cyanophenoxy)-propyl]-pyrrolidine
1-[3-(4-cyanophenoxy)-propyl]-piperidine
N-[3-(4-cyanophenoxy)-propyl]-hexamethyleneimine
N-[6-(4-cyanophenoxy)-hexyl]-diethylamine
N-[3-(4-cyanophenoxy)-propyl]-dipropylamine
N-3-[4-(1-hydroxyethyl)-phenoxy]-propyl-diethylamine
4-(3-diethylaminopropoxy)-acetophenone-oxime
1-[3-(4-acetylphenoxy)-propyl]-piperidine
1-[3-(4-acetylphenoxy)-propyl]-3-methyl-piperidine
1-[3-(4-acetylphenoxy)-propyl]-3,5-trans-dimethyl-piperidine
1-[3-(4-acetylphenoxy)-propyl]-4-methyl-piperidine
1-[3-(4-propionylphenoxy)-propyl]-piperidine
1-[3-(4-acetylphenoxy)-propyl]-3,5-cis-dimethyl-piperidine
1-[3-(4-formylphenoxy)-propyl]-piperidine
1-[3-(4-isobutyrylphenoxy)-propyl]-piperidine
N-[3-(4-propionylphenoxy)-propyl]-diethylamine
1-[3-(4-butyrylphenoxy)-propyl]-piperidine
1-[3-(4-acetylphenoxy)-propyl]-1,2,3,6-tetrahydropyridine

More preferred compounds are:
1-[5-(4-nitrophenoxy)-pentyl]-pyrrolidine
N-[3-(4-cyanophenoxy)-propyl]-diethylamine
N-[3-(4-acetylphenoxy)-propyl]-diethylamine
1-{5-[4-(1-hydroxyethyl)-phenoxy]-pentyl}-pyrrolidine
N-[4-(4-cyanophenoxy)-butyl]-diethylamine
1-[3-(4-cyanophenoxy)-propyl]-piperidine
N-[3-(4-cyanophenoxy)-propyl]-hexamethyleneimine
N-3-[4-(1-hydroxyethyl)-phenoxy]-propyl-diethylamine
4-(3-diethylaminopropoxy)-acetophenone-oxime
1-[3-(4-acetylphenoxy)-propyl]-3-methyl-piperidine
1-[3-(4-acetylphenoxy)-propyl]-4-methyl-piperidine
1-[3-(4-propionylphenoxy)-propyl]-piperidine

Compounds of formula (I) in which:
- -NR¹R² is a pyrrolidinyl group, CₙH₂ₙ is a linear chain -(CH₂)ₙ- and n₃ is zero, X being an oxygen atom with n ranging from 3 to 5, or X being a sulfur atom with n being 4 or 5;
- -NR¹R² is a piperidinyl group, CₙH₂ₙ is a linear chain -(CH₂)ₙ- and X is an oxygen atom, n₃ being zero with n being 2, 5 or 8 or n₃ being 1 with R³ being 4-CN and n being 5;
- -NR¹R² is a diethylamine group, X is an oxygen atom, CₙH₂ₙ is a linear chain -(CH₂)ₙ- and n₃ is 1, R³ being 4-NO₂ or 4-COCH₃ with n being 3 or R³ being 4-CN with n being 2 to 4;
- -NR¹R² is a dimethylamine group, X is an oxygen atom, CₙH₂ₙ is a linear chain -(CH₂)ₙ- and n³ is 1, R³ being 4-CN with n being 3,
are known in the art.

According to a second aspect, it is herein described non-imidazole compounds analogous to the compounds disclosed in WO 96/29315 and WO 93/14070.

Thus, a first sub-class of the compounds (A) is defined by the compounds having the following general formula (IIa) and (IIb): 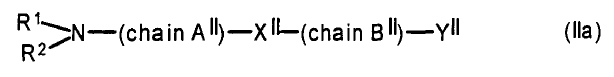 or 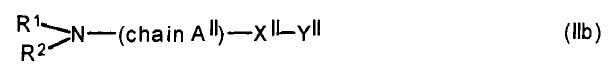 in which
- R¹ and R² are as defined with reference to general formula (A);
- the chain A^{II} represents a saturated or unsaturated, straight or branched hydrocarbon chain containing 1 to 6 carbon atoms, it being possible for the saturated hydrocarbon chain to be interrupted by a hetero atom such as a sulphur atom;
- X^{II} represents an oxygen or sulphur atom, -NH-, -NHCO-, -N(alkyl)CO-, -NHCONH-, -NH-CS-NH-, -NHCS-, -O-CO-, -CO-O-, -OCONH-, -OCON(alkyl)-, -OCON(alkene),-OCONH-CO-, -CONH-, -CON(alkyl)-, -SO-, -CO-, -CHOH-, -N(saturated or unsaturated alkyl), -S-C(=NY")-NH-Y"- with the Y" identical or different and as defined previously, or -NR_{II}-C(=NR"_{II})-NR'_{II}-, R_{II} and R'_{II} denoting a hydrogen atom or a lower alkyl radical and R"_{II} a hydrogen atom or another powerful electronegative group, such as a cyano or COY₁^{II} group, Y₁^{II} denoting an alkoxy group;
- the chain B^{II} represents an aryl, arylalkyl or arylalkanoyl group, a straight alkylene chain -(CH₂)_{nII}-, n being an integer which can vary between 1 and 5 or a branched alkylene chain containing from 2 to 8 carbon atoms, the alkylene chain being optionally interrupted by one or a number of oxygen or sulphur atoms, or a group -(CH₂)_{nII}-O- or -(CH₂)_{nII}-S- where n_{II} is an integer equal to 1 or 2;
- Y^{II} represents a straight or branched alkyl group containing 1 to 8 carbon atoms; a cycloalkyl containing 3 to 6 carbon atoms; a bicycloalkyl group; a cycloalkenyl group; an aryl group such as an optionally substituted phenyl group; a 5- or 6-membered heterocyclic radical containing one or two heteroatoms chosen from nitrogen and sulphur atoms, the said heterocyclic radical optionally being substituted; or also a bicyclic radical resulting from the fusion of a benzene ring to a heterocycle as defined above.

The chain A can be a straight alkylene chain -(CH₂)_{nII}-, n_{II} representing an integer between 1 and 6 carbon atoms, preferably between 1 and 4 carbon atoms, or a branched alkylene chain, preferably a chain substituted by one or a number of methyl or ethyl radicals.

The chain A^{II} can also be a straight or branched unsaturated alkylene chain, and can be, for example, the allyl group.

When Y" represents a cycloalkyl group, the latter can be, for example, cyclopentyl, cyclohexyl or a bicycloalkyl group.

When Y^{II} represents a substituted phenyl group, the phenyl group can be mono- or polysubstituted, for example, by a halogen, by a lower alkyl, for example CH₃, by CF₃, CN, COCH₃, COOR^{II}₁ or OR^{II}₁, R^{II}₁ representing a lower alkyl, for example COOCH₃, the NO₂ group or the group NR^{II}₂R^{II}₃, R^{II}₂ and R^{II}₃ representing a hydrogen atom and/or a lower alkyl radical ("lower alkyl" means an alkyl radical containing at most 6 carbon atoms).

When Y^{II} represents a heterocyclic radical, the latter can be, for example, the pyridyl radical, the pyridyl N-oxide radical or the pyrazinyl radical, optionally mono- or polysubstituted by NO₂, CF₃, CH₃, NH₂, a halogen such as Cl, the COOCH₃ group or also the thiazolyl radical.

When Y^{II} represents a polycyclic radical resulting from condensed aromatic or heteroaromatic moieties the radical can be, for example, the benzothiazolyl, quinolinyl, isoquinolinyl radical or related moieties.

A second sub-class of the compounds (A) comprises the compounds having the above-formulae (IIa) and (IIb) in which:
- R¹R² are as defined with reference to general formula (A);
- the chain A" represents an unbranched, branched or unsaturated alkyl group -(CH₂)_{nII}- where n_{II} is an integer which can vary between 1 and 8 and preferably between 1 and 4; an unbranched or branched alkene group comprising from 1 to 8 carbon atoms and preferably 1 to 4 carbon atoms; an unbranched or branched alkyne group comprising from 1 to 4 carbon atoms;
- the group X^{II} represents -OCONH-; -OCON(alkyl)-; -OCON(alkene)-; -OCO-; -OCSNH-; -CH₂-; -O-; -OCH₂CO-; -S-; -CO-; -CS-; amine; saturated or unsaturated alkyl;
- the chain B" represents an unbranched, branched or unsaturated lower alkyl comprising from 1 to 8 carbon atoms and preferably 1 to 5 carbon atoms; -(CH₂)_{nII}(hetero atom)- where the hetero atom is preferably a sulphur or oxygen atom; n_{II} being an integer which can vary between 1 and 5, preferably between 1 and 4;
- the group Y^{II} represents a phenyl group, unsubstituted or mono- or polysubstituted with one or more identical or different substituents selected from halogen atoms, OCF₃, CHO, CF₃, SO₂N(alkyl)₂ such as SO₂N(CH₃)₂, NO₂, S(alkyl), S(aryl), SCH₂(phenyl), an unbranched or branched alkene, an unbranched or branched alkyne optionally substituted with a trialkylsilyl radical, -O(alkyl), -O(aryl), -CH₂CN, a ketone, an aldehyde, a sulphone, an acetal, an alcohol, a lower alkyl, -CH=CH-CHO, -C(alkyl)=N-OH, -C(alkyl)=N-O(alkyl) and other keto derivatives, -CH=NOH, -CH=NO(alkyl), and other aldehyde derivatives, -C(alkyl)=NH-NH-CONH₂, an O-phenyl or -OCH₂(phenyl) group, -C(cycloalkyl)=NOH, -C(cycloalkyl)=N-O(alkyl), an optionally substituted heterocycle; a heterocycle comprising a sulphur hetero atom; a cycloalkyl; a bicyclic group and preferably a norbornyl group; a phenyl ring fused to a heterocycle comprising a nitrogen hetero atom or to a carbocycle or a heterocycle bearing a keto function; an unbranched or branched lower alkyl comprising from 1 to 8 carbon atoms; an unbranched or branched alkyne comprising from 1 to 8 carbon atoms and preferably 1 to 5 carbon atoms; a linear or branched alkyl mono- or polysubstituted with phenyl groups which are either unsubstituted or mono- or polysubstituted; a phenyl alkyl ketone in which the alkyl group is branched or unbranched or cyclic; a substituted or unsubstituted benzophenone; a substituted or unsubstituted, unbranched or branched or cyclic phenyl alcohol; an unbranched or branched alkene; a piperidyl group; a phenylcycloalkyl group; a polycyclic group, in particular a fluorenyl group, a naphthyl or polyhydronaphthyl group or an indanyl group; a phenol group; a ketone or keto derivative; a diphenyl group; a phenoxyphenyl group; a benzyloxyphenyl group.

Group X^{II} representing an amine is understood to mean a secondary or tertiary amine.

The alkyl, alkene, alkyne, keto, aldehyde, cycloalkyl, S-alkyl, O-alkyl, phenyl alcohol and phenyl-cycloalkyl groups mentioned above as well as in the remainder of the description and the claims of the present patent comprise from 1 to 8 carbon atoms, and preferably 1 to 5.

Likewise, keto derivatives are understood to mean any oxime, alkyloxime, hydrazone, acetal, aminal, ketal, thione, carbazone or semicarbazone group and the thio analogues of these derivatives.

Likewise, by mono- or polysubstituted phenyl and/or benzophenone groups, it is understood to mean that these groups are substituted with one or more identical or different substituents selected from halogen atoms, OCF₃, CHO, CF₃, SO₂N(alkyl)₂, SO₂N(CH₃)₂, NO₂, S(alkyl), S(aryl), SCH₂(phenyl), an unbranched or branched alkene, an unbranched or branched alkyne optionally substituted with a trialkylsilyl radical, -O(alkyl), -O(aryl), -CH₂CN, a ketone, an aldehyde, a sulphone, an acetal, an alcohol, a lower alkyl, -CH=CH-CHO, -C(alkyl)=N-OH, -C(alkyl)=N-O(alkyl) an other keto derivatives, -CH=NOH, -CH=NO(alkyl), and other aldehyde derivatives, -C(alkyl)=NH-NH-CONH₂, an O-phenyl or -OCH₂(phenyl) group, -C(cycloalkyl)=NOH, -C(cycloalkyl)=NO(alkyl), an optionally substituted heterocycle.

The keto substituent is preferably selected from a linear- or branched-chain aliphatic ketone, it being possible for the said chain to comprise from 1 to 8 carbon atoms and optionally to bear a hydroxyl group, a cycloalkyl ketone, an aryl alkyl ketone or aryl alkenyl ketone in which the aryl group is unsubstituted or mono- or polysubstituted, or a heteroaryl ketone in which the heteroaryl unit is preferably monocyclic.

The acetal substituent preferably consists of an aliphatic acetal comprising from 1 to 8 carbon atoms and optionally bearing a hydroxyl radical.

Group Y^{II} representing a ketone is understood to mean, in particular, a ketone substituted with an alkyl or aryl group, it being possible for these groups to be substituted or unsubstituted.

As regards the heterocycles, these comprise from 1 to 3 hetero atoms, preferably sulphur, oxygen or nitrogen atoms.

The heterocycle substituent is preferably selected from an oxadiazole or an imidazole.

Preferred compounds (IIa) and (IIb) are those in which X^{II} is selected from -O-, -NH-, -CH₂-, -OCONH-, -NHCO-, -NHCONH-. X^{II} represents more preferably an oxygen atom.

Preferred compounds (IIa) and (IIb) are also those in which Y" is selected from a linear or branched alkyl group as above defined; a cycloalkyl group as above-defined, in particular cyclopentyl or cyclohexyl group; a phenyl group unsubstituted or mono-substituted, preferred substituent being halogen atom, in particular chorine; a heterocyclic radical, in particular pyridyl N-oxide or pyrazinyl radicals; a bicyclic radical such as a benzothiazolyl radical.

Y" is preferably a phenyl group at least mono-substituted with -CHO, a ketone, an aldehyde, -CH=CH-CHO, -C(alkyl)=N-OH, -C(alkyl)=NO(alkyl) and other keto derivatives, -CH=N-OH, -CH=NO(alkyl) and other aldehyde derivatives, -C(cycloalkyl)=NOH, -C(cycloalkyl)=N-O(alkyl).

Y" represents especially a phenyl group at least mono-substituted with a keto-substituent or an oxime-substituent, or an halogen atom.

Particularly preferred keto-substituent is cycloalkyl ketone.

Other preferred compounds are those wherein Y^{II} represents a phenyl group fused to a carbocycle bearing a keto-function.

Yet other preferred Y^{II} are phenylalkyl ketone in which the alkyl group is branched or unbranched or cyclic; an optionally substituted benzophenone, a ketone.

Particularly preferred group Y^{II} are a phenyl group unsubstituted or mono-substituted as above-defined.

The chain A^{II} is preferably a chain -(CH₂)ₙ^{II}- with n_{II} varying from 1 to 6, preferably from 1 to 4. The chain A^{II} represents especially -(CH₂)₃-.

Preferred chain B^{II} is -(CH₂)₂- or -(CH₂)₃-.

Among compounds (IIa) and (IIb), particularly preferred compounds are those in which X^{II} is an oxygen atom, the chain A^{II} represents -(CH₂)₃- and, for compounds of formula (IIa), the chain B" represents -(CH₂)₃- also.

In this group, Y^{II} is preferably an aryl group.

Preferred group R¹ and R² are as above-defined with reference to formula (A).

Examples of compounds (IIa) and (IIb) are:
- 3,3-Dimethylbutyl 3-piperidinopropyl ether
- 3-Phenylpropyl 3-piperidinopropyl ether
- 3-(4-Chlorophenyl)propyl 3-piperidinopropyl ether
- 2-Benzothiazolyl 3-piperidinopropyl ether
- 3-Phenylpropyl 3-(4-methylpiperidino)propyl ether
- 3-Phenylpropyl 3-(3,5-cis-dimethylpiperidino)propyl ether
- 3-Phenylpropyl 3-(3,5-trans-dimethylpiperidino)propyl ether
- 3-Phenylpropyl 3-(3-methylpiperidino)propyl ether
- 3-Phenylpropyl 3-pyrrolidinopropyl ether
- 3-(4-Chlorophenyl)propyl 3-(4-methylpiperidino)propyl ether
- 3-(4-Chloro phenyl) propyl 3-(3,5-cis-dimethyl piperidino) propyl ether
- 3-(4-Chloro phenyl) propyl 3-(3,5-trans-dimethyl piperidino) propyl ether
- 3-Phenylpropyl 3-(N,N-diethylamino)propyl ether
- N-Phenyl-3-piperidinopropyl carbamate
- N-Pentyl-3-piperidinopropyl carbamate
- (S)-(+)-N-[2-(3,3-Dimethyl)butyl]-3-piperidinopropyl carbamate
- 3-Cyclopentyl-N-(3-(1-pyrrolidinyl)propyl)propanamide
- N-Cyclohexyl-N'-(1-pyrrolidinyl-3-propyl)urea
- 2-((2-Piperidinoethyl)amino)benzothiazole
- 5-Piperidinopentylamine
- 2-Nitro-5-(6-piperidinohexyl)pyridine
- 3-Nitro-2-(6-piperidinohexylamino)pyridine
- 2-(6-Piperidinohexylamino)pyrimidine
- N-(6-Phenylhexyl)piperidine
- N-(3-(N,N-Diethylamino)propyl)N'-phenylurea
- N-Cyclohexylmethyl-N'-(3-piperidinopropyl)guanidine

Preferred compounds according to the application of the invention include compounds of formula (IIa): 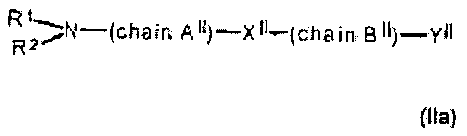 wherein:
R¹ and R² form together with the nitrogen atom to which they are attached a saturated nitrogen-containing ring 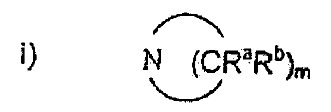 with m ranging from 2 to 8, or
R^{a-b} being independently a hydrogen atom or an alkyl containing 1 to 6 carbon atoms,
the chain A^{II} selected from an unbranched alkyl group -(CH₂)_{nII}- where n_{II} is 3 ; the group X" is -O- ;
the chain B" is an unbranched alkyl comprising 3 carbon atoms; and
the group Y^{II} represents a phenyl group, unsubstituted or mono- or polysubstituted with one or more identical or different substituents selected from halogen atoms, OCF₃, CHO, CF₃, SO₂N(alkyl)₂ such as SO₂N(CH₃)₂, NO₂, S(aryl), SCH₂(phenyl), an unbranched or branched alkene or alkyne optionally substituted with a trialkylsilyl radical, -O(alkyl), -O(aryl), -CH₂CN, a ketone, an aldehyde, a sulphone, an acetal, an alcohol, a linear or branched alkyl group containing 1 to 6 carbon atoms, -CH=CH-CHO, -C(alkyl)=N-OH, -C(alkyl)=N-O(alkyl), -CH=NOH, -CH=NO(alkyl), -C(alkyl)=NH-NH-CONH₂, an O-phenyl or -OCH₂(phenyl) group, -C(cycloalkyl)=NOH, -C(cycloalkyl)=N-O(alkyl);
or their pharmaceutically acceptable salts, hydrates, or hydrated salts, or the polymorphic crystalline structures of these compounds or their optical isomers, racemates, diastereoisomers or enantiomers.

Preferably, -NR¹R² is a saturated nitrogen-containing ring of formula: 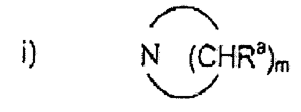 with R^{a} and m being as defined above. Preferably, R^{a} is a hydrogen atom and m is 4 or 5.

More preferably, -NR¹R² is selected from the group consisting in piperidyl, pyrrolidinyl.

Preferably, the nitrogen-containing ring i) is one of mono- and di-substituted; more preferably mono-substituted with an alkyl group, such as with a methyl group.

According to a preferred aspect, the substituent(s) is(are) in beta-position with respect to the nitrogen atom.

Preferably, Y^{II} represents a phenyl group at least mono-substituted with a halogen atom, a keto-substituent which may include a linear or branched chain aliphatic ketone comprising from 1 to 8 carbon atoms and optionally bearing a hydroxyl group, a cycloalkylketone, an arylalkylketone or arylalkenylketone in which the aryl group is optionally substituted, or a heteroaryl ketone.

More preferably, Y" is a phenyl group at least mono-substituted with a halogen atom, -CHO, a ketone, an aldehyde, -CH=CH-CHO, -C(alkyl)=N-OH, -C(alkyl)=N-O(alkyl), -CH=N-OH, -CH=NO(alkyl), -C(cycloalkyl)=NOH, -C(cycloalkyl)=N-O(alkyl).

According to a more preferred aspect, compounds of formula (IIa) are selected from:
- 3-Phenylpropyl 3-piperidinopropyl ether
- 3-(4-Chlorophenyl)propyl 3-piperidinopropyl ether
- 3-Phenylpropyl 3-(4-methylpiperidino)propyl ether
- 3-Phenylpropyl 3-(3,5-cis-dimethylpiperidino)propyl ether
- 3-Phenylpropyl 3-(3,5-trans-dimethylpiperidino)propyl ether
- 3-Phenylpropyl 3-(3-methylpiperidino)propyl ether
- 3-Phenylpropyl 3-pyrrolidinopropyl ether
- 3-(4-Chlorophenyl)propyl 3-(4-methylpiperidino)propyl ether
- 3-(4-Chlorophenyl) propyl 3-(3,5-cis-dimethyl piperidino)propyl ether
- 3-(4-Chlorophenyl) propyl 3-(3,5-trans-dimethyl piperidino)propyl ether.
or their pharmaceutically acceptable salts, hydrates, or hydrated salts, or the polymorphic crystalline structures of these compounds or their optical isomers, racemates, diastereoisomers or enantiomers.

According to a still more preferred aspect, a compound of formula (IIa) is selected from 3-(4-chlorophenyl)propyl-3-piperidinopropylether, or its pharmaceutically acceptable salts, hydrates, or hydrated salts, or the polymorphic crystalline structures of this compound or its optical isomers, racemates, diastereoisomers or enantiomers

Preferably, compounds are in the form of a pharmaceutically acceptable salt and said salt is chosen from the group consisting in hydrochloride, hydrobromide, hydrogen maleate or hydrogen oxalate. The hydrochloride salt of 3-(4-chlorophenyl)propyl-3-piperidinopropylether is preferred.

According to a third aspect, non-imidazole compounds analogous to the compounds disclosed in EP 197 840 are described herein.

Thus, a sub-class of compounds (A) comprises compounds having the following formula (III) 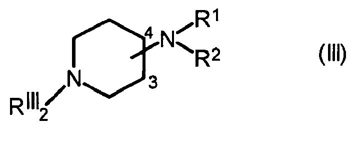 in which:
- NR¹R² is either in 3-position or in 4-position on the piperidyl moiety, R¹ and R² being as defined with reference to formula (A);
- R₂^{III} denotes a linear or branched alkyl group having 1 to 6 carbon atoms; a piperonyl group, a 3-(1-benzimidazolonyl)propyl group; a group of formula 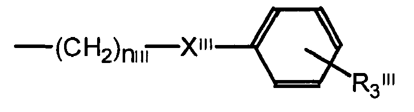
in which n_{III} is 0, 1, 2 or 3, X^{III} is a single bond or alternatively -0-, -S-, -NH-, -CO-, -CH=CH- or 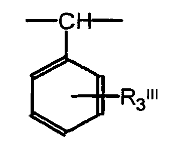 and R₃^{III} is H, CH₃, halogen, CN, CF₃ or an acyl group -COR₄^{III}, R₄^{III} being a linear or branched alkyl group having 1 to 6 carbon atoms, a cycloalkyl group having 3 to 6 carbon atoms or a phenyl group which can bear a CH₃ or F substituent; or alternatively a group of formula 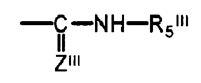 in which Z^{III} denotes an O or S atom or a divalent group NH, N-CH₃ or N-CN and R₅^{III} denotes a linear or branched alkyl group having 1 to 8 carbon atoms, a cycloalkyl group having 3 to 6 carbon atoms which can bear a phenyl substituent, a (C₃-C₆ cycloalkyl) (linear or branched, C₁-C₃ alkyl) group, a phenyl group which can bear a CH₃, halogen or CF₃ substituent, a phenyl(linear or branched, C₁-C₃ alkyl) group or a naphthyl, adamantyl or p-toluenesulphonyl group.

Preferred compounds (III) are those with R^{III} representing the group 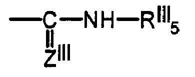 Z^{lll} and R^{lll}₅ being as above-defined and Z^{lll} is especially O, S or NH.

Preferred group R^{lll}₅ is a (C₃-C₆)cycloalkyl group.

Preferred R¹ and R² groups are as above-described in formula (A).

An example of such compound (III) is N'-Cyclohexylthiocarbamoyl-N-1,4'-bipiperidine (compound 123).

According to a fourth aspect, a sub-class of compounds (A) includes the compounds which have the following formula (IV), analogous to compounds disclosed in EP 494 010: 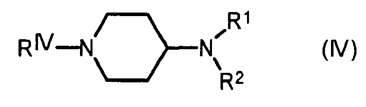 in which
- R¹ and R² are as defined with reference to general formula (A);
- R^{IV} represents a hydrogen atom or a group COR₃^{IV}, in which R₃^{IV} represents
   (a) a linear or branched aliphatic group containing 1 to 11, and in particular 1 to 9, carbon atoms;
   (b) a cyclane ring-system such as cyclopropane, phenylcyclopropane, cyclobutane, cyclopentane, cyclohexane, cycloheptane, norbornane, adamantane, noradamantane, chlorooxonorbornane, chloroethylenedioxynorbornane, bromoethylenedioxynorbornane and the anhydride group of hydroxycarboxy-1,2,2-trimethylcyclopentanecarboxylic acid;
   (c) a benzene ring, unsubstituted or substituted at the para-position with a linear or branched aliphatic group containing 3 to 5 carbon atoms, as well as with a halogen;
   (d) a group (CH₂)_{mlV}R₄^{lV} in which m_{lV} is a number between 1 and 10, and R₄^{lV} represents a cyclane ring system such as cyclopropane, cyclobutane, cyclopentane, cyclopentene, cyclohexane, cycloheptane, norbornane, noradamantane, adamantane and 6,6-dimethylbicyclo[3.1.1] heptene; a benzene ring, unsubstituted or monosubstituted with a fluorine atom, a chlorine atom, a methyl group or a methoxy group; a thiophene ring grafted via its ring-position 2 or its ring-position 3; a carboxylic acid ester group COOR₅^{IV}, in which R₅^{lV} is a cyclane ring-system such as cyclopropane, cyclobutane, cyclopentane, cyclohexane or norbornane; a carboxylic acid amide group of structure CONHR₆^{lV}, in which R₆^{lV} represents a cyclane ring-system such as cyclopropane, cyclobutane, cyclopentane, cyclohexane or norbornane; a carboxylic acid amide group of structure 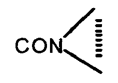 in which the group 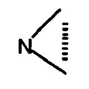 represents pyrrolidine, piperidine or 2,6-dimethylmorpholine; or an ether group - O-R₇^{lV}, it being possible for R₇^{IV} to be a benzene ring, unsubstituted or monosubstituted with a chlorine or fluorine atom or disubstituted with a chlorine atom and with a methyl group;
   (e) a group -CH=CHR₈^{IV}, in which R₈^{lV} represents a cyclane ring-system such as cyclopropane, cyclobutane, cyclopentane, cyclohexane, norbomane or norbornene;
   (f) a secondary amine group -NH(CH₂)_{nIV}R₉^{lV}, in which n_{IV} is a number between 1 and 5 and R₉^{lV} constitutes a cyclane ring-system such as cyclopropane, cyclobutane, cyclopentane, cyclohexane or norbornane, or a benzene ring, unsubstituted, mono-substituted with a fluorine or chlorine atom or with a methoxy group or trisubstituted with methoxy groups;
      R^{IV} also represents a hydroxyalkenyl group 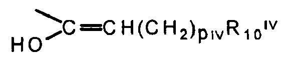 in which p_{lV} is a number between 2 and 9 and R₁₀^{lV}, represents a benzene ring or a phenoxy group; as well as a group

      CSNH(CH₃)_{nlV}R₉^{lV}

   - in which n_{IV} is a number between 1 and 5 and R₉^{lV} has the meaning stated above.

Preferred compounds (IV) are those in which R^{lV} represents the group COR₃^{IV}, R₃^{IV} representing especially an aliphatic group a).

An example of compound (IV) is N-Heptanoyl-1,4'-bipiperidine or 1-(5-Cyclohexylpentanoyl)-1,4-bipiperidine.

According to a fifth aspect, the application describes non-imidazole compounds analogous to those disclosed by Plazzi et al. (Eur. J. Med. Chem. 1995,30,881).

Thus, another sub-class of compounds (A) comprises compounds having the following formula (V): 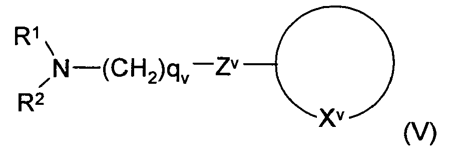 in which
- R¹ and R² are as defined with reference to formula (A) in claim 1;
- q^{v} is 2 to 5
- Z^{V} represents NH, O or S
- Xᵥ represents a heterocycle, optionally condensed, containing one or more heteroatoms like nitrogen, oxygen or sulfur, unsubstituted or substituted by one or more groups like aryl, lower alkyl and halogen.

Preferred compounds are those with X^{V} being an heterocycle like : 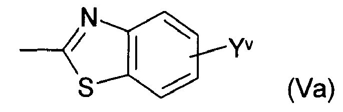 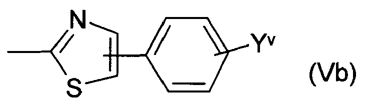 or 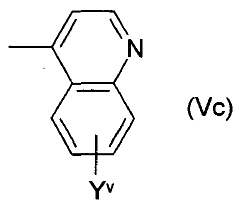 with Y^{v} representing an hydrogen atom, an halogen or a lower alkyl.

Examples of compounds (V) are :
2-((2-Piperidinoethyl)amino)benzothiazole
2-(6-Piperidinohexylamino)benzothiazole
4-(6-Piperidinohexylamino)quinoline
2-Methyl 4-(3-piperidinopropylamino)quinoline
2-Methyl 4-(6-piperidinohexylamino)quinoline
7-Chloro-4-(3-piperidinopropylamino)quinoline
7-Chloro-4-(4-piperidinobutylamino)quinoline
7-Chloro-4-(8-piperidinooctylamino)quinoline
7-Chloro-4-(10-piperidinodecylamino)quinoline
7-Chloro-4-(12-piperidinododecylamino)quinoline
7-Chloro-4-(4-(3-piperidinopropoxy)phenylamino)quinoline
7-Chloro-4-(2-(4-(3-piperidinopropoxy) phenyl) ethylamino) quinoline

According to a sixth aspect, the application describes non-imidazole compounds which are analogous to those disclosed in WO 95/14007.

Thus, another subclass of compounds (A) includes the compounds having the following formula (VI): 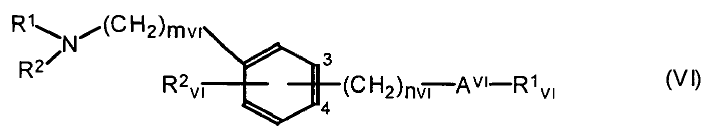 wherein:
- A^{VI} is selected from -O-CO-NR¹_{VI}-, -O-CO-, -NR¹_{VI}-CO-,
- NR¹_{VI}-, -NR¹_{VI}-CO-, -NR¹_{VI}-, -0-, -CO-NR¹_{VI}-, -CO-O-, and -C(=NR¹_{VI})-NR¹_{VI}-;
- the groups R¹_{VI}, which may be the same or different when there are two or three such groups in the molecule of formula VI, are selected from hydrogen, and lower alkyl, aryl, cycloalkyl, heterocyclic and heterocyclyl-alkyl groups, and groups of the formula -(CH₂)_{yVI}-G^{VI}, where G^{VI} is selected from CO₂R³_{VI}, COR³_{VI}, CONR³_{VI}R⁴_{VI}, OR³_{VI}, SR³_{VI}, NR³_{VI}R⁴_{VI}, heteroaryl and phenyl, which phenyl is optionally substituted by halogen, lower alkoxy or polyhaloloweralkyl, and y_{VI} is an integer from 1 to 3;
- R²_{VI} is selected from hydrogen and halogen atoms, and alkyl, alkenyl, alkynyl and trifluoromethyl groups, and groups of the formula OR³_{VI}. SR³_{VI} and NR³_{VI}R⁴_{VI};
- R³_{VI} and R⁴_{VI} are independently selected from hydrogen, and lower alkyl and cycloalkyl groups, or R³_{VI} and R⁴_{VI} together with the intervening nitrogen atom can form a saturated ring containing 4 to 6 carbon atoms that can be substituted with one or two lower alkyl groups;
- the group -(CH₂)_{nVI}-A^{VI}-R¹_{VI} is at the 3- or 4-position, and the group R²_{VI} is at any free position;
- m_{VI} is an integer from 1 to 3;
- and n_{VI} is 0 or an integer from 1 to 3.

When used herein, the following terms have the given meanings:
lower alkyl (including the alkyl portions of lower alkoxy) - represents a straight or branched, saturated hydrocarbon chain having from 1 to 6 carbon atoms, preferably from 1 to 4;
lower alkenyl (in R²_{VI}) - represents a straight or branched aliphatic hydrocarbon radical having at least one carbon-to-carbon double bond (preferably in conjugation with the benzene ring that the group R² substitutes) and having from 2 to 6 carbon atoms;
lower alkynyl (in R²_{VI}) - represents a straight or branched aliphatic hydrocarbon radical having at least one carbon-to-carbon triple bond (preferably in conjugation with the benzene ring that the group R² substitutes) and having from 2 to 6 carbon atoms;
aryl - represents a carbocyclic group having from 6 to 14 carbon atoms and having at least one benzenoid ring, with all available substitutable aromatic carbon atoms of the carbocyclic group being intended as possible points of attachment, said carbocyclic group being optionally substituted with 1 to 3 Y_{VI} groups, each independently selected from halo, alkyl, hydroxy, loweralkyoxy, phenoxy, amino, loweralkylamino, diloweralkylamino, and polyhaloloweralkyl. Preferred aryl groups include 1-naphthyl, 2-naphthyl and indanyl, and especially phenyl and substituted phenyl;
cycloalkyl - represents a saturated carbocyclic ring having from 3 to 8 carbon atoms, preferably 5 or 6;
halogen - represents fluorine, chlorine, bromine and iodine;
heterocyclic - represents, in addition to the heteroaryl groups defined below, saturated and unsaturated cyclic organic groups having at least one O, S and/or N atom interrupting a carbocyclic ring structure that consists of one ring or two fused rings, wherein each ring is 5-, 6- or 7-membered and may or may not have double bonds that lack delocalized pi electrons, which ring structure has from 2 to 8, preferably from 3 to 6 carbon atoms; e.g., 2- or 3-piperidinyl, 2-or 3-piperazinyl, 2- or 3-morpholinyl, or 2- or 3-thiomorpholinyl;
heteroaryl - represents a cyclic organic group having at least one O, S and/or N atom interrupting a carbocyclic ring structure and having a sufficient number of delocalized pi electrons to provide aromatic character, with the aromatic heterocyclic group having from 2 to 14, preferably 4 or 5 carbon atoms, e.g., 2-, 3- or 4-pyridyl, 2- or 3-furyl, 2- or 3-thienyl, 2-, 4- or 5-thiazolyl, 2- or
2-, 4- or 5-pyrimidinyl, 2-pyrazinyl, or 3- or 4-pyridazinyl, etc.

Preferred heteroaryl groups are 2-, 3- and 4-pyridyl;
heterocyclyl-alkyl - represents a heterocyclic group defined above substituting an alkyl group; e.g., 2-(3-piperidinyl)-ethyl, (2-piperazinyl)-methyl, 3-(2-morpholinyl)-propyl, (3-thiomorpholinyl)-methyl, 2-(4-pyridyl)-ethyl, (3-pyridyl)-methyl, or (2-thienyl)-methyl.

Preferably, A^{VI} is -CH₂-NR¹_{VI}- or especially -C(=NH)-NR¹_{VI}- preferred compounds include those wherein m_{VI}, is 1 or 2, and n_{VI} is 0, 1 or 2.

Other preferred values of A include -O-CO-NR¹_{VI}-, -0-, and -CO-O-. In all these compounds, the groups R¹_{VI} are as defined above, and the side chain is preferably at the 4-position. In compounds of formula VI, one group R¹_{VI} is preferably selected from hydrogen, 2-phenylethyl, 4-chlorophenylmethyl, 4-methoxyphenylmethyl, 4-trifluoromethylphenylmethyl and 4-pyridylmethyl, but is especially 4-chlorophenylmethyl; any other group R¹_{VI} that is present is preferably a hydrogen atom or a methyl group.

Particularly preferred compounds are those wherein n_{VI} and m_{VI} are each 1, and A^{VI} represents an oxygen atom.

R¹_{VI} is preferably an aryl or -(CH₂)_{yVI}-G^{VI} with G^{VI} being a phenyl.

R¹ and R² are preferably selected as specified with reference to formula (A).

Another sub-class of compounds (A) comprises compounds of formula (VI) wherein R¹_{VI} represents an aryl group, especially a phenyl optionally substituted with a keto substituent, R²_{VI}, n_{VI}, m_{VI} and A^{VI} having the above-meaning.

The keto substituent is as above-defined in Y^{II} with reference to compounds (IIa) and (IIb).

Preferred compounds are those with n_{VI} and m_{VI} being each 1 and A^{VI} being an oxygen atom.

Examples of compounds VI are :
α-(Acetylphenoxy)-α'-piperidino p-xylol
α-(4-Acetylphenoxy)-α'-(1-pyrrolidinyl)p-xylol
α-(3-Phenylpropoxy)-α'-piperidino p-xylol
α-(4-Acetylphenoxy)-α'-(4-methylpiperidino)p-xylol
α-(4-Acetylphenoxy)-α'-(3,5-cis-dimethylpiperidino)p-xylol
α-(4-Acetylphenoxy)-α'-(3,5-trans-dimethylpiperidino)p-xylol
α-(4-Acetylphenoxy)-α'-(2-methylpyrrolidino)p-xylol
α-(4-Cyclopropylcarbonylphenoxy)-α'-piperidino-p-xylol
α-(4-Cyclopropylcarbonylphenoxy)-α'-(4-methylpiperidino)p-xylol
α-(4-Cyclopropylcarbonylphenoxy)-α'-pyrrolidino-p-xylol
N-(4-Chlorobenzyl)-2-(4-piperidino methyl) phenyl) ethan -amidine

According to a seventh aspect, it is herein described another sub-class of compounds (A) including non-imidazole compounds having the following formula (VII) which are analogous to compounds disclosed in Clitherow et al. (Bioorg. & Med. Chem. Lett., 6 (7), 833, 1996) : 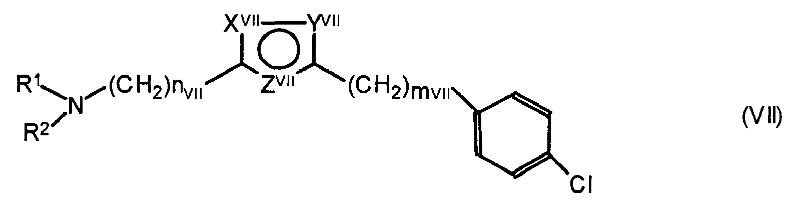 in which
- R¹ and R² are as defined in reference to formula (A);
- X^{VII}, Y^{VII} and Z^{VII} are identical or different and represent O, N or S;
- n_{VII} is varying from 1 to 3;
- m_{VII} is 1 or 2.

n_{VII} is preferably 2 or 3, especially 2 and m_{VI} is preferably 1.

Preferred compounds are those with X^{VII} being 0 and Y^{VII} and Z^{VII} each being N to represent a 1, 2, 4-oxadiazolyl group.

An illustrative compound is given in example 130.

According to a eighth aspect, the application describes another sub-class of compounds (A) including the non-imidazole compounds having the following formula (VIII), which are analogous to those disclosed in WO 95/06037: 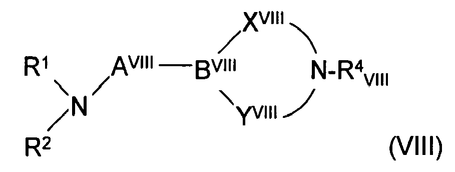 wherein R¹ and R² are as defined with reference to formula (A) and wherein A^{VIII} is
1) a group of the formula (CH₂)_{MVIII}, wherein m_{VIII} = 0-9; or
2) a group of the formula: 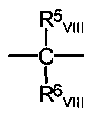 wherein R⁵_{VIII} represents hydrogen, (C₁-C₃)alkyl-, aryl(C₁-C₃)alkyl-, aryl-,
   wherein aryl may optionally be substituted, hydroxyl-, (C₁-C₃)alkoxy-, halogen, amino-, cyano- or nitro; and R⁶_{VIII} represents hydrogen, (C₁-C₃)alkyl-, aryl(C₁₋C₃)alkyl-, or aryl-, wherein aryl may optionally be substituted; or
3) a group of the formula: 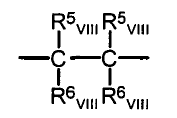 wherein R⁵_{VIII} and R⁶_{VIII} are as defined above; or
4) a group of the formula: 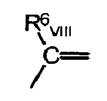 if B^{VIII} is a group of the formula: 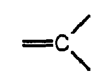 such that A^{VIII} and B^{VIII} together form a group of the formula: 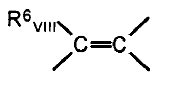 wherein R⁶_{VIII} is as defined above; or
5) a group of the formula: 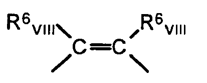 wherein R⁶_{VIII} is as defined above; or
6) a group of the formula: 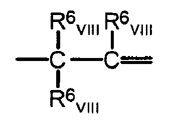 if B^{VIII} is a group of the formula: 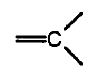 such that A^{VIII} and B^{VIII} together form a group of the formula: 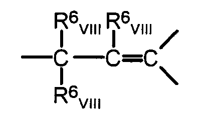 wherein R⁶_{Vlll} is as defined above; or
7) a group of the formula:

   ― (CH₂)_{xVIII}―S―(CH₂)_{yVIII}―

   wherein x_{VIII} + y_{VIII} = m_{VIII}-1 ;
   B^{VIII} is
   1) a group of the formula: 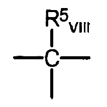 wherein R⁵_{VIII} is as defined above; or
   2) a group of the formula: 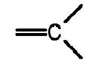 if A is a group of one of the formulas: 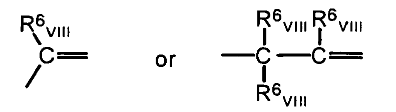 such that A and B together form a group of one of the formulas: 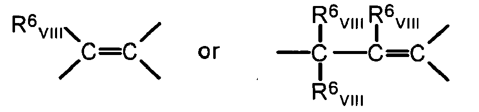 wherein R⁶_{VIII} is as defined above; or
   3) a group of the formula: 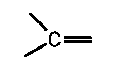 if X^{VIII} is a group of the formula: 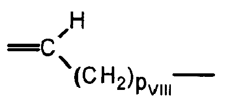 such that B^{VIII} and X^{VIII} together form a group of the formula 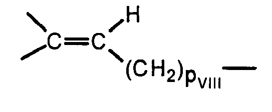
   wherein p_{VIII} = 1-3; or
   X^{VIII} is
   1) a group of the formula (CH₂)_{nVIII} wherein n_{VIII} = 2-4; or
   2) a group of the formula: 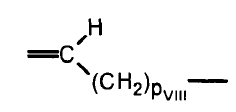 if B^{VIII} is a group of the formula: 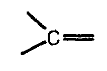 such that X^{VIII} and B^{VIII} together form a group of the formula: 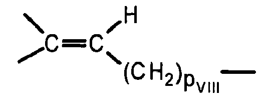 wherein p_{VIII} = 1-3; or
   3) two hydrogens (one on the carbon and one on the nitrogen); or
   4) one hydrogen on the carbon atom and one R⁷_{VIII} group on the nitrogen atom,
   wherein R⁷_{VIII} represents hydrogen, (C₁-C₁₀)alkyl-, aryl (C₁-C₁₀)alkyl-, or aryl,
   wherein aryl may optionally be substituted;
   Y^{VIII} is a group of the formula (CH₂)_{kVIII}, wherein k_{VIII} = 0-2;
   R⁴_{VIII} represents hydrogen, (C₁-C₁₀)alkyl-, (C₁-C₃)alkyl-sulfonamide-, aryl(C₁₋C₁₀)alkyl-, aryl, wherein aryl may optionally be substituted;
   or a group of the formula: 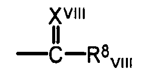 or a group of the formula: 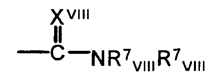 wherein X^{VIII} represents O, S, or NH,
   R⁷VIII is as defined as above;
   R⁸_{VIII}represents (C₁-C₁₀)alkyl-, aryl(C₁-C₁₀)alkyl-or aryl,
   wherein aryl may optionally be substituted and wherein aryl is phenyl, substituted phenyl, naphtyl, substituted naphtyl, pyridyl.

Both linear and ringstructured compounds are encompassed.

The linear compounds have for example one of the formulas

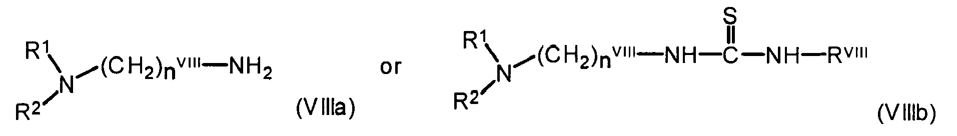

Preferred R¹ and R² groups are as defined with reference to formula (A).

A compound (VIII) is described in examples 132 and 169.

According to a ninth aspect, the instant application describess a sub-class of compounds (A) consisting of compounds having the following formula (IX) which are analogous to those described in WO 97/29092: 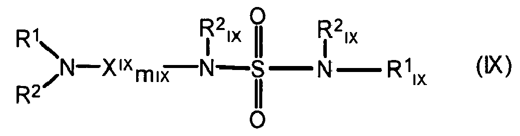 wherein:
R¹ and R² are as defined with reference to formula (A)
R¹_{IX} is C₄ to C₂₀ hydrocarbyl (in which one or more hydrogen atoms may be replaced by halogen, and up to four carbon atoms [and especially from 0 to 3 carbon atoms] may be replaced by oxygen, nitrogen or sulphur atoms, provided that R¹_{IX} does not contain an -O-O-group),
R²_{IX} identical or different, are H or C₁ to C₁₅ hydrocarbyl (in which one or more hydrogen atoms may be replaced by halogen, and up to three carbon atoms may be replaced by oxygen, nitrogen or sulphur atoms, provided that R²_{IX} does not contain an -O-O-group),
m_{IX} is from 1 to 15 (preferably 1 to 10, more preferably 3 to 10, eg. 4 to 9) each X^{IX} group is independently 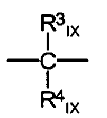 , or one X^{IX} group is -N(R⁴_{IX})-, -O- or -S- (provided that this X^{IX} group is not adjacent the -NR²_{IX-}group) and the remaining X^{IX} groups are independently 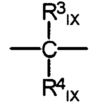 , wherein R³_{IX} is H, C₁ to C₆ alkyl, C₂ to C₆ alkenyl, -CO₂R⁵_{IX}, -CON(R⁵_{IX})₂, -CR⁵_{IX2}OR⁶_{IX} or -OR⁵_{IX} (in which R⁵_{IX} and R⁶_{IX} are H or C₁ to C₃ alkyl), and R⁴_{IX} is H or C₁ to C₆ alkyl.

The term "hydrocarbyl", as used herein, refers to monovalent groups consisting of carbon and hydrogen. Hydrocarbyl groups thus include alkyl, alkenyl, and alkynyl groups (in both straight and branched chain forms), cycloalkyl (including polycycloalkyl), cycloalkenyl, and aryl groups, and combinations of the foregoing, such as alkylaryl, alkenylaryl, alkynylaryl, cycloalkylaryl, and cycloalkenylaryl groups.

A "carbocyclic" group, as the term is used herein, comprises one or more closed chains or rings, which consist entirely of carbon atoms. Included in such groups are alicyclic groups (such as cyclopropyl, cyclobutyl, cyclopentyl, cyclohexyl and adamantyl), groups containing both alkyl and cycloalkyl moieties (such as adamantanemethyl), and aromatic groups (such as phenyl, naphthyl, indanyl, fluorenyl, (1,2,3,4)-tetrahydronaphthyl, indenyl and isoindenyl).

The term "aryl" is used herein to refer to aromatic carbocyclic groups, including those mentioned above.

When reference is made herein to a substituted carbocyclic group (such as substituted phenyl), or a substituted heterocyclic group, the substituents are preferably from 1 to 3 in number and selected from C₁ to C₆ alkyl, C₁ to C₆ alkoxy, C₁ to C₆ alkylthio, carboxy, C₁ to C₆ carboalkoxy, nitro, trihalomethyl, hydroxy, amino, C₁ to C₆ alkylamino, di(C₁ to C₆ alkyl)amino, aryl, C₁ to C₆ alkylaryl, halo, sulphamoyl and cyano.

The term "halogen", as used herein, refers to any of fluorine, chlorine, bromine and iodine.

Preferably, R²_{IX} is selected from H, C₁ to C₆ alkyl, C₁ to C₆ cycloalkyl, C₁ to C₆ hydroxyalkyl, C₁ to C₆ alkylhydroxyalkyl, aryl C₁ to C₆ alkyl and substituted aryl C₁ to C₆ alkyl. For example, R²_{IX} may be H or C₁ to C₃ alkyl.

In certain embodiments, -X^{IX}_{mIX}- is a C₁ to C₈ alkylene group, e.g. a butylene group.

Included in the definition of R¹_{IX} are aryl-containing groups (such as phenyl, substituted phenyl, naphthyl and substituted naphthyl), and (cycloalkyl)alkyl groups (such as cyclohexylpropyl and adamantylpropyl).

Preferably, R¹_{IX} is a group of the formula 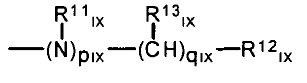 wherein
p_{IX} is 0 or 1,
R¹¹_{IX} is H or C₁ to C₃ alkyl,
q_{IX} is from 0 to 4,
R¹²_{IX} is a carboxyclic, substituted carbocyclic, heterocyclic or substituted heterocyclic group, and
R¹³_{IX} is independently selected from H, C₁ to C₆ alkyl, C₁ to C₆ cycloalkyl, C₁ to C₆ hydroxyalkyl, C₁ to C₆ alkylhydroxyalkyl, aryl C₁ to C₆ alkyl and substituted aryl C₁ to C₆ alkyl.

Preferably, R¹³_{IX} is hydrogen.

Compounds (IX) wherein R¹_{IX} is a group -NH-CH₂-Ph where Ph represents an optionally substituted phenyl, are preferred.

Preferred groups R¹ and R² are as specified with reference to formula (A).

An illustrative example is compound 173.

According to a tenth aspect, another sub-class of compounds (A) is described that comprises compounds having the following formula (X), which are analogous to compounds disclosed by Wolin et al. (Bioorg. & Med. Chem. Lett., 8, 2157 (1998)): 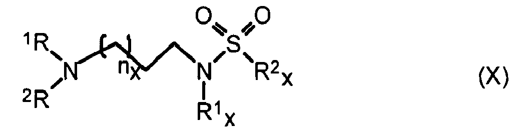 wherein:
- R¹ and R² are as defined with reference to formula (A);
- R¹ₓ is H or CH₃;
- R²ₓ is selected from a phenyl optionally substituted with a halogen atom, preferably chlorine, a (C₁-C₄)alkyl, a (C₁-C₄)alkoxy, CF₃, OCF₃, NO₂, NH₂; or a CH₂-phenyl optionally substituted as above-specified;
- nₓ is from 0 to 3.
nₓ is preferably 1. R² is preferably a phenyl group, especially a mono-substituted phenyl group.

Preferred R¹ and R² are as above-specified for formula (A).

Compound 174 is illustrative of compounds (X).

According to a eleventh aspect, the application describes non-imidazole compounds which are analogous to those disclosed in WO 96/38142.

Thus, another sub-class of compounds (A) is directed to compounds having the following formula (XI): 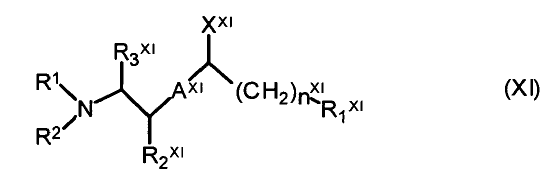 where R¹ and R² are as defined with reference to formula (A);
where A^{XI} is -NHCO-, -N(CH₃)-CO-, -NHCH₂-, -N(CH₃)-CH₂-, -CH=CH-, -COCH₂-, CH₂CH₂-, -CH(OH)CH₂-, or -C=C- ;
X^{XI} is H, CH₃, NH₂, NH(CH₃), N(CH₃)₂, OH, OCH₃, or SH;
R₂^{XI} is hydrogen or a methyl or ethyl group;
R₃^{XI} is hydrogen or a methyl or ethyl group;
n^{XI} is 0, 1, 2, 3, 4, 5 or 6; and
R₁^{XI} is selected from the group consisting of C₃ to C₈ cycloalkyl; phenyl or substituted phenyl; decahydronaphthalene and octahydroindene; or
R₁^{XI} and X^{XI} may be taken together to denote a 5,6 or 6,6 saturated bicyclic ring structure when X^{XI} is NH, O, S, or SO₂.

Preferably for compounds of formula (XI):
A^{XI} is -NHCO-, -N(CH₃)-CO-, -NHCH₂-, -N(CH₃)-CH₂-, -CH=CH-, -COCH₂-, -CH₂CH₂-, -CH(OH)CH₂-, or -C≡C-;
X^{XI} is H, CH₃, NH₂, NH(CH₃), N(CH₃)₂, OH, OCH₃, or SH;
R₂^{XI} is hydrogen or a methyl or ethyl group;
R₃^{XI} is hydrogen or a methyl or ethyl gorup;
n^{XI} is 0, 1, 2, 3, 4, 5, or 6; and
R₁^{XI} is selected from the group consisting of (a) C₃ to C₈ cycloalkyl; (b) phenyl or substituted phenyl; (d) heterocyclic (e) decahydronaphthalene and (f) octahydroindene; or
R₁^{XI} and X^{XI} may be taken together to denote a 5,6 or 6,6 saturated bicyclic ring structure when X^{XI} can be NH, O, or S.

More preferably, the present invention provides compounds
where A^{XI} is -NHCH₂-, -N(CH₃)-CH₂-, -CH=CH-,
-COCH₂-, -CH₂CH₂, -CH(OH)CH₂-, or-C=C-;
X^{XI} is H, CH₃, NH₂, NH(CH₃), N(CH₃)₂, OH, OCH₃, or SH;
R^{XI}₂ is hydrogen or a methyl or ethyl group;
R^{XI}₃ is hydrogen or a methyl or ethyl group;
n^{XI} is 0, 1, 2, 3, 4, 5, or 6; and
R^{XI}₁ is selected from the group consisting of (a) C₃ to C₈ cycloalkyl; (b) phenyl or substituted phenyl; (d) heterocyclic; (e) decahydronaphthalene and (f) octahydroindene; or
R^{XI}₁ and X^{XI} may be taken together to denote a 5,6 or 6,6 saturated bicyclic ring structure when X^{XI} can be NH, 0, or S.

Most preferably, the present invention provides compounds
where A^{XI} is -CH=CH or -C≡C-;
X^{XI} is H, CH₃ or NH₂;
R₂^{XI} and R₃^{XI} are H;
n^{XI} is 1, 2, or 3;
R₁^{XI} is selected from the group consisting of (a) C₃ to C₈ cycloalkyl; (b) phenyl or substituted phenyl; (d) heterocyclic; (e) decahydronaphthalene and (f) octahydroindene; or
R₁^{XI} and X^{XI} may be taken together to denote a 5,6 or 6,6 saturated bicyclic ring structure when X^{XI} is NH, O, or S.

The term "substituted phenyl" as used herein refers to a phenyl group substituted by one or more groups such as alkyl, halogen, amino, methoxy and cyano groups.

The term "alkyl" refers to straight or branched chain radicals. Representative examples of alkyl groups include methyl, ethyl, n-propyl, isopropyl, n-butyl, sec-butyl, iso-butyl, tert-butyl and the like.

Compounds (XI) where A^{XI} is -CH=CH- or -C=C-, X^{XI}, R₂^{XI} and R₃^{XI} are each H, n_{XI} is 1 and R₁^{XI} is a C₃-C₈ cycloalkyl, are especially preferred.

R¹ and R² are preferably selected as above-indicated in reference to formula (A).

Representative particularly preferred compounds are compounds 177, 178 or 179.

According to a twelfth aspect, it is herein described non-imidazole compounds which are analogous to those disclosed in WO 96/38141.

These compounds have the following formula (XII): 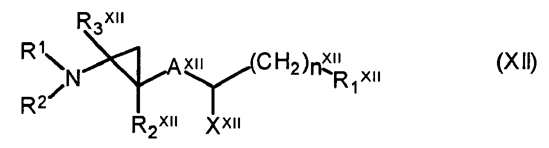 where R¹ and R² are as defined in reference to formula (A),
where R₂^{XII} is a hydrogen or a methyl or ethyl group;
R₃^{x}II is a hydrogen or a methyl or ethyl group;
n^{XII} is 0,1,2,3,4,5, or 6; and
R₁^{XII} is selected from the group consisting of (a) C₃ to C₈ cycloalkyl; (b) phenyl substituted or not by one or more groups such as a halogen atom, a lower alkyl or cycloalkyl, a trifluoromethyl, aryl, alkoxy, α-alkyloxyalkyl, aryloxy, nitro, formyl, alkanoyl, aroyl, arylalkanoyl, amino, carboxamido, cyano, alkyloximino, alkylalkoximino, aryloximino, α-hydroxyalkyl, alkenyl, alkynyl, sulphamido, sulfamoyl, sulphonamido, carboxamide, carbonylcycloalkyl, alkylcarbonylalkyl, carboalkoxy, arylalkyl or oxime group, or two substituants taken together with the carbon atoms of the phenyl ring to which it is fused form 5- or 6-membered saturated or unsaturated ring or a benzene ring ; (c) alkyl; (d) heterocyclic; (e) decahydronaphthalene; and (f) octahydroindene;
with the provisos that
when X^{XII} is H, A^{XII} can be -CH₂CH₂-, -COCH₂-, -CONH-, -CON(CH₃)-, -CH=CH-, -C=C-, -CH₂-NH-, -CH₂-N(CH₃)-, -CH(OH)CH₂-, -NH-CH₂-, -N(CH₃)-CH₂-, -CH₂O-, -CH₂S-, or -NHCOO-;
when X^{XII} is NH₂, NH(CH₃), N(CH₃)₂, OH, OCH₃, CH₃, SH or SCH₃; A^{XII} can be -NHCO-, -N(CH₃)-CO-, -NHCH₂-, -N(CH₃)-CH₂-, -CH=CH-, -COCH₂-, -CH₂CH₂-, -CH(OH)CH₂-, or -C=C- ; and
when R₁^{XII} and X^{XII} taken together denote a 5,6 or 6,6 saturated bicyclic ring structure X^{XII} can be NH, O, or S.

The term "alkyl" as used herein refers to straight or branched chain radicals derived from saturated hydrocarbons by the removal of one hydrogen atom. Representative examples of alkyl groups include methyl, ethyl, n-propyl, iso-propyl, n-butyl, sec-butyl, iso-butyl, tert-butyl, and the like.

The term "substituted phenyl" as used herein refers to a phenyl group substituted by one or more groups such as alkyl, halogen, amino, methoxy, and cyano groups.

The term "bicyclic alkyl" as used herein refers to an organic compound having two ring structures connected to an alkyl group. They may or may not be the same type of ring and the rings may be substituted by one or more groups. Representative bicyclic alkyl groups include adamanthyl, decahydronaphthalene and norbornane.

The cyclopropane attached to the NR¹R² moiety is preferably in trans configuration.

More preferably, it is described compounds of the general formula (Xll):
where A^{XII} is -CONH, -CH=CH-, -NHCOO-, or -C≡C-;
X^{XII} is H or NH₂;
R₂^{XII} and R₃^{XII} are H;
n^{XII} is 0, 1, 2 or 3;
R₁^{XII} is cyclohexyl, phenyl or substituted phenyl.
In compounds (XII), A^{XII} is especially -CH=CH- or -C≡C-;
R₂^{XII}, R₃^{XII} and X^{XII} are each especially a hydrogen atom;
n_{XII} is preferably 1 and R₁^{XII} is especially an alkyl group.
R¹ and R² are preferably selected as above-indicated with reference to formula (A).

Representative example of compounds (XII) is compound 180.

According to a thirteenth aspect, to the instant application describes non-imidazole compounds analogous to those disclosed in WO 95/11894.

Thus, the sub-class of compounds (A) comprises compounds having the following formula (XIII): 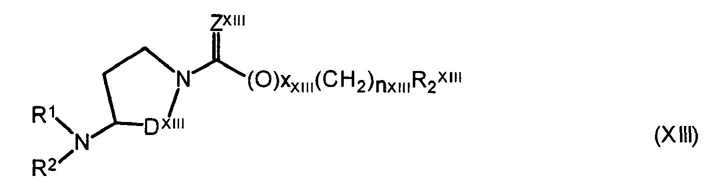 wherein R¹ and R² are as defined with reference to formula (A)
wherein D^{XIII} is CH₂ or CH₂-CH₂, Z^{XIII} represents sulfur (S) or oxygen (0), preferably 0, X_{XIII} is 0 or 1, n_{XIII} is an integer from 0 to 6,
and R₂^{XIII} represents a substituted or unsubstituted linear chain or branched chain alkyl group of up to about 20 carbon atoms, a substituted or unsubstituted carbocyclic group of up to about 20 carbon atoms including mono and bicyclic moieties, and a substituted or an unsubstituted aryl group of up to about 20 carbon atoms, or any combination of above-mentioned groups, or salts thereof and with the substituants being represented by one or more groups such as a halogen atom, a lower alkyl or cycloalkyl, a trifluoromethyl, aryl, alkoxy, α-alkyloxyalkyl, aryloxy, nitro, formyl, alkanoyl, aroyl, arylalkanoyl, amino, carboxamido, cyano, alkyloximino, alkylalkoximino, aryloximino, α-hydroxyalkyl, alkenyl, alkynyl, sulphamido, sulfamoyl, sulphonamido, carboxamide, carbonylcycloalkyl, alkylcarbonylalkyl, carboalkoxy, arylalkyl or oxime group, or two substituants taken together with the carbon atoms of the phenyl ring to which it is fused form 5- or 6-membered saturated or unsaturated ring or a benzene ring.

In a specific embodiment, R₂^{XIII} can represents a disubstituted methyl, such as but not limited to dicyclohexyl methyl (-CH(C₆H₁₁)₂), diphenyl methyl (-CH(C₆H₅)₂), and the like. If R₂^{XIII} is tert-butyl, cyclohexyl, or dicyclohexylmethyl, X_{XIII} or n_{XIII} must not be 0. If R₂^{XIII} is adamantane, the sum of X_{XIII} and n_{XIII} must be greater than 1.

In a preferred embodiment, D^{XIII} is CH₂-CH₂, resulting in a piperidine ring structure. However, it is contemplated that D^{XIII} can be CH₂, yielding a pyrrolidine ring structure. In yet another embodiment, D^{XIII} can be (CH₂)₃, yielding a cycloheptimide (seven membered heterocycle with one nitrogen).

In a specific embodiment, a tetramethylene bound to the amide or carbamate group is used. Preferably a cyclic alkyl or aryl group is linked to the amide or carbamate via the straight chain alkyl group. In a specific embodiment, tetramethylene cyclohexane (cyclohexylbutyl) is bound to an amide. Although specific hydrophobic alkyl and aryl groups have been mentioned, one of ordinary skill in the art will recognize that there are many possible hydrophobic groups for use in the compounds of the invention. These fall within the scope of the instant invention.

Thus, R₂^{XIII} can be one or more bulky substituent groups. As stated above, in a preferred aspect of the invention, the bulky substituents are removed from the amide or carbamate group on the piperidyl, by increasing n_{XIII}. In one embodiment, R₂^{XIII} is CHR₃^{XIII}R₄^{XIII}, in which n_{XIII} is 3 or 4 and R₃^{XIII} and R₄^{XIII} are cyclohexyl, phenyl, or the like. R₃^{XIII} and R₄^{XIII} can be the same group or different groups. In another embodiment, R₂^{XIII} is decalin or adamantane or the like. If R₂^{XIII} is adamantane, preferably n_{XIII} is greater than 1, but the sum of X_{XIII} and n_{XIII} must be greater than 1.

As used herein, the phrase linear chain or branched chained alkyl groups of up to about 20 carbon atoms means any substituted or unsubstituted acyclic carbon-containing compounds, including alkanes, alkenes and alkynes. Examples of alkyl groups include lower alkyl, for example, methyl, ethyl, n-propyl, iso-propyl, n-butyl, iso-butyl or tert-butyl; upper alkyl, for example, octyl, nonyl, decyl, and the like; and lower alkylene, for example, ethylene, propylene, propyldiene, butylene, butyldiene, and the like. The ordinary skilled artisan is familiar with numerous linear and branched alkyl groups, which are with the scope of the present invention.

In addition, such alkyl group may also contain various substituents in which one or more hydrogen atoms has been replaced by a functional group. Functional groups include but are not limited to hydroxyl, amino, carboxyl, amide, ester, ether, and halogen (fluorine, chlorine, bromine and iodine), to mention but a few.

As used herein, substituted and unsubstituted carbocyclic groups of up to about 20 carbon atoms means cyclic carbon-containing compounds, including but not limited to cyclopentyl, cyclohexyl, cycloheptyl, admantyl, and the like. Such cyclic groups may also contain various substituents in which one or more hydrogen atoms has been replaced by a functional group. Such functional groups include those described above, and lower alkyl groups as describe above. The cyclic groups of the invention may further comprise a heteroatom. For example, in a specific embodiment, R₂^{III} is cyclohexanol.

As used herein, substituted and unsubstituted aryl groups means a hydrocarbon ring bearing a system of conjugated double bonds, usually comprising six or more even number of *π* (pi) electrons. Examples of aryl groups include, by are not limited to, phenyl, naphthyl, anisyl, toluyl, xylenyl and the like. According to the present invention, aryl also includes heteroaryl groupss, e.g., pyrimidine or thiophene. These aryl groups may also be substituted with any number of a variety of functional groups. In addition to the functional groups described above in connection with substituted alkyl groups and carbocyclic groups, functional groups on the aryl groups can be nitro groups.

As mentioned above, R₂^{XIII} can also represents any combination of alkyl, carbocyclic or aryl groups, for example, 1-cyclohexylpropyl, benzyl cyclohexylmethyl, 2-cyclohexylpropyl, 2,2-methylcyclohexylpropyl, 2,2-methylphenylpropyl, 2,2-methylphenylbutyl.

In a specific embodiment, R₂ represents cyclohexane, and n_{XIII}=4 (cyclohexylvaleroyl). In another specific embodiment, R₂^{XIII} represents cinnamoyl.

Particularly preferred are compounds (XIII) wherein Z^{XIII} is an oxygen atom and wherein X_{XIII} is 0 or 1, n_{XIII} is an integer from 0 to 6, more preferably n_{XIII} = 3-6, and most preferably n_{XIII}=4, and R₂^{XIII}is as defined above. Examples of preferred alkyl groups for R₂^{XIII} include but are not limited to cyclopentyl, cyclohexyl, admantane methylene, dicyclohexyl methyl, decanyl and t-butyryl and the like. Examples of preferred aryl and substituted aryl groups include but are not limited to phenyl, aryl cyclohexyl methyl and the like.

Preferred R¹ and R² are selected as indicated with reference to formula (A).

Representative examples are compounds 123 and 176.

According to a fourteenth aspect, the application describes compounds analogous to those disclosed in WO 93/12107.

Thus, a sub-class of compounds (A) concerns compounds having the following formula (XIV) 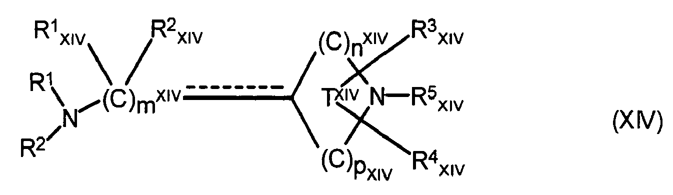 wherein R¹ and R² are as defined in reference of formula (A);
- (A): m_{XIV} is an integer selected from the group consisting of: 1 and 2;
- (B): n_{XIV} and p_{XIV} are intergers and are each independently selected from the group consisting of: 0, 1, 2, 3, and 4 such that the sum of n_{XIV} and p_{XIV} is 4 and T^{XIV} is a 6-membered ring;
- (C): R³_{XIV} and R⁴_{XIV} are each independently bound to the same or different carbon atom of ring T^{XIV} such that there is only one R³_{XIV} group and one R⁴_{XIV} group in ring T^{XIV}, and each R¹_{XIV}, R²_{XIV}, R³_{XIV} and R⁴_{XIV} is independently selected from the group consisting of:
(1) H;
(2) C₁ to C₆ akyl; and
(3) -(CH₂)_{qXIV}-R⁶_{XIV} wherein q_{XIV} is an integer of: 1 to 7, and R⁶_{XIV} is selected from the group consisting of: phenyl, substituted phenyl, -OR⁷_{XIV}, -C(O)OR⁷_{XIV}, -C(O)R⁷_{XIV}, -OC(O)R⁷_{XIV}, -C(O)NR⁷_{XIV}R⁸_{XIV}, CN and -SR⁷_{XIV} wherein R⁷_{XIV} and R⁸_{XIV} are as defined below, and wherein the substituents on said substituted phenyl are each independently selected from the group consisting of: -OH, -O-(C₁ to C₆)alkyl, halogen, C₁ to C₆ alkyl, -CF₃, -CN, and -NO₂, and wherein said substituted phenyl contains from 1 to 3 substituents;
- (D): R⁵_{XIV} is selected from the group consisting of:
(1) H;
(2) C₁ to C₂₀ alkyl;
(3) C₃ to C₆ cycloalkyl;
(4) -C(O)OR^{7'}_{XIV}; wherein R^{7'}_{XIV} is the same as R⁷_{XIV} defined below except that R^{7'}_{XIV} is not H;
(5) -C(O)R^{7'}_{XIV};
(6) -C(O)NR^{7'}_{XIV}R⁸_{XIV};
(7) allyl;
(8) propargyl; and
(9) -(CH₂)_{q}-R⁶_{XIV} wherein q_{XIV} and R⁶_{XIV} are as defined above, and when q_{XIV} is equal to 1, then R⁶_{XIV} is not OH or SH;
- (E): R⁷_{XIV} and R⁸_{XIV} are each independently selected from the group consisting of: H, C₁ to C₆ alkyl, and C₃ to C₆ cycloalkyl;
- (F): the dotted line (--------) represents a double bond that is optionally present when m_{XIV} is 1, and n_{XIV} is not 0, and p is not 0 (i.e., the nitrogen in the ring is not bound directly to the carbon atom bearing the double bond), and when said double bond is present then R²_{XIV} is absent; and
- (G): when m_{XIV} is 2, each R¹_{XIV} is the same or different substituent for each m_{XIV}, and each R²_{XIV} is the same or different substituent for each m_{XIV}, and at least two of the substituents R¹_{XIV} and/or R²_{XIV} are H.

Those skilled in the art will appreciate that the total number of substituents on each of the -(C)ₙ^{XIV}- and -(C)ₚ^{XIV}- groups is two, and that such substituents are independently selected from the group consisting of hydrogen, R³_{XIV} and R⁴_{XIV} such that there is a total of only one R³_{XIV} and one R⁴_{XIV} substituent in ring T^{XIV}.

As used herein the following terms have the following meanings unless indicated otherwise:
alkyl - represents a straight or branched, saturated hydrocarbon chain having from 1 to 20 carbon atoms;
cycloalkyl - represents a saturated carbocyclic ring having from 3 to 6 carbon atoms;
halogen (halo) - represents fluoro, chloro, bromo or iodo.

Preferably, for compounds of formula (XIV) m is 1; R⁵_{XIV} is selected from the group consisting of H and C₁ to C₁₅ alkyl; and R¹_{XIV} to R⁴_{XIV} are each independently selected from the group consisting of: H, C₁ to C₆ alkyl, and -(CH₂)_{qXIV}-R⁶_{XIV} wherein R⁶_{XIV} is phenyl. Most preferably, R⁵_{XIV} is selected from the group consisting of H and C₁ to C₆ alkyl with H and methyl being even more preferable; and R³_{XIV} and R⁴_{XIV} are each independently selected from the group consisting of: H and methyl.

Representative compounds include compounds of the formula: 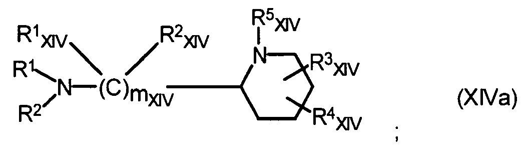 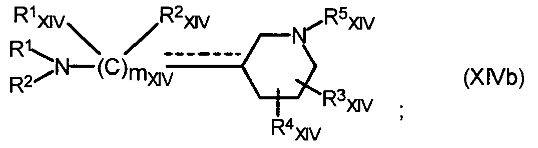 and

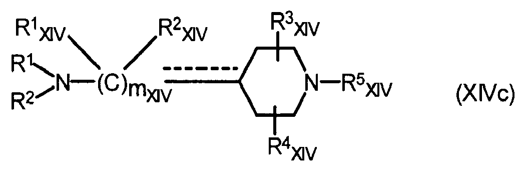

For formula (XIVa), (XIVb) or (XIVc), R⁵_{XIV} is preferably H or CH₃; R³_{XIV} and R⁴_{XIV} are preferably each an hydrogen atom.

Preferred R¹ and R² are as specified for formula (A).

According to a fifteenth aspect, the application describes to compounds analogous to those disclosed in WO 93/12108.

Thus, these compounds have the following formula (XV): 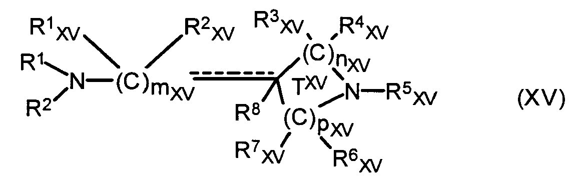 wherein R¹ and R² are as defined in reference to formula (A)
- (A): mₓᵥ is an integer selected from the group consisting of: 0,1, and 2;
- (B): nₓᵥ and pₓᵥ are integers and are each independently selected from the group consisting of: 0, 1, 2, and 3 such that the sum of n_{XV} and p_{XV} is 2 or 3 such that when the sum of n_{XV} and p_{XV} is 2, T^{XV} is a 4-membered ring and when the sum of n_{XV} and p_{XV} is 3, T^{XV} is a 5-membered ring;
- (C): each R¹_{XV}, R²_{XV}, R³_{XV}, R⁴_{XV}, R⁶_{XV}, R⁷_{XV} and R⁸_{XV} is independently selected from the group consisting of:
(1) H;
(2) C₁ to C₆ alkyl;
(3) C₃ to C₆ cycloalkyl; and
(4) -(CH₂)_{q}^{XV}-R⁹_{XV} wherein q_{XV} is an integer of: 1 to 7, and R⁹_{XV} is selected from the group consisting of: phenyl, substituted phenyl, -OR¹⁰_{XV}, -C(O)OR¹⁰_{XV}, -C(O)R¹⁰_{XV}, -OC(O)R¹⁰_{XV}, -C(O)NR¹⁰_{XV}R¹¹_{XV}, CN and -SR¹⁰_{XV} wherein R¹⁰_{XV} and R¹_{XV} are as defined below, and wherein the substituents on said substituted phenyl are each independently selected from the group consisting of: -OH, -O-(C₁ to C₆) alkyl, halogen, C₁ to C₆ alkyl, -CF₃, -CN, and -NO₂, and wherein said substituted phenyl contains from 1 to 3 substituents; examples of -(CH₂)_{qXV}-R⁹_{XV} include benzyl, substituted benzyl and the like, wherein the substitutents on the substituted benzyl are as defined above for said substituted phenyl;
- (D): R⁵_{XV} is selected from the group consisting of:
(1) H;
(2) C₁ to C₂₀ alkyl;
(3) C₃ to C₆ cycloalkyl;
(4) -C(O)OR^{10'}_{XV}; wherein R^{10'}_{XV} is the same as R¹⁰_{XV} defined below except that R^{10'}_{XV} is not H;
(5) -C(O)R¹⁰_{XV};
(6) -C(O)NR¹⁰_{XV}R¹¹_{XV};
(7) allyl;
(8) propargyl; and
(9) -(CH₂)_{q}^{XV}-R⁹_{XV}, wherein q_{XV} and R⁹_{XV} are as defined above with the proviso that when q_{XV} is 1 then R⁹_{XV} is not -OH or -SH;
- (E): R¹⁰_{XV} and R¹¹_{XV} are each independently selected from the group consisting of: H, C₁ to C₆ alkyl, and C₃ to C₆ cycloalkyl; and, for the substituent -C(O)NR¹⁰_{XV}R_{XV}¹¹, R¹⁰_{XV} and R¹¹_{XV}, together with the nitrogen to which they are bound, can form a ring having 5, 6, or 7 atoms;
- (F): the dotted line (-----) represents a double bond that is optionally present when m_{XV} is 1, and T^{XV} is a 5-membered ring, and n_{XV} is not 0, and p_{XV} is not 0 (i.e., the nitrogen in the ring is not bound directly to the carbon atom bearing the double bond), and when said double bond is present then R²ₓᵥ and R⁸ₓᵥ are absent;
- (G): when mₓᵥ is 2, each R¹_{XV} is the same or different substituent for each mₓᵥ, and each R²ₓᵥ is the same or different substituent for each mₓᵥ;
- (H): when nₓᵥ is 2 or 3, each R³ₓᵥ is the same or different substituent for each nₓᵥ, and each R⁴ₓᵥ is the same or different substituent for each nₓᵥ; and
- (I): when pₓᵥ is 2 or 3, each R⁶ₓᵥ is the same or different substituent for each p, and each R7ₓᵥ is the same or different substituent for each pₓᵥ.

As used herein the following terms have the following meanings unless indicated otherwise:
alkyl - represents a straight or branched, saturated hydrocarbon chain having from 1 to 20 carbon atoms;
cycloalkyl - represents a saturated carbocyclic ring having from 3 to 6 carbon atoms; and
halogen (halo) -represents fluoro, chloro, bromo or iodo.

Preferably, for compounds of formula (XV) m_{XV} is 0 or 1; R⁵_{XV} is selected from the group consisting of H and C₁ to C₂₀ alkyl; and R¹_{XV} to R⁴_{XV} and R⁶_{XV} to R⁸_{XV} are each independently selected from the group consisting of: H, C₁ to C₆ alkyl, and -(CH₂)_{qXV}-R⁹_{XV} wherein R⁹_{XV} is phenyl. Most preferably, R⁵_{XV} is selected from the group consisting of H and methyl; and R¹_{XV}, R²_{XV}, R³_{XV}, R4_{XV}, R⁶_{XV}, R⁷_{XV}, and R⁸_{XV} are each independently selected from the group consisting of: H, methyl, ethyl, pentyl, benzyl, and 2-phenylethyl.

Representative compounds include compounds of the formula: 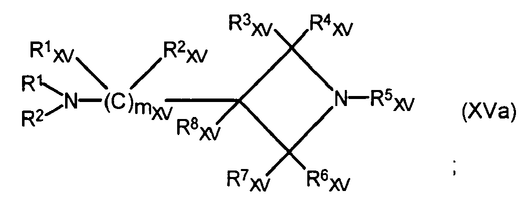 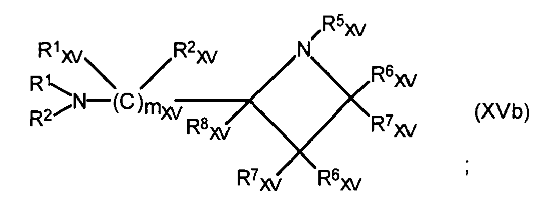 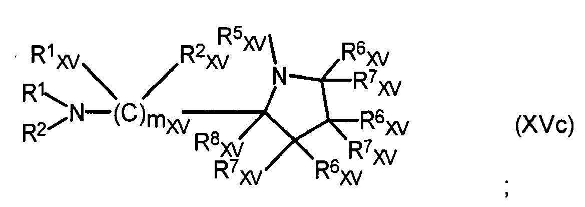 and 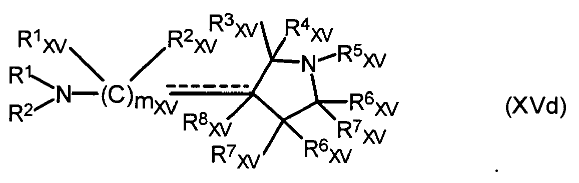 wherein m_{XV} and R¹_{XV} to R⁸_{XV} are as defined for formula (XV)

Compounds (XVc) or (XVd) are preferred.

Representative compounds (XVa) to (XVd) are those wherein R⁵_{XV} is H or CH₃.

Preferably, only one or two of substituents R³_{XV}, R⁴_{XV}, R⁶_{XV}, R⁷_{XV}, R⁸_{XV} is different from H and represents especially CH₃.

R¹ and R² are preferably selected as indicated in reference to formula (A).

According to a sixteenth aspect, the application describes to compounds analogous to those disclosed in WO 92/15567.

Thus, this sub-class of compounds (A) consists of compounds having the following formula (XVI) 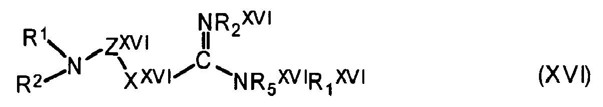 wherein R¹ and R² are as defined in reference to formula (A)
Z^{XVI} is a group of the formula (CH₂)_{mXVI} wherein m_{XVI} = 1-5 or a group of the formula: 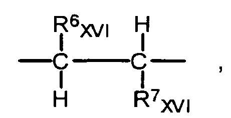 wherein R⁶_{XVI} = (C₁-C₃)alkyl R⁷_{XVI} = (C₁-C₃)alkyl ;
wherein Z^{XVI} may optionally comprise other substituents selected such that the activity of the derivative is not negatively affected,
X^{XVI} represents S, NH or CH₂
R¹_{XVI} represents hydrogen, (C₁-C₃)alkyl-, aryl(C₁-C₁₀)alkyl, wherein aryl may optionally be substituted, aryl, (C₅-C₇)cycloalkyl(C₁-C₁₀)alkyl-, or a group of the formula: 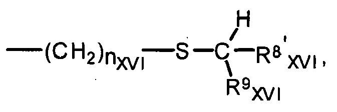 wherein n_{XVI} = 1-4, R⁸_{XVI} is aryl, aryl(C₁-C₁₀)alkyl-, (C₅-C₇)cycloalkyl- or (C₅-C₇) cycloalkyl(C₁-C₁₀)alkyl-, and R⁹_{XVI}, is hydrogen, (C₁-C₁₀)alkyl- or aryl; R₂^{XVI} and R₅ ^{XVI} represent hydrogen, (C₁-C₃)alkyl-, aryl or arylalkyl-, wherein aryl may optionally be substituted; wherein aryl is phenyl, substituted phenyl, naphthyl, substituted napththyl, pyridyl or substituted pyridyl;
R₂^{XVI} and R₅^{XVI} are preferably a hydrogen atom.
m_{XVI} is preferably 2 or 3
X^{XVI} is preferably S or NH
R₁^{XVI} is preferably selected from H or an optionally substituted aryl.

Preferred R¹ and R² are selected as specified for formula A.

According to a seventeenth aspect, a sub-class of compounds (A) comprises compounds having the following formula (XVII), which can be considered as analogousX to those disclosed in EP 680 960: 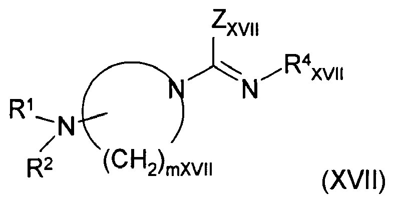 wherein m_{XVII} represents an integer of from 4 to 6.

R⁴_{XVII} represents a hydrogen atom, a linear or branched alkyl group, a cycloalkyl group, a cycloalkylalkyl group, a substituted or unsubstituted aryl group or a substituted or unsubstituted aralkyl group; and Z^{XVII} represents R⁵_{XVII} or A^{XVII}-R⁶_{XVII}, wherein A^{XVII} represents S or O, R₅^{XVII} represents a hydrogen atom, a lower alkyl group, a substituted or unsubstituted aryl group or a substituted or unsubstituted aralkyl group, and R₆^{XVII} represents a lower alkyl group, a lower alkenyl group, a lower alkynyl group or a substituted or unsubstituted aralkyl group;

The lower alkyl groups are preferably linear or branched alkyl groups having 1 to 6 carbon atoms. Specific examples thereof include methyl, ethyl, n-propyl, iso-propyl, n-butyl, iso-butyl, sec-butyl, tert-butyl, n-pentyl and n-hexyl groups.

The linear or branched alkyl groups are preferably those having 1 to 8 carbon atoms. Specific examples thereof include methyl, ethyl, n-propyl, iso-propyl, n-butyl, iso-butyl, sec-butyl, tert-butyl, n-pentyl, n-hexyl, 1,1-dimethylpropyl, 1,2-dimethylpropyl and 1,2,2-trimethylpropyl groups.

The cycloalkyl groups are preferably those having 3 to 10 carbon atoms. The cycloalkyl groups include not only monocycloalkyl groups (for example, cyclopentyl, cyclohexyl and cycloheptyl) but also polycycloalkyl groups (for example, bicycloalkyl and tricycloalkyl). Examples of the bicycloalkyl groups include norbornyl (for example, exo-2-norbornyl and endo-2-norbornyl), 3-pinanyl and bicyclo[2.2.2]oct-2-yl groups, while examples of the tricycloalkyl groups include adamantyl groups (for example, 1-adamantyl and 2-adamantyl). Such a cycloalkyl group may be substituted by alkyl group(s), etc.

The cycloalkylalkyl groups are preferably those composed of a cycloalkyl group having 3 to 10 carbon atoms with a linear or branched alkyl group having 1 to 3 carbon atoms. Specific examples thereof include 1-cyclohexylethyl and 1-cyclopropylethyl groups.

The lower alkenyl groups are preferably linear or branched alkenyl groups having 3 to 6 carbon atoms. Specific examples thereof include allyl, 1-methyl-2-propenyl, 2-methyl-2-propenyl, cis-2-butenyl, trans-2-butenyl and 3-methyl-2-butenyl groups.

The lower alkynyl groups are preferably those having 3 to 6 carbon atoms. A specific example thereof includes a 2-propynyl group.

The substituted aryl groups are preferably phenyl and naphthyl groups which may be substituted by halogen atoms and trifluoromethyl, lower alkyl, lower alkoxy, lower alkylthio, cyano and nitro groups.

Specific examples thereof include phenyl, 1-naphthyl, 2-chlorophenyl, 3-chlorophenyl, 4-chlorophenyl, 4-trifluoromethylphenyl, 3-fluorophenyl, 4-fluorophenyl, 2-methoxyphenyl, 4-methoxyphenyl, 2-tolyl and 3-tolyl groups.

The aralkyl groups are preferably benzyl, diarylmethyl and trityl groups.

The substituted aralkyl groups are preferably arylalkyl groups composed of a phenyl or naphthyl group, which may be substituted by halogen atoms and trifluoromethyl, lower alkyl, lower alkoxy, lower alkylthio, cyano and nitro groups, and a linear or branched alkyl group having 1 to 4 carbon atoms.

Specific examples thereof include benzyl, α-methylbenzyl, phenethyl, 3-phenylpropyl, 4-phenylbutyl, 4-chlorobenzyl, 4-fluorobenzyl, 4-methoxybenzyl, 4-chloro-α-methylbenzyl, 4-fluoro-amethylbenzyl and 4-methoxy-α-methylbenzyl groups.

Among the compounds represented by the general formula (XVII) preferable examples include those wherein:
m_{XVII} is from 4 to 6;
R⁴_{XVII} is a hydrogen atom; a linear or branched alkyl group having 1 to 8 carbon atoms, a cycloalkyl group having 3 to 10 carbon atoms, a cycloalkylalkyl group composed of a cycloalkyl moiety having 3 to 10 carbon atoms and an alkyl moiety having 1 to 3 carbon atoms, a substituted or unsubstituted aryl group or a substituted or unsubstituted aralkyl group carrying an alkyl moiety having 1 to 4 carbon atoms;
R⁵_{XVII} is a hydrogen atom, an alkyl group having 1 to 6 carbon atoms, a substituted or unsubstituted aryl group or a substituted or unsubstituted aralkyl group carrying an alkyl moiety having 1 to 4 carbon atoms; and
R⁶_{XVII} is an alkyl group having 1 to 6 carbon atoms, an alkenyl group having 3 to 6 carbon atoms, an alkynyl group having 3 to 6 carbon atoms or a substituted or unsubstituted aryl group.

Preferable examples of the compounds represented by the general formula (XVII) are those satisfying the following requirements:
(1) A compound wherein m^{XVII} is 5 and R¹, R² and R³ are each a hydrogen atom.
(2) A compound wherein R⁴_{XVII} is a cycloalkyl group, such as monocycloalkyl, bicycloalkyl and tricycloalkyl groups. A preferable example of the monocycloalkyl group is a cyclohexyl group. A preferable example of the bicycloalkyl group is a norbornyl group, more preferably a 2-exo-norbornyl group. A preferable example of the tricycloalkyl group is an adamantyl group, more preferably a 1-adamantyl group.
(3) A compound wherein R⁴_{XVII} is a substituted or unsubstituted phenyl group or a substituted or unsubstituted phenylalkyl group.
(4) A compound wherein R⁵_{XVII} is a hydrogen atom.
(5) A compound wherein A^{XVII} is S and R⁶_{XVII} is a lower alkyl group.
(6) A compound wherein a lower alkyl group is a methyl group.

R¹ and R² are preferably selected as specified for the formula (A).

According to a eighteenth aspect, the invention is directed to non imidazole compounds having the following formula (XVIII), analogous to those disclosed in Van der Goot et al. (Eur. J. Med. Chem. (1992) 27, 511-517): 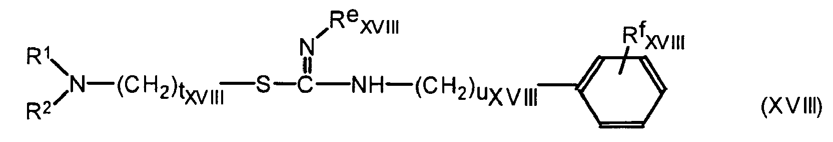 in which:
- R¹ and R² are as defined with reference to formula (A);
- R^{e}_{XVIII} is H, alkyl or cycloalkyl;
- R^{f}_{XVIII} is H or halogen, in particular Cl, F, Br, or an alkyl;
- t_{XVIII} is 1 to 3;
- u_{XVIII} is 1 to 4.

Preferred groups R¹ and R² are as defined with reference to formula (A).

Representative example is compound 122 and 167.

The W residue as defined in formula (A) and in particular as illustrated by formulae (I) to (XVIII), preferably contains no imidazole moiety attached in 4(5)-position and more preferably W contains no imidazole moiety.

The compounds may be prepared according to one of the schemes described in the international patent application WO 00/06254.

### Treatment of epilepsy

The compounds of formula (A) according to the invention have antagonistic and/or agonistic properties at the histamine H₃-receptors. They affect the synthesis and release of histamine monoamines or neuropeptides in brain and peripheral tissues.

The inventors have now clearly demonstrated that the H₃-receptor antagonists/inverse agonists as described herein, probably by virtue of their enhancement of histaminergic transmission in brain, constitute a novel class of antiepileptic drugs. One major interest of this new class lies in the fact that, in contrast with many conventional antiepileptics, the H₃-antagonists enhance vigilance and cognition, thus facilitating treatment of subjects during professional or car driving activities.

The invention thus provides a method of treatment of epilepsy comprising administering a patient in need thereof with a therapeutically effective amount of a compound of formula (A), as described above, optionally in combination with a therapeutically acceptable vehicle or excipient.

The invention also relates to the use of a compound of formula (A) for the manufacture of a medicament intended for the treatment of epilepsy.

Preferably, a compound of formula (A) intended for the treatment of epilepsy is a compound of formula (I) to (XVIII).

Still preferably,a method of treatment of epilepsy comprises administering a patient in need thereof with a therapeutically effective amount of at least one following compounds:
1-(5-phenoxypentyl)-piperidine
1-(5-phenoxypentyl)-pyrrolidine
N-methyl-N-(5-phenoxypentyl)-ethylamine
1-(5-phenoxypentyl)-morpholine
N-(5-phenoxypentyl)-hexamethyleneimine
N-ethyl-N-(5-phenoxypentyl)-propylamine
1-(5-phenoxypentyl)-2-methyl-piperidine
1-(5-phenoxypentyl)-4-propyl-piperidine
1-(5-phenoxypentyl)-4-methyl-piperidine
1-(5-phenoxypentyl)-3-methyl-piperidine
1-acetyl-4-(5-phenoxypentyl)-piperazine
1-(5-phenoxypentyl)-3,5-trans-dimethyl-piperidine
1-(5-phenoxypentyl)-3,5-cis-dimethyl-piperidine
1-(5-phenoxypentyl)-2,6-cis-dimethyl-piperidine
4-carboethoxy-1-(5-phenoxypentyl)-piperidine
3-carboethoxy-1-(5-phenoxypentyl)-piperidine
1-[3-(4-cyclopropylcarbonylphenoxy) propyl]-piperidine
1-[3-(4-acetylphenoxy)-2-R-methylpropyl] piperidine
1-[3-(4-cyanophenoxy)propyl]-4-methylpiperidine
1-[3-(4-cyanophenoxy)propyl]-3-methylpiperidine
1-[3-(4-acetylphenoxy)-2-S-methylpropyl] piperidine
1-{3-[4-(3-oxobutyl)phenoxy] propyl}piperidine
1-[3-(4-cyano-3-fluorophenoxy)propyl] piperidine
1-[3-(4-nitrophenoxy)propyl]-3-methylpiperidine
1-[3-(4-cyanophenoxy)propyl]-2-methylpiperidine
1-[3-(4-nitrophenoxy)propyl]-2-methylpiperidine
1-[3-(4-nitrophenoxy)propyl]-4-methylpiperidine
1-[3-(4-cyanophenoxy)propyl]-2,6-dimethylpiperidine
1-[3-(4-propionylphenoxy)propyl]-3-methylpiperidine
1-[3-(4-cyclobutylcarbonylphenoxy)propyl] piperidine
1-[3-(4-cyclopentylcarbonylphenoxy) propyl]piperidine
1-[3-(4-cyanophenoxy)propyl]-cis-2-methyl-5-ethylpiperidine
1-[3-(4-cyanophenoxy)propyl]-trans-2-methyl-5-ethylpiperidine
1-[3-(4-cyanophenoxy)propyl]-cis-3,5-dimethylpiperidine
1-[3-(4-propionylphenoxy)propyl]-4-methylpiperidine
1-[3-(4-propionylphenoxy)propyl]-2-methylpiperidine
1-{3-[4-(1-hydroxypropyl)phenoxy]propyl}-3-methylpiperidine
1-{3-[4-(1-hydroxypropyl)phenoxy]propyl}-4-methylpiperidine
1-[3-(4-propionylphenoxy)propyl]-2-methylpiperidine
1-[3-(4-propionylphenoxy)propyl]-4-methylpiperidine methoxime
1-[3-(4-cyanophenoxy)propyl]-trans-3,5-dimethylpiperidine
1-[3-(4-cyclopropylcarbonylphenoxy)propyl]-trans-3,5-dimethyl piperidine
1-[3-(4-cyclopropylcarbonylphenoxy) propyl] -cis-3,5-dimethyl piperidine
1-[3-(4-carbomethoxyphenoxy)propyl] piperidine
1-[3-(4-propenylphenoxy)propyl]-2-methyl piperidine
1-[3-(4-propionylphenoxy)propyl]-2-methylpiperidine
1-{3-[4-(1-ethoxypropyl)phenoxy]propyl}-2-methyl piperidine
1-[3-(4-propionylphenoxy)propyl]-4-methylpiperidine
1-[3-(4-bromophenoxy)propyl]piperidine
1-[3-(4-nitrophenoxy)propyl]piperidine
1-[3-(4-N,N-dimethylsulfonamidophenoxy) propyl]piperidine
1-[3-(4-isopropylphenoxy)propyl]piperidine
1-[3-(4-sec-butylphenoxy)propyl]piperidine
1-[3-(4-propylphenoxy)propyl]piperidine
1-[3-(4-ethylphenoxy)propyl]piperidine
1-(5-phenoxypentyl)-1,2,3,6-tetrahydropyridine
1-[5-(4-nitrophenoxy)-pentyl]-pyrrolidine
1-[5-(4-chlorophenoxy)-pentyl]-pyrrolidine
1-[5-(4-methoxyphenoxy)-pentyl]-pyrrolidine
1-[5-(4-methylphenoxy)-pentyl]-pyrrolidine
1-[5-(4-cyanophenoxy)-pentyl]-pyrrolidine
1-[5-(2-naphthyloxy)-pentyl]-pyrrolidine
1-[5-(1-naphthyloxy)-pentyl]-pyrrolidine
1-[5-(3-chlorophenoxy)-pentyl]-pyrrolidine
1-[5-(4-phenylphenoxy)-pentyl]-pyrrolidine
1-{5-[2-(5,6,7,8-tetrahydronaphthyl)-oxy]-pentyl}-pyrrolidine
1-[5-(3-phenylphenoxy)-pentyl]-pyrrolidine
1-(5-phenoxypentyl)-2,5-dihydropyrrole
1-{5-[1-(5,6,7,8-tetrahydronaphthyl)-oxy]-pentyl}-pyrrolidine
1-(4-phenoxybutyl)-pyrrolidine
1-(6-phenoxyhexyl)-pyrrolidine
1-(5-phenylthiopentyl)-pyrrolidine
1-(4-phenylthiobutyl)-pyrrolidine
1-(3-phenoxypropyl)-pyrrolidine
1-[5-(3-nitrophenoxy)-pentyl]-pyrrolidine
1-[5-(4-fluorophenoxy)-pentyl]-pyrrolidine
1-[5-(4-nitrophenoxy)-pentyl]-3-methyl-piperidine
1-[5-(4-acetylphenoxy)-pentyl]-pyrrolidine
1-[5-(4-aminophenoxy)-pentyl]-pyrrolidine
1-[5-(3-cyanophenoxy)-pentyl]-pyrrolidine
N-[3-(4-nitrophenoxy)-propyl]-diethylamine
N-[3-(4-cyanophenoxy)-propyl]-diethylamine
1-[5-(4-benzoylphenoxy)-pentyl]-pyrrolidine
1-{5-[4-(phenylacetyl)-phenoxy]-pentyl}-pyrrolidine
N-[3-(4-acetylphenoxy)-propyl]-diethylamine
1-[5-(4-acetamidophenoxy)-pentyl]-pyrrolidine
1-[5-(4-phenoxyphenoxy)-pentyl]-pyrrolidine
1-[5-(4-N-benzamidophenoxy)-pentyl]-pyrrolidine
1-{5-[4-(1-hydroxyethyl)-phenoxy]-pentyl}-pyrrolidine
1-[5-(4-cyanophenoxy)-pentyl]-diethylamine
1-[5-(4-cyanophenoxy)-pentyl]-piperidine
N-[5-(4-cyanophenoxy)-pentyl]-dimethylamine
N-[2-(4-cyanophenoxy)-ethyl]-diethylamine
N-[3-(4-cyanophenoxy)-propyl]-dimethylamine
N-[4-(4-cyanophenoxy)-butyl]-diethylamine
N-[5-(4-cyanophenoxy)-pentyl]-dipropylamine
1-[3-(4-cyanophenoxy)-propyl]-pyrrolidine
1-[3-(4-cyanophenoxy)-propyl]-piperidine
N-[3-(4-cyanophenoxy)-propyl]-hexamethyleneimine
N-[6-(4-cyanophenoxy)-hexyl]-diethylamine
N-[3-(4-cyanophenoxy)-propyl]-dipropylamine
N-3-[4-(1-hydroxyethyl)-phenoxy]-propyl-diethylamine
4-(3-diethylaminopropoxy)-acetophenone-oxime
1-[3-(4-acetylphenoxy)-propyl]-piperidine
1-[3-(4-acetylphenoxy)-propyl]-3-methyl-piperidine
1-[3-(4-acetylphenoxy)-propyl]-3,5-trans-dimethyl-piperidine
1-[3-(4-acetylphenoxy)-propyl]-4-methyl-piperidine
1-[3-(4-propionylphenoxy)-propyl]-piperidine
1-[3-(4-acetylphenoxy)-propyl]-3,5-cis-dimethyl-piperidine
1-[3-(4-formylphenoxy)-propyl]-piperidine
1-[3-(4-isobutyrylphenoxy)-propyl]-piperidine
N-[3-(4-propionylphenoxy)-propyl]-diethylamine
1-[3-(4-butyrylphenoxy)-propyl]-piperidine
1-[3-(4-acetylphenoxy)-propyl]-1,2,3,6-tetrahydropyridine
α-(4-Acetylphenoxy)-α'-(4-methylpiperidino)p-xylol
α-(4-Acetylphenoxy)-α'-(3, 5-cis-d imethylpiperid ino)p-xylol
α-(4-Acetylphenoxy)-α'-(3,5-trans-dimethylpiperidino)p-xylol
α-(4-Acetylphenoxy)-α'-(2-methylpyrrolidino)p-xylol
α-(4-Cyclopropylcarbonylphenoxy)-α'-piperidino-p-xylol
α-(4-Cyclopropylcarbonylphenoxy)-α'-(4-methylpiperidino)p-xylol
α-(4-Cyclopropylcarbonylphenoxy)-α'-pyrrolidino-p-xylol
3-Phenylpropyl 3-(4-methylpiperidino)propyl ether
3-Phenylpropyl 3-(3,5-cis-dimethylpiperidino)propyl ether
3-Phenylpropyl 3-(3,5-*trans*-dimethylpiperidino)propyl ether
3-Phenylpropyl 3-(3-methylpiperidino)propyl ether
3-Phenylpropyl 3-pyrrolidinopropyl ether
3-(4-Chlorophenyl)propyl 3-(4-methylpiperidino)propyl ether
3-(4-Chlorophenyl)propyl 3-(3,5-cis-dimethylpiperidino)propyl ether
3-(4-Chlorophenyl)propyl3-(3,5-trans-dimethylpiperidino)propyl ether
4-(6-Piperidinohexylamino)quinoline
2-Methyl 4-(3-piperidinopropylamino)quinoline
2-Methyl 4-(6-piperidinohexylamino)quinoline
7-Chloro-4-(3-piperidinopropylamino)quinoline
7-Chloro-4-(4-piperidinobutylamino)quinoline
7-Chloro-4-(8-piperidinooctylamino)quinoline
7-Chloro-4-(10-piperidinodecylamino)quinoline
7-Chloro-4-(12-piperidinododecylamino)quinoline
7-Chloro-4-(4-(3-piperidinopropoxy)phenylamino)quinoline
7-Chloro-4-(2-(4-(3-piperidinopropoxy)phenyl)ethylamino)quinoline
4-(6-Piperidinohexanoyl)phenyl 3-piperidinopropyl ether
5-Nitro-2-(5-piperidinopentylamino)pyridine
3-Nitro-2-(6-piperidinopentylamino)pyridine
5-Amino-2-(6-piperidinopentylamino)pyridine
2-(6-Piperidinohexylamino)quinoline
*N*-(4-Chlorobenzyl)-*N*'-cyclohexyl-3-piperidinopropyl isothiourea
2-(6-Piperidinohexylamino)benzothiazole
10-Piperidinodecylamine
3-Phenylpropyl 3-(*N*,*N*-diethylamino)propyl ether
N-(3-(N,N-Diethylamino)propyl)N'-phenylurea
N-Cyclohexylmethyl-N'-(3-piperidinopropyl)guanidine
N-(4-Bromobenzyl)-N'-(4-piperidinobutyl)sulphamide
3-Chloro-N-(4-piperidinobutyl)-N-methyl-benzene sulphonamide
N-(4-Chlorobenzyl)-2-(4-piperidinomethyl) phenyl) ethan amidine
1-(5-Cyclohexylpentanoyl)-1,4-bipiperidine
cis-1-(6-Cyclohexyl-3-hexen-1-yl)piperidine
trans-1-(6-Cyclohexyl-3-hexen-1-yl)piperidine
1-(2-(5, 5-Dimethyl-1-hexin-1-yl)cyclopropyl)piperidine.

According to a preferred embodiment, the method of treatment according to the invention comprises administering a patient in need thereof with a therapeutically effective amount of 3-(4-Chlorophenyl)propyl 3-piperidinopropyl ether, optionally in combination with a therapeutically acceptable vehicle or excipient.

The invention further relates to the use of 3-(4-Chlorophenyl)propyl 3-piperidinopropyl ether for the manufacture of a medicament intended for the treatment of epilepsy.

As used herein, "epilepsy" denotes a brain disorder in which clusters of nerve cells, or neurons, in the brain sometimes signal abnormally. Epilepsy is also known as a seizure disorder. A seizure is a sudden surge of electrical activity in the brain. Epilepsy is usually diagnosed after a person has had at least two seizures that were not caused by some known medical condition like alcohol withdrawal or extremely low blood sugar.

Preferably, epilepsy is selected from the group consiting of absence epilepsy, in children and adults, pharmaco-resistant temporal lobe seizures, and photosensitive seizures.

"Pharmaceutically" or "pharmaceutically acceptable" refer to molecular entities and compositions that do not produce an adverse, allergic or other untoward reaction when administered to an animal, or a human, as appropriate.

As used herein, "pharmaceutically acceptable carrier" includes any diluents, adjuvants, excipients, or vehicles, such as preserving agents, fillers, disintegrating agents, wetting agents, emulsifying agents, suspending agents, solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

In the context of the invention, the term "treating" or "treatment", as used herein, means reversing, alleviating, inhibiting the progress of, or preventing the disorder or condition to which such term applies, or one or more symptoms of such disorder or condition.

"Therapeutically effective amount" means an amount of a compound/medicament according to the present invention effective in producing the desired therapeutic effect.

According to the invention, the term "patient", or "patient in need thereof", is intended for a human or non-human mammal affected or likely to be affected with a neuropsychological disorder. Preferably, the patient is a human.

The compound or medicament according to the invention can be administered via oral, parenteral or topical routes, the active ingredient being combined with a therapeutically suitable excipient or vehicle.

According to the invention, oral administration of the compound or medicament in an appropriate formulation is advantageously used. Formulations which are suitable to be administered orally to a patient include discrete units such as capsules, cachets or tablets each containing a predetermined amount of the compound of formula (A); they also include a powder or granules; as solution or a suspension in an aqueous liquid or a nonaqueous liquid; or as an oil-in-water liquid emulsion or a water-in-oil liquid emulsion.

Actual dosage levels of compounds of formula (A) of the invention may be varied so as to obtain an amount of active ingredient that is effective to obtain a desired therapeutic response for a particular composition and method of administration. The selected dosage level therefore depends upon the desired therapeutic effect, on the route of administration, on the desired duration of treatment and other factors, e.g. the condition of the patient.

Total daily dose of the compounds useful according to this invention administered to a host in single or divided doses may be in amounts, for example, of from about 0.001 to about 100 mg/kg body weight daily and preferably 0.01 to 10 mg/kg/day. A suitable effective dose will be in general in the range of from 10 to 500 mg per day and of from 1 to 10 mg/day for particularly active compounds.

Dosage unit compositions may contain such amounts of such submultiples thereof as may be used to make up the daily dose. It will be understood, however, that the specific dose level for any particular patient will depend upon a variety of factors including the body weight, general health, sex, diet, time and route of administration, rates of absorption and excretion, combination with other drugs and the severity of the particular disease being treated.

These doses are given on the basis of the compound and should be adapted for the salts, hydrates or hydrated salts thereof.

The amount of each component administered is determined by the attending clinicians taking into consideration the etiology and severity of the disease, the patient condition and age, the potency of each component and other factors.

The invention is now illustrated by the following examples.

### EXAMPLES

### Example 1: Efficiency of a H₃ antagonist in a rat model of absence epilepsy

In a rat genetic model of human epilepsy (particularly of "absence" epilepsy in children and adults), the GAERS (Genetic Absence Epilepsy, Rat from Strasbourg; Vergnes et al, Epilepsy Res. 1989; 4, 8-13), 3-(4-Chlorophenyl)propyl 3-piperidinopropyl ether diminished by up to 77% the number and duration of spike-and-wave discharges, i.e. characteristic EEG changes of the disorder, at a dose of 20 mg/kg. In this strain of Wistar rats with spontaneous generalized non convulsive seizures (absence seizures), seizures are characterized by bilateral and synchronous spike-and-wave discharges (7-9 Hz) on EEG, concomitant with behavioural arrests. These discharges generally last about 20 sec and occur spontaneously every minute when the animals are in a state of quiet wakefulness. Pharmacological reactivity of this model is similar to human absence-epilepsy (e.g., valproate and ethosuccimide are protective). After a recovery period, implanted rats (cortical and hippocampal EEG electrodes) were recorded over a 20 min reference period. Then, 3-(4-Chlorophenyl)propyl 3-piperidinopropyl ether (5 or 20 mg/kg, i.p.) or saline were administered (n=8 per group) and the EEG recording was continued for 60 min. Rats received the alternate treatment one week later. Cumulated duration of absence-seizures was measured by 20 min periods during the two sessions. Fast Fourier transform analysis of EEG recordings allowed detection of any rhythm change during both ictal and inter-ictal periods (background activity. At 20 mg/kg, there was a total suppression of spike and wave discharges at 20 min and a nearly total suppression at 1 h.

### Example 2: Efficiency of a H₃ antagonist in a mice model of pharmacoresistant epilepsy

In intrahippocampal kainate-induced temporal lobe seizures in mice, a model of pharmacoresistant epilepsy in humans (Riban et al, J. Pharmacol. Exp. Ther. 2002; 112, 101), the same H₃-receptor antagonist at doses of 10 or 20 mg/kg reduced significantly the frequency of discharges. Interestigly, these changes occurred without any significant modification of the interictal EEG profiles, which excludes a non specific sedative effect. Unitalateral injection of kainic acid (1 nmol in 50 nl) into the dorsal hippocampus in mice induces a non-convulsive status that results in spontaneous recurrent focal seizures after 2-3 weeks. Seizures (5 to 20 times per hour in quiet mice) are characterized by a behavioural arrest and/or stereotypies, concomitant with spike and poly-spike discharges recorded in the injected hippocampus. All antiepileptic drugs tested in this model (valproate, carbamazepine, phenytoin, levetiracetam) are without significant effects, except benzodiazepines which, only transiently, suppress seizures. This model reproduces the behavioural, EEG, pharmacological and histological characteristics of mesial temporal lobe epilepsy, a form of epilepsy which is often drug-resistant in humans. After a recovery period, implanted mice (for EEG recordings) were injected with kainic acid. They were EEG recorded at least 3 weeks after injection for selection of animals with consistent hippocampal seizure. Then, after a 20 min reference recording period, selected mice received either 3-(4-Chlorophenyl)propyl 3-piperidinopropyl ether (10 or 20 mg/kg i.p.) or saline and the recording was continued for 60 min. Treatments were given in a counter balanced order (after a one week washout period between two sessions). The suppressive effects observed in kainite mice at dose of 10 mg/kg suggest that 3-(4-Chlorophenyl)propyl 3-piperidinopropyl ether could be effective on temporal lobe seizures, a form of seizures which is generally drug resistant. In this model, only benzodiazepines have been shown to suppress seizures whereas conventional antiepileptic drugs have no effects. 3-(4-Chlorophenyl)propyl 3-piperidinopropyl ether is the first compound able to stop hippocampal seizures in this model, reducing both the number and duration of seizures.

### Example 3: Drug tolerance in humans

In healthy human volunteers, orally-administered 3-(4-Chlorophenyl)propyl 3-piperidinopropyl ether dose-dependently elicited electroencephalographic changes (i.e. enhanced high-frequency cortical rhythms at the expense of low-frequency rhythms) which are regarded as predictory of facilitated thalamocortical activation and inhibition of pathological synchronisation underlying spike-wave discharges in epileptic states (Avanzini et al, Clinical Neurophysiol, 2000, 111, Suppl 2, S19). Groups of 6 male subjects received 40-120 mg 3-(4-Chlorophenyl)propyl 3-piperidinopropyl ether orally. EEG were recorded both on anterior and posterior leads and frequency distributions analysed by Fourier transform significant enhancement of fast activities were recorded particularly at anterior leads, the effect showing dose-dependency from 40 to 120 mg. After receiving a 40 mg-dose for 1 week a group of 6 subjects showed an enhancement of the response observed on single administration.

### Example 4: Treatment of photosensitive seizures in human

In a series of human epileptic subjects prone to photosensitive seizures remaining in spite of their treatment with various commercially available antiepileptic drugs, 3-(4-Chlorophenyl)propyl 3-piperidinopropyl ether dose-dependently suppressed (or at least enhanced the threshold of) EEG changes preceding convulsions that were experimentally triggered by repetitive photic stimulation. Photosensitivity, defined as a generalized epileptiform reaction on intermittent photic stimulation (IPS) outlasting the stimulus train is found in about 5 % of epileptic patients (Kasteleijn-Nolst trenite DGA. Acta Neurol Scand, 1989; 80:1-149). Unlike most other epilepsies, photosensitive epilepsy is a reflex epilepsy and epileptiform discharges can be evoked at any time by IPS in the laboratory. By determination of both upper and lower sensitivity limits (frequencies per flash) a so-called photosensitivity range can be determined. This range is related to liability of seizures in daily life of the patient. This photosensitivity range is relatively stable within a patient and can be diminished of abolished by antiepileptic medication. Thus the technique of using the photosensitivity range proved therefore to be a good model to study the antiepileptic properties of a single dose of an experimental drug in humans in early clinical development. Furthermore, it is possible to explore the time, onset and duration of the effect, a dose-response relationship and to document serum concentration-time profiles of the study drug. Hence, the goal of this single-blind study was to evaluate the anti epileptic effect of 3-(4-Chlorophenyl)propyl 3-piperidinopropyl ether (single dose) in the human model predictive of generalized absence epilepsy. The single blind design was chosen in order to reduce comparison bias of IPS response observed after treatment with 3-(4-Chlorophenyl)propyl 3-piperidinopropyl ether administration at Day 2 with placebo evaluation at Day 1. Five single doses will be studied (10 to 90 mg). the margin of safety was determined from the nonclinical and clinical experiments with 3-(4-Chlorophenyl)propyl 3-piperidinopropyl ether. To date, twelve patients have been enrolled:

Four patients received the dose of 20 mg. Among them one patient showed total suppression of photo paroxymal response (PPR) lasting 6 hours.

Four patients received the dose of 40 mg. Among them one showed partial suppression of PPR and one total suppression of PPR.

Four received the dose of 60 mg, with clinical response in all four patients. Moreover, two patients showed total suppression of PPR. The effect is appearing 1-2 hours post administration and is lasting more than 8 hours up to 36 hours (one patient)

Interestingly, most of the patients were under treatment with conventional antiepileptic drugs (Depakine,Tegretol, Keppra, Lamictal) which had not prevented the persistence of photosensitivity. The addition of the H3 antagonist to these treatments was well tolerated , thus demonstrating the feasibility of "add on" therapies.

One major interest of the new class lies also in the fact that, in contrast with many conventional antiepileptics, the H3-antagonists enhance vigilance and cognition, thus facilitating treatment of subjects during professional or car driving activities.

## Claims

1. Use of a compound having the general formula (A): 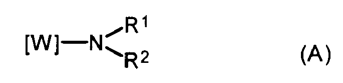 in which:
- W is a residue which imparts antagonistic and/or agonistic activity at histamine H₃-receptors when attached to an imidazole ring in 4(5)-position;
- R¹ and R² may be identical or different and represent each independently
• a lower alkyl or cycloalkyl, or taken together with the nitrogen atom to which they are attached,
• a saturated nitrogen-containing ring 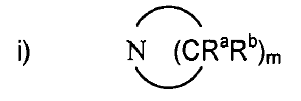 with m ranging from 2 to 8, or
• a non-aromatic unsaturated nitrogen-containing ring 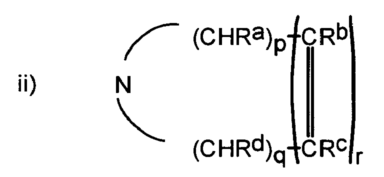 with p and q being from 0 to 3 independently and r being from 0 to 4, provided that p and q are not simulteously 0 and 2≤ p + q + r ≤ 8,
R^{a-d} being independently a hydrogen atom or a lower alkyl, cycloalkyl, or carboalkoxy group, or
• a morpholino group, or
• a N-substituted piperazino group: 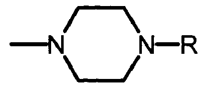 with R being a lower alkyl, cycloalkyl, carboalkoxy, aryl, arylalkyl, an alkanoyl or aroyl group,
as well as their pharmaceutically acceptable salts, their hydrates, their hydrated salts, the polymorphic crystalline structures of these compounds and their optical isomers, racemates, diastereoisomers and enantiomers, for the preparation of a medicament intended for the treatment of epilepsy.

2. Use according to claim 1, in which R¹ and R² are independently a lower alkyl group.

3. Use according to claim 2, in which R¹ and R² are each an ethyl group.

4. Use according to claim 1, in which -NR¹R² is a saturated nitrogen-containing ring: 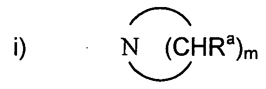 m being as defined in claim 1.

5. Use according to claim 4, **characterized in that** m is 4, 5 or 6.

6. Use according to claim 5, **characterized in that** -NR¹R² represents a piperidyl group.

7. Use according to claim 5, **characterized in that** -NR¹R² represents a pyrrolidinyl group.

8. Use according to claim 1, **characterized in that** -NR¹R² is a non-aromatic unsaturated nitrogen-containing ring: 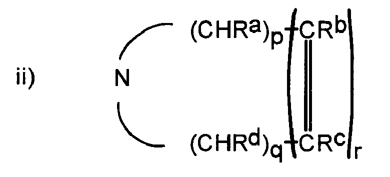 R^{a-d} and p, q and r being as defined in claim 1.

9. Use according to claim 8, **characterized in that** p, q and r are 1 or 2, more preferably p is 2 and q and r are 1.

10. Use according to anyone of claims 4 to 9, **characterized in that** R^{ad} represents each an hydrogen atom.

11. Use according to anyone of claim 4 to 9, **characterized in that** the nitrogen-containing ring i) or ii) is substituted, preferably mono- or di-substituted, more preferably mono-substituted, with an alkyl group.

12. Use according to claim 11, **characterized in that** the nitrogen-containing ring is mono-substituted with a methyl group.

13. Use according to anyone of claims 11 and 12, **characterized in that** the substituent(s) is(are) in meta-position with respect to the nitrogen atom.

14. Use according to claim 1, **characterized in that** -NR¹R² is a morpholino group.

15. Use according to claim 1, **characterized in that** -NR¹R² is a N-substituted piperazino group, preferably N-acetylpiperazino.

16. Use according to anyone of claims 1 to 15, of general formula (I): 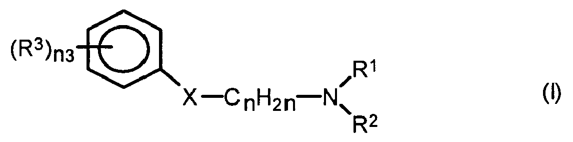 in which:
- CₙH₂n is a linear or branched hydrocarbon chain with n ranging from 2 to 8;
- X is an oxygen or sulfur atom;
- R¹ and R² are as defined in claim 1;
- n₃ is an integer from 0 to 5 ; and
- R³ represents each independently
• a halogen atom,
• a lower alkyl or cycloalkyl, a trifluoromethyl, aryl, alkoxy, α-alkyloxyalkyl, aryloxy, nitro, formyl, alkanoyl, aroyl, arylalkanoyl, amino, carboxamido, cyano, alkyloximino, aryloximino, alkylalkoximino, α-hydroxyalkyl, alkenyl, alkynyl, sulphamido, sulfamoyl, sulphonamido, carboxamide, carbonylcycloalkyl, alkylcarbonylalkyl, carboalkoxy, arylalkyl or oxime group,
• or taken together with the carbon atoms of the phenyl ring to which it is fused, a 5- or 6-membered saturated or unsaturated ring or a benzene ring.

17. Use according to claim 16, **characterized in that** n₃ is zero.

18. Use according to anyone of claims 16 and 17, **characterized in that** n₃ is 1 with R³ being as defined in claim 1 and preferably in para-position.

19. Use according to anyone of claims 16 and 18, **characterized in that** R³ is a lower alkyl, preferably a C₁-C₄ alkyl.

20. Use according to anyone of claims 16 and 18, **characterized in that** R³ is a halogen atom, a cyano, nitro, alkanoyl, alkyloximino or hydroxyalkyl, preferably CN, NO₂, COCH₃, COC₂H₅, H₃C-C=N-OH or H₃C-CHOH or cycloalkyl-CO.

21. Use according to claim 16, **characterized in that** R³ taken together with the carbon atoms of the phenyl group to which it is fused, form a 5- or 6-membered saturated or unsaturated ring, in particular a 5,6,7,8-tetrahydronaphthyl group.

22. Use according to claim 16, **characterized in that** R³ taken together with the phenyl group to which it is fused, form a naphthyl group.

23. Use according to anyone of claims 16 to 22, **characterized in that** - CₙH₂ₙ- is a linear hydrocarbon chain -(CH₂)ₙ-, n being as defined in claim 16.

24. Use according to anyone of claims 16 to 23, **characterized in that** X is an oxygen atom.

25. Use according to anyone of claims 16 to 23, **characterized in that** X is a sulfur atom.

26. Use according to anyone of claims 16 to 25, **characterized in that** n is varying from 3 to 5 and is preferably 3.

27. Use according to anyone of claims 16 to 26, **characterized in that** it is one of the following compounds:
1-(5-phenoxypentyl)-piperidine
1-(5-phenoxypentyl)-pyrrolidine
N-methyl-N-(5-phenoxypentyl)-ethylamine
1-(5-phenoxypentyl)-morpholine
N-(5-phenoxypentyl)-hexamethyleneimine
N-ethyl-N-(5-phenoxypentyl)-propylamine
1-(5-phenoxypentyl)-2-methyl-piperidine
1-[3-(4-cyclopropanecarbonylphenoxy) propyl]-piperidine
1-[3-(4-acetylphenoxy)-2-R-methylpropyl] piperidine
1-[3-(4-cyanophenoxy)propyl]-4-methylpiperidine
1-[3-(4-cyanophenoxy)propyl]-3-methylpiperidine
1-[3-(4-acetylphenoxy)-2-S-methylpropyl] piperidine
1-{3-[4-(3-oxobutyl)phenoxy] propyl}piperidine
1-[3-(4-cyano-3-fluorophenoxy)propyl] piperidine
1-[3-(4-nitrophenoxy)propyl]-3-methylpiperidine
1-[3-(4-cyanophenoxy)propyl]-2-methylpiperidine
1-[3-(4-nitrophenoxy)propyl]-2-methylpiperidine
1-[3-(4-nitrophenoxy)propyl]-4-methylpiperidine
1-[3-(4-cyanophenoxy)propyl]-2,6-dimethylpiperidine
1-[3-(4-propionylphenoxy)propyl]-3-methylpiperidine
1-[3-(4-cyclobutanecarbonylphenoxy)propyl] piperidine
1-[3-(4-cyclopentanecarbonylphenoxy) propyl]piperidine
1-[3-(4-cyanophenoxy)propyl]-cis-2-methyl-5-ethylpiperidine
1-[3-(4-cyanophenoxy)propyl]-trans-2-methyl-5-ethylpiperidine
1-[3-(4-cyanophenoxy)propyl]-cis-3,5-dimethylpiperidine
1-[3-(4-propionylphenoxy)propyl]-4-methylpiperidine
1-[3-(4-propionylphenoxy)propyl]-2-methylpiperidine
1-{3-[4-(1-hydroxypropyl)phenoxy]propyl}-3-methylpiperidine
1-{3-[4-(1-hydroxypropyl)phenoxy]propyl}-4-methylpiperidine
1-[3-(4-propionylphenoxy)propyl]-2-methylpiperidine
1-[3-(4-propionylphenoxy)propyl]-4-methylpiperidine methoxime
1-[3-(4-cyanophenoxy)propyl]-trans-3,5-dimethylpiperidine
1-[3-(4-cyclopropyl carbonyl phenoxy) propyl] -trans-3,5-dimethylpiperidine
1-[3-(4-cyclopropyl carbonyl phenoxy) propyl] -cis-3,5-dimethylpiperidine
1-[3-(4-carbomethoxyphenoxy)propyl] piperidine
1-[3-(4-propenylphenoxy)propyl]-2-methyl piperidine
1-[3-(4-propionylphenoxy)propyl]-2-methylpiperidine
1-{3-[4-(1-ethoxypropyl)phenoxy]propyl}-2-methyl piperidine
1-[3-(4-propionylphenoxy)propyl]-4-methylpiperidine
1-[3-(4-bromophenoxy)propyl]piperidine
1-[3-(4-nitrophenoxy)propyl]piperidine
1-[3-(4-N,N-dimethylsulfonamidophenoxy) propyl]piperidine
1-[3-(4-isopropylphenoxy)propyl]piperidine
1-[3-(4-sec-butylphenoxy)propyl]piperidine
1-[3-(4-propylphenoxy)propyl]piperidine
1-[3-(4-ethylphenoxy)propyl]piperidine
1-(5-phenoxypentyl)-4-propyl-piperidine
1-(5-phenoxypentyl)-4-methyl-piperidine
1-(5-phenoxypentyl)-3-methyl-piperidine
1-acetyl-4-(5-phenoxypentyl)-piperazine
1-(5-phenoxypentyl)-3,5-trans-dimethyl-piperidine
1-(5-phenoxypentyl)-3,5-cis-dimethyl-piperidine
1-(5-phenoxypentyl)-2,6-cis-dimethyl-piperidine
4-carboethoxy-1-(5-phenoxypentyl)-piperidine
3-carboethoxy-1-(5-phenoxypentyl)-piperidine
1-(5-phenoxypentyl)-1,2,3,6-tetrahydropyridine
1-[5-(4-nitrophenoxy)-pentyl]-pyrrolidine
1-[5-(4-chlorophenoxy)-pentyl]-pyrrolidine
1-[5-(4-methoxyphenoxy)-pentyl]-pyrrolidine
1-[5-(4-methylphenoxy)-pentyl]-pyrrolidine
1-[5-(4-cyanophenoxy)-pentyl]-pyrrolidine
1-[5-(2-naphthyloxy)-pentyl]-pyrrolidine
1-[5-(1-naphthyloxy)-pentyl]-pyrrolidine
1-[5-(3-chlorophenoxy)-pentyl]-pyrrolidine
1-[5-(4-phenylphenoxy)-pentyl]-pyrrolidine
1-{5-[2-(5,6,7,8-tetrahydronaphthyl)-oxy]-pentyl}-pyrrolidine
1-[5-(3-phenylphenoxy)-pentyl]-pyrrolidine
1-(5-phenoxypentyl)-2,5-dihydropyrrole
1-{5-[1-(5,6,7,8-tetrahydronaphthyl)-oxy]-pentyl}-pyrrolidine
1-(4-phenoxybutyl)-pyrrolidine
1-(6-phenoxyhexyl)-pyrrolidine
1-(5-phenylthiopentyl)-pyrrolidine
1-(4-phenylthiobutyl)-pyrrolidine
1-(3-phenoxypropyl)-pyrrolidine
1-[5-(3-nitrophenoxy)-pentyl]-pyrrolidine
1-[5-(4-fluorophenoxy)-pentyl]-pyrrolidine
1-[5-(4-nitrophenoxy)-pentyl]-3-methyl-piperidine
1-[5-(4-acetylphenoxy)-pentyl]-pyrrolidine
1-[5-(4-aminophenoxy)-pentyl]-pyrrolidine
1-[5-(3-cyanophenoxy)-pentyl]-pyrrolidine
N-[3-(4-nitrophenoxy)-propyl]-diethylamine
N-[3-(4-cyanophenoxy)-propyl]-diethylamine
1-[5-(4-benzoylphenoxy)-pentyl]-pyrrolidine
1-{5-[4-(phenylacetyl)-phenoxy]-pentyl}-pyrrolidine
N-[3-(4-acetylphenoxy)-propyl]-diethylamine
1-[5-(4-acetamidophenoxy)-pentyl]-pyrrolidine
1-[5-(4-phenoxyphenoxy)-pentyl]-pyrrolidine
1-[5-(4-N-benzamidophenoxy)-pentyl]-pyrrolidine
1-{5-[4-(1-hydroxyethyl)-phenoxy]-pentyl}-pyrrolidine
1-[5-(4-cyanophenoxy)-pentyl]-diethylamine
1-[5-(4-cyanophenoxy)-pentyl]-piperidine
N-[5-(4-cyanophenoxy)-pentyl]-dimethylamine
N-[2-(4-cyanophenoxy)-ethyl]-diethylamine
N-[3-(4-cyanophenoxy)-propyl]-dimethylamine
N-[4-(4-cyanophenoxy)-butyl]-diethylamine
N-[5-(4-cyanophenoxy)-pentyl]-dipropylamine
1-[3-(4-cyanophenoxy)-propyl]-pyrrolidine
1-[3-(4-cyanophenoxy)-propyl]-piperidine
N-[3-(4-cyanophenoxy)-propyl]-hexamethyleneimine
N-[6-(4-cyanophenoxy)-hexyl]-diethylamine
N-[3-(4-cyanophenoxy)-propyl]-dipropylamine
N-3-[4-(1-hydroxyethyl)-phenoxy]-propyl-diethylamine
4-(3-diethylaminopropoxy)-acetophenone-oxime
1-[3-(4-acetylphenoxy)-propyl]-piperidine
1-[3-(4-acetylphenoxy)-propyl]-3-methyl-piperidine
1-[3-(4-acetylphenoxy)-propyl]-3,5-trans-dimethyl-piperidine
1-[3-(4-acetylphenoxy)-propyl]-4-methyl-piperidine
1-[3-(4-propionylphenoxy)-propyl]-piperidine
1-[3-(4-acetylphenoxy)-propyl]-3,5-cis-dimethyl-piperidine
1-[3-(4-formylphenoxy)-propyl]-piperidine
1-[3-(4-isobutyrylphenoxy)-propyl]-piperidine
N-[3-(4-propionylphenoxy)-propyl]-diethylamine
1-[3-(4-butyrylphenoxy)-propyl]-piperidine
1-[3-(4-acetylphenoxy)-propyl]-1,2,3,6-tetrahydropyridine.

28. Use according to anyone of claims 16 to 27, **characterized in that** it is one of the following compounds :
1-[5-(4-nitrophenoxy)-pentyl]-pyrrolidine
1-{5-[4-(1-hydroxyethyl)-phenoxy]-pentyl}-pyrrolidine
1-[3-(4-cyanophenoxy)-propyl]-piperidine
N-[3-(4-cyanophenoxy)-propyl]-hexamethyleneimine
N-3-[4-(1-hydroxyethyl)-phenoxy]-propyl-diethylamine
4-(3-diethylaminopropoxy)-acetophenone-oxime
1-[3-(4-acetylphenoxy)-propyl]-3-methyl-piperidine
1-[3-(4-acetylphenoxy)-propyl]-4-methyl-piperidine
1-[3-(4-propionylphenoxy)-propyl]-piperidine
N-[3-(4-cyanophenoxy)-propyl]-diethylamine
N-[3-(4-acetylphenoxy)-propyl]-diethylamine
N-[4-(4-cyanophenoxy)-butyl]-diethylamine

29. Use according to anyone of claims 1 to 15, having the following general formula (IIa) and (IIb): 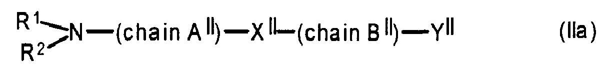 or 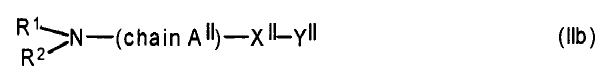 in which
- R¹ and R² are as defined with reference to general formula (A) in claim 1;
- the chain A^{II} represents a saturated or unsaturated, straight or branched hydrocarbon chain containing 1 to 6 carbon atoms, it being possible for the saturated hydrocarbon chain to be interrupted by a hetero atom such as a sulphur atom;
- X^{II} represents an oxygen or sulphur atom, -NH-, -NHCO-, -N(alkyl)CO-, -NHCONH-, -NH-CS-NH-, -NHCS-, -O-CO-, -CO-O-, -OCONH-, -OCON(alkyl)-, -OCON(alkene), -OCONH-CO-, -CONH-, -CON(alkyl)-, -SO-, -CO-, -CHOH-, -N(saturated or unsaturated alkyl), -S-C(=NY")-NH-Y"- with the Y" identical or different, as defined previsouly, or -NR_{II}-C(=NR"_{II})-NR'_{II}-, R_{II} and R'_{II}denoting a hydrogen atom or a lower alkyl radical and R"_{II} a hydrogen atom or another powerful electronegative group, such as a cyano or COY₁^{II} group, Y₁^{II} denoting an alkoxy group;
- the chain B" represents an aryl, arylalkyl or arylalkanoyl group, a straight alkylene chain -(CH₂)_{nII}-, n being an integer which can vary between 1 and 5 or a branched alkylene chain containing from 2 to 8 carbon atoms, the alkylene chain being optionally interrupted by one or a number of oxygen or sulphur atoms, or a group -(CH2)nll-0- or -(CH₂)ₙₗₗ-S- where nₗₗ is an integer equal to 1 or 2;
- Y" represents a straight or branched alkyl group containing 1 to 8 carbon atoms; a cycloalkyl containing 3 to 6 carbon atoms; a bicycloalkyl group; a cycloalkenyl group; an aryl group such as an optionally substituted phenyl group; a 5- or 6-membered heterocyclic radical containing one or two heteroatoms chosen from nitrogen and sulphur atoms, the said heterocyclic radical optionally being substituted; or also a bicyclic radical resulting from the fusion of a benzene ring to a heterocycle as defined above.

30. Use according to anyone of claims 1 to 15, having the following formula (IIa) and (IIb): 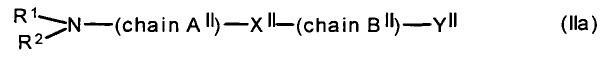 or 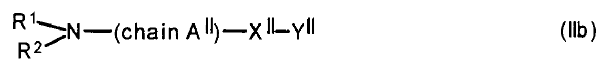 in which:
- R¹ and R² are as defined with reference to general formula (A) in claim 1;
- the chain A" represents an unbranched, branched or unsaturated alkyl group -(CH₂)_{nII}- where nₗₗ is an integer which can vary between 1 and 8 and preferably between 1 and 4; an unbranched or branched alkene group comprising from 1 to 8 carbon atoms and preferably 1 to 4 carbon atoms; an unbranched or branched alkyne group comprising from 1 to 4 carbon atoms;
- the group X^{II} represents -OCONH-; -OCON(alkyl)-; -OCON(alkene)-; -OCO-; -OCSNH-; -CH₂-; -0-; -OCH₂CO-; -S-; -CO-; -CS-; amine; saturated or unsaturated alkyl;
- the chain B" represents an unbranched, branched or unsaturated lower alkyl comprising from 1 to 8 carbon atoms and preferably 1 to 5 carbon atoms; -(CH₂)ₙₗₗ(hetero atom)- where the hetero atom is preferably a sulphur or oxygen atom; nₗₗ being an integer which can vary between 1 and 5, preferably between 1 and 4;
- the group Y^{ll} represents a phenyl group, unsubstituted or mono- or polysubstituted with one or more identical or different substituents selected from halogen atoms, OCF₃, CHO, CF₃, SO₂N(alkyl)₂ such as SO₂N(CH₃)₂, NO₂, S(alkyl), S(aryl), SCH₂(phenyl), an unbranched or branched alkene, an unbranched or branched alkyne optionally substituted with a trialkylsilyl radical, -O(alkyl), -O(aryl), -CH₂CN, a ketone, an aldehyde, a sulphone, an acetal, an alcohol, a lower alkyl, -CH=CH-CHO, -C(alkyl)=N-OH, -C(alkyl)=N-O(alkyl) and other keto derivatives, -CH=NOH, -CH=NO(alkyl), and other aldehyde derivatives, -C(alkyl)=NH-NH-CONH₂, an O-phenyl or -OCH₂(phenyl) group, -C(cycloalkyl)=NOH, -C(cycloalkyl)=N-O(alkyl), an optionally substituted heterocycle; a heterocycle comprising a sulphur hetero atom; a cycloalkyl; a bicyclic group and preferably a norbornyl group; a phenyl ring fused to a heterocycle comprising a nitrogen hetero atom or to a carbocycle or a heterocycle bearing a keto function; an unbranched or branched lower alkyl comprising from 1 to 8 carbon atoms; an unbranched or branched alkyne comprising from 1 to 8 carbon atoms and preferably 1 to 5 carbon atoms; a linear or branched alkyl mono- or polysubstituted with phenyl groups which are either unsubstituted or mono- or polysubstituted; a phenyl alkyl ketone in which the alkyl group is branched or unbranched or cyclic; a substituted or unsubstituted benzophenone; a substituted or unsubstituted, unbranched or branched or cyclic phenyl alcohol; an unbranched or branched alkene; a piperidyl group; a phenylcycloalkyl group; a polycyclic group, in particular a fluorenyl group, a naphthyl or polyhydronaphthyl group or an indanyl group; a phenol group; a ketone or keto derivative; a diphenyl group; a phenoxyphenyl group; a benzyloxyphenyl group.

31. Use according to claim 29 or 30, **characterized in that** X^{II} is selected from -0-, -NH-, -CH₂-, -OCONH-, -NHCO-, -NHCONH- and represents more preferably an oxygen atom.

32. Use according to anyone of claims 29 to 31, **characterized in that** Y^{II} is selected from a linear or branched alkyl group; a cycloalkyl group, in particular cyclopentyl or cyclohexyl group; a phenyl group unsubstituted or mono-substituted, preferred substituent being halogen atom, in particular chorine; a heterocyclic radical, in particular pyridyl N-oxide or pyrazinyl radicals; a bicyclic radical such as a benzothiazolyl radical, Y^{II} being more preferably a phenyl group unsubstituted or mono-substituted as above-defined.

33. Use according to anyone of claims 29 to 31, **characterized in that** Y^{II} represents a phenyl group at least mono-substituted with a keto-substituent, in particular a linear or branched chain aliphatic ketone comprising from 1 to 8 carbon atoms and optionnally bearing a hydroxyl group, a cycloalkylketone, an aryl alkyl ketone or arylalkenylketone in which the aryl group is optionally substituted, or a heteroaryl ketone, preferably a cycloalkylketone; an oxime-substituent or an halogen atom.

34. Use according to anyone of claims 29 to 31, **characterized in that** Y^{II} is a phenyl group at least mono-substituted with -CHO, a ketone, an aldehyde, -CH=CH-CHO, -C(alkyl)=N-OH, -C(alkyl)=N-O(alkyl) and other keto derivatives, -CH=N-OH, -CH=NO(alkyl) and other aldehyde derivatives, -C(cycloalkyl)=NOH, -C(cycloalkyl)=N-O(alkyl).

35. Use according to anyone of claims 29 to 34, **characterized in that** chain A^{II} is a chain -(CH₂)_{nII}- with n varying from 1 to 6, preferably from 1 to 4, the chain A^{II} representing especially -(CH₂)₃-.

36. Use according to anyone of claims 29 to 35, **characterized in that** the chain B" is -(CH₂)₂- or -(CH₂)₃-.

37. Use according to anyone of claims 29 to 36, **characterized in that** X is an oxygen atom, the chain A represents -(CH₂)₃- and, for compounds of formula (IIa), the chain B represents -(CH₂)₃- also.

38. Use according to anyone of claims 29 to 37, **characterized in that** it is one of the following compounds:
- 3,3-Dimethylbutyl 3-piperidinopropyl ether
- 3-Phenylpropyl 3-piperidinopropyl ether
- 3-(4-Chlorophenyl)propyl 3-piperidinopropyl ether
- 2-Benzothiazolyl 3-piperidinopropyl ether
- 3-Phenylpropyl 3-(4-methylpiperidino)propyl ether
- 3-Phenylpropyl 3-(3,5-cis-dimethylpiperidino)propyl ether
- 3-Phenylpropyl 3-(3,5-trans-dimethylpiperidino)propyl ether
- 3-Phenylpropyl 3-(3-methylpiperidino)propyl ether
- 3-Phenylpropyl 3-pyrrolidinopropyl ether
- 3-(4-Chlorophenyl)propyl 3-(4-methylpiperidino)propyl ether
- 3-(4-Chlorophenyl) propyl 3-(3,5-cis-dimethyl piperidino) propyl ether
- 3-(4-Chlorophenyl) propyl 3-(3,5-trans-dimethyl piperidino) propyl ether
- 3-Phenylpropyl 3-(N,N-diethylamino)propyl ether
- N-Phenyl-3-piperidinopropyl carbamate
- N-Pentyl-3-piperidinopropyl carbamate
- (S)-(+)-N-[2-(3,3-Dimethyl)butyl]-3-piperidinopropyl carbamate
- 3-Cyclopentyl-N-(3-(1-pyrrolidinyl)propyl)propanamide
- N-Cyctohexyf-N'-(1 -pyrrolidinyl-3-propyl)urea
- 2-((2-Piperidinoethyl)amino)benzothiazole
- 5-Piperidinopentylamine
- 2-Nitro-5-(6-piperidinohexyl)pyridine
- 3-Nitro-2-(6-piperidinohexylamino)pyridine
- 2-(6-Piperidinohexylamino)pyrimidine
- N-(6-Phenylhexyl)piperidine
- N-phenyl-N'-(diethylamino-3-propyl)urea
- N-benzyl-N'-(3-piperidinopropyl)guanidine
- N-(3-(N,N-Diethylamino)propyl)N'-phenylurea
- N-Cyclohexylmethyl-N'-(3-piperidinopropyl)guanidine

39. Use according to anyone of claims 1 to 15, having the following formula (III) 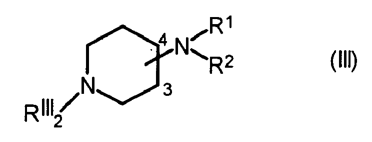 in which:
• NR¹R² is either in 3-position or in 4-position on the piperidyl moiety, R¹ and R² being as defined with reference to formula (A) in claim 1;
• R₂^{III} denotes a linear or branched alkyl group having 1 to 6 carbon atoms; a piperonyl group, a 3-(1-benzimidazolonyl)propyl group; a group of formula 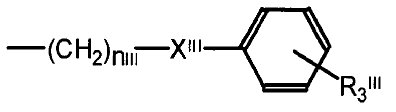
in which n_{III} is 0, 1, 2 or 3, X^{III} is a single bond or alternatively -0-, -S-, -NH-, - CO-, -CH=CH- or 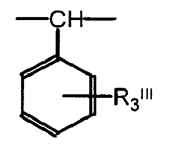 and R₃^{III} is H, CH₃, halogen, CN, CF₃ or an acyl group -COR₄^{III}, R₄^{III} being a linear or branched alkyl group having 1 to 6 carbon atoms, a cycloalkyl group having 3 to 6 carbon atoms or a phenyl group which can bear a CH₃ or F substituent; or alternatively a group of formula 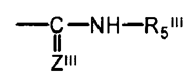 in which Z^{III} denotes an O or S atom or a divalent group NH, N-CH₃ or N-CN and R₅^{III} denotes a linear or branched alkyl group having 1 to 8 carbon atoms, a cycloalkyl group having 3 to 6 carbon atoms which can bear a phenyl substituent, a (C₃-C₆ cycloalkyl) (linear or branched, C₁-C₃ alkyl) group, a phenyl group which can bear a CH₃, halogen or CF₃ substituent, a phenyl(linear or branched, C₁-C₃ alkyl) group or a naphthyl, adamantyl or p-toluenesulphonyl group.

40. Use according to claim 39, **characterized in that** R^{III} represents the group 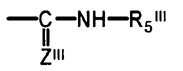 Z^{III} and R^{III}₅ being as defined in claim 39, Z^{III} being especially O, S or NH.

41. Use according to claim 40, **characterized in that** R^{III}₅ is a (C₃₋C₆)cycloalkyl group.

42. Use according to anyone of claims 39 to 41, which is N'-Cyclohexylthiocarbamoyl-N-1,4'-bipiperidine.

43. Use according to anyone of claims 1 to 15, which have the following formula (IV): 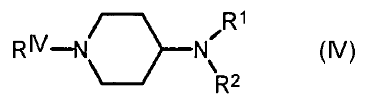 in which
- R¹ and R² are as defined with reference to general formula (A) in claim 1;
- R^{IV} represents a hydrogen atom or a group COR₃^{IV}, in which R₃^{IV} represents
(a) a linear or branched aliphatic group containing 1 to 11, and in particular 1 to 9, carbon atoms;
(b) a cyclane ring-system such as cyclopropane, phenylcyclopropane, cyclobutane, cyclopentane, cyclohexane, cycloheptane, norbornane, adamantane, noradamantane, chlorooxonorbornane, chloroethylenedioxynorbornane, bromoethylenedioxynorbornane and the anhydride group of hydroxycarboxy-1,2,2-trimethylcyclopentanecarboxylic acid;
(c) a benzene ring, unsubstituted or substituted at the para-position with a linear or branched aliphatic group containing 3 to 5 carbon atoms, as well as with a halogen;
(d) a group (CH₂)_{mIV}R₄^{IV} in which m_{IV} is a number between 1 and 10, and R₄^{IV} represents a cyclane ring system such as cyclopropane, cyclobutane, cyclopentane, cyclopentene, cyclohexane, cycloheptane, norbornane, noradamantane, adamantane and 6,6-dimethylbicyclo[3.1.1] heptene; a benzene ring, unsubstituted or monosubstituted with a fluorine atom, a chlorine atom, a methyl group or a methoxy group; a thiophene ring grafted via its ring-position 2 or its ring-position 3; a carboxylic acid ester group COOR₅^{IV}, in which R₅^{IV} is a cyclane ring-system such as cyclopropane, cyclobutane, cyclopentane, cyclohexane or norbornane; a carboxylic acid amide group of structure CONHR₆^{IV}, in which R₆^{IV} represents a cyclane ring-system such as cyclopropane, cyclobutane, cyclopentane, cyclohexane or norbornane; a carboxylic acid amide group of structure 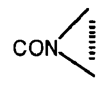 in which the group 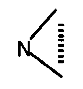 represents pyrrolidine, piperidine or 2,6-dimethylmorpholine; or an ether group ―O-R₇^{IV}, it being possible for R₇^{IV} to be a benzene ring, unsubstituted or monosubstituted with a chlorine or fluorine atom or disubstituted with a chlorine atom and with a methyl group;
(e) a group -CH=CHR₈^{IV}, in which R₈^{IV} represents a cyclane ring-system such as cyclopropane, cyclobutane, cyclopentane, cyclohexane, norbomane or norbornene;
(f) a secondary amine group -NH(CH₂)_{nIV}R₉^{IV}, in which n_{IV} is a number between 1 and 5 and R₉^{IV} constitutes a cyclane ring-system such as cyclopropane, cyclobutane, cyclopentane, cyclohexane or norbornane, or a benzene ring, unsubstituted, mono-substituted with a fluorine or chlorine atom or with a methoxy group or trisubstituted with methoxy groups;
R^{IV} also represents a hydroxyalkenyl group 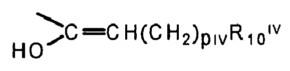
in which p_{IV} is a number between 2 and 9 and R₁₀^{IV}, represents a benzene ring or a phenoxy group; as well as a group
CSNH(CH₃)_{nIV}R₉^{IV}
in which n_{IV} is a number between 1 and 5 and R₉^{IV} has the meaning stated above.

44. Use according to claim 43, **characterized in that** R^{IV} represents the group COR₃^{IV}, R₃^{IV} representing especially an aliphatic group a).

45. Use according to anyone of claims 43 and 44, which is N-Heptanoyl-1,4'-bipiperidine or 1-(5-Cyclohexylpentanoyl)-1,4-bipiperidine

46. Use according to anyone of claims 1 to 15, having the following formula (VI): 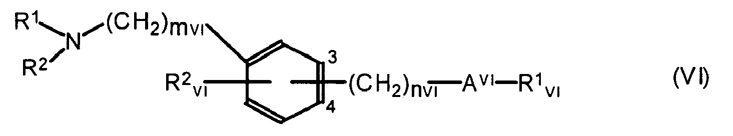 wherein:
- A^{VI} is selected from -O-CO-NR¹_{VI}-, -O-CO-, -NR¹_{VI}-CO- NR¹_{VI}-, - NR¹_{VI}-CO-, -NR¹_{VI}-, -0-, -CO-NR¹_{VI}-, -CO-O-, and -C(=NR¹_{VI})-NR¹_{VI}-;
- the groups R¹_{VI}, which may be the same or different when there are two or three such groups in the molecule of formula VI, are selected from hydrogen, and lower alkyl, aryl, cycloalkyl, heterocyclic and heterocyclyl-alkyl groups, and groups of the formula -(CH₂)_{y}VI-G^{VI}, where G^{VI} is selected from CO₂R³_{VI}, COR³_{VI}, CONR³_{VI}R⁴_{VI}, OR³_{VI}, SR³_{VI}, NR³_{VI}R⁴_{VI}, heteroaryl and phenyl, which phenyl is optionally substituted by halogen, lower alkoxy or polyhaloloweralkyl, and y_{VI} is an integer from 1 to 3;
- R²_{VI}, is selected from hydrogen and halogen atoms, and alkyl, alkenyl, alkynyl and trifluoromethyl groups, and groups of the formula OR³_{VI}, SR³_{VI} and NR³_{VI}R⁴_{VI};
- R³_{VI} and R⁴_{VI} are independently selected from hydrogen, and lower alkyl and cycloalkyl groups, or R³_{VI} and R⁴_{VI} together with the intervening nitrogen atom can form a saturated ring containing 4 to 6 carbon atoms that can be substituted with one or two lower alkyl groups;
- the group -(CH₂)_{nVI}-A^{VI}-R¹_{VI} is at the 3- or 4-position, and the group R²_{VI} is at any free position;
- m_{VI} is an integer from 1 to 3;
- and n_{VI} is 0 or an integer from 1 to 3.

47. Use according to anyone of claims 1 to 15, having the following formula (VI): 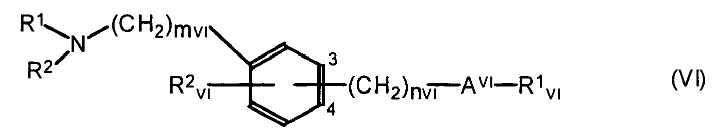 wherein R¹_{VI} is an aryl group, preferably a phenyl group optionally substituted with a keto-substituent, in particular a linear or branched chain aliphatic ketone comprising from 1 to 8 carbon atoms and optionnally bearing a hydroxyl group, a cycloalkylketone, an aryl alkyl ketone or arylalkenylketone in which the aryl group is optionally substituted, or a heteroaryl ketone, preferably a cycloalkylketone, R²_{VI}, n_{VI}, m_{VI} and A^{VI} being as defined in claim 46.

48. Use according to claim 46 or 47, **characterized in that** n_{VI} and m_{VI} are each 1, and A^{VI} represents an oxygen atom.

49. Use according to claim 46 or 48, **characterized in that** R¹_{VI} is an aryl or -(CH₂)_{yVI}-G^{VI} with G^{VI} being a phenyl.

50. Use according to anyone of claims 46 to 49, with one of the following compounds:
- α-(4-Acetylphenoxy)-α'-piperidino p-xylol
- α-(4-Acetylphenoxy)-α'-(1-pyrrolidinyl) p-xylol
- α-(3-Phenylpropoxy)-α'-piperidino p-xylol
- α-(4-Acetylphenoxy)-α'-(4-methylpiperidino)p-xylol
- α-(4-Acetylphenoxy)-α'-(3,5-cis-dimethylpiperidino)p-xylol
- α-(4-Acetylphenoxy)-α'-(3,5-trans-dimethylpiperidino)p-xylol
- α-(4-Acetylphenoxy)-α'-(2-methylpyrrolidino)p-xylol
- α-(4-Cyclopropylcarbonylphenoxy)-α'-piperidino-p-xylol
- α-(4-Cyclopropylcarbonylphenoxy)-α'-(4-methylpiperidino) p-xylol
- α-(4-Cyclopropylcarbonylphenoxy)-α'-pyrrolidino-p-xylol
- N-(4-Chlorobenzyl)-2-(4-piperidinomethyl)phenyl) ethan amidine

51. Use according to anyone of claims 1 to 15, having the following formula (VII): 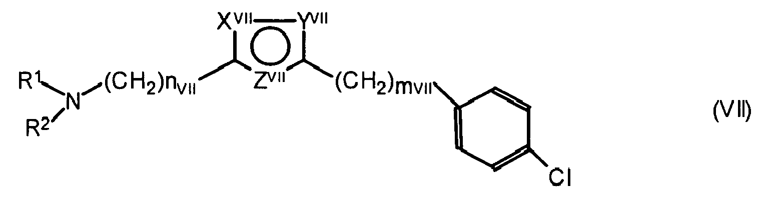 in which
- R¹ and R² are as defined in reference to formula (A) in claim 1;
- X^{VII}, Y^{VII} and Z^{VII} are identical or different and represent O, N or S;
- n_{VII} is varying from 1 to 3;
- m_{VII} is 1 or 2.

52. Use according to claim 51, **characterized in that** X^{VII} is 0 and Y^{VII} and Z^{VII} are each N to represent a 1, 2, 4-oxadiazolyl group.

53. Use according to claims 51 or 52 of a compound which is 3-(4-Chlorobenzyl)-5-(2-piperidinoethyl)-1,2,4-oxadiazole

54. Use according to anyone of claims 1 to 15 of a compound having the following formula (VIII): 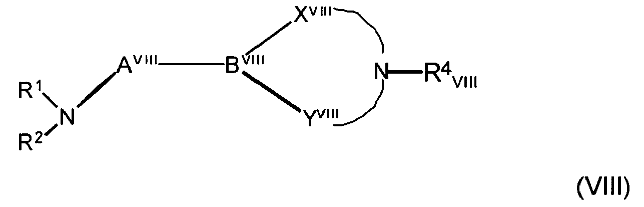 wherein R¹ and R² are as defined with reference to formula (A) in claim 1 and
wherein
A^{VIII} is
1) a group of the formula (CH₂)_{mVIII}, wherein m_{VIII} = 0-9; or
2) a group of the formula: 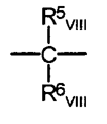 wherein R⁵_{VIII} represents hydrogen, (C₁-C₃)alkyl-, aryl(C₁-C₃)alkyl-, aryl-,
wherein aryl may optionally be substituted, hydroxyl-, (C₁-C₃)alkoxy-, halogen, amino-, cyano- or nitro; and R⁶_{VIII} represents hydrogen, (C₁-C₃)alkyl-, aryl(C₁₋C₃)alkyl-, or aryl-, wherein aryl may optionally be substituted; or
3) a group of the formula: 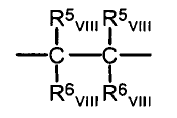 wherein R⁵_{VIII} and R⁶_{VIII} are as defined above; or
4) a group of the formula: 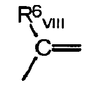 if B^{VIII} is a group of the formula: 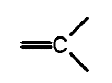 such that A^{VIII} and B^{VIII} together form a group of the formula: 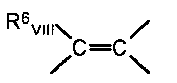 wherein R⁶_{VIII} is as defined above; or
5) a group of the formula: 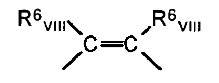 wherein R⁶_{VIII} is as defined above; or
6) a group of the formula: 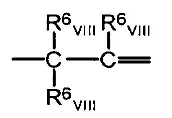 if B^{VIII} is a group of the formula: 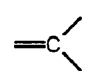 such that A^{VIII} and B^{VIII} together form a group of the formula: 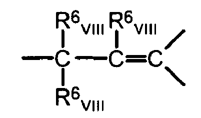 wherein R⁶_{VIII} is as defined above; or
7) a group of the formula:
― (CH₂)_{XVIII}―S―(CH₂)_{yVIII}―
wherein x_{VIII} + y_{VIII} = m_{VIII}-1;
B^{VIII} is
1) a group of the formula: 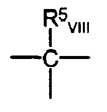 wherein R⁵_{VIII} is as defined above; or
2) a group of the formula: 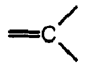 if A is a group of one of the formulas: 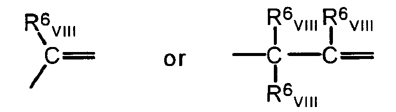 such that A and B together form a group of one of the formulas: 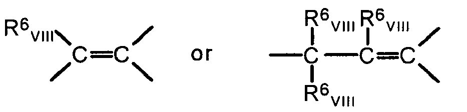 wherein R⁶_{VIII} is as defined above; or
3) a group of the formula: 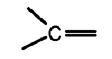 if X^{VIII} is a group of the formula: 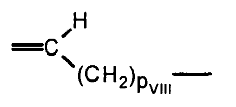 such that B^{VIII} and X^{VIII} together form a group of the formula 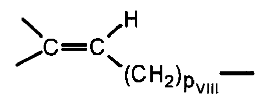
wherein p_{VIII} = 1-3; or
X^{VIII} is
1) a group of the formula (CH₂)_{nVIII} wherein n_{VIII} = 2-4; or
2) a group of the formula: 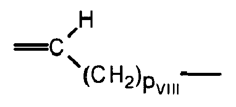 if B^{VIII} is a group of the formula: 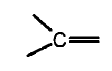 such that X^{VIII} and B^{VIII} together form a group of the formula: 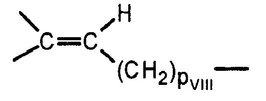 wherein p_{VIII} = 1-3; or
3) two hydrogens (one on the carbon and one on the nitrogen); or
4) one hydrogen on the carbon atom and one R⁷_{VIII} group on the nitrogen atom,
wherein R⁷_{VIII} represents hydrogen, (C₁-C₁₀)alkyl-, aryl (C₁-C₁₀)alkyl-, or aryl,
wherein aryl may optionally be substituted;
Y^{VIII} is a group of the formula (CH₂)_{kVIII}, wherein k_{VIII} = 0-2;
R⁴_{VIII} represents hydrogen, (C₁-C₁₀)alkyl-, (C₁-C₃)alkyl-sulfonamide-, aryl(C₁₋C₁₀)alkyl-, aryl, wherein aryl may optionally be substituted;
or a group of the formula: 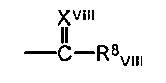 or a group of the formula: 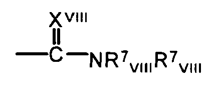 wherein X^{VIII} represents O, S, or NH,
R⁷_{VIII} is as defined as above;
R⁸_{vIII} represents (C₁-C₁₀)alkyl-, aryl(C₁-C₁₀)alkyl-or aryl,
wherein aryl may optionally be substituted and wherein aryl is phenyl, substituted phenyl, naphtyl, substituted naphtyl, pyridyl;

55. Use according to claim 54 of a compound having the formula 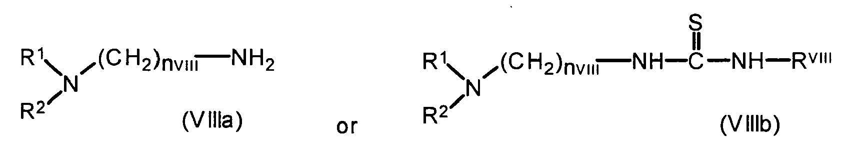 R¹ and R² having the meaning given in claim 1 and n_{VIII} and R^{VIII} having the meaning given in claim 54.

56. Use according to claim 54 or 55 of a compound which is 2-Nitro-5-(6-piperidinohexyl)pyridine or 10-piperidinodecylamine.

57. Use according to anyone of claims 1 to 15 of a compound having the following formula (IX): 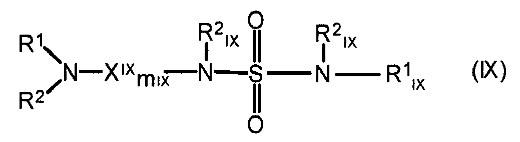 wherein:
R¹ and R² are as defined with reference to formula (A) in claim 1.
R¹_{IX} is C₄ to C₂₀ hydrocarbyl (in which one or more hydrogen atoms may be replaced by halogen, and up to four carbon atoms [and especially from 0 to 3 carbon atoms] may be replaced by oxygen, nitrogen or sulphur atoms, provided that R¹_{IX} does not contain an -O-O-group),
R²_{IX} identical or different, are H or C₁ to C₁₅ hydrocarbyl (in which one or more hydrogen atoms may be replaced by halogen, and up to three carbon atoms may be replaced by oxygen, nitrogen or sulphur atoms, provided that R²_{IX} does not contain an -O-O-group.
m_{IX} is from 1 to 15 (preferably 1 to 10, more preferably 3 to 10, eg. 4 to 9)
each X^{IX} group is independently 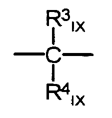 , or one X^{IX} group is -N(R⁴_{IX})-, -0- or -S- (provided that this X^{IX} group is not adjacent the -NR²_{IX-}group) and the remaining X^{IX} groups are independently 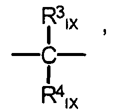 wherein R³_{IX} is H, C₁ to C₆ alkyl, C₂ to C₆ alkenyl, -CO₂R⁵_{IX}, -CON(R⁵_{IX})₂, -CR⁵_{IX2}OR⁶_{IX} or -OR⁵IX (in which R⁵_{IX} and R⁶_{IX} are H or C₁ to C₃ alkyl), and R⁴_{IX} is H or C₁ to C₆ alkyl.

58. Use according to claim 57 of a compound which is N-(4-Bromobenzyl)-N'-(4-piperidinobutyl)sulphamide.

59. Use according to anyone of claims 1 to 15 of a compound having the following formula (X): 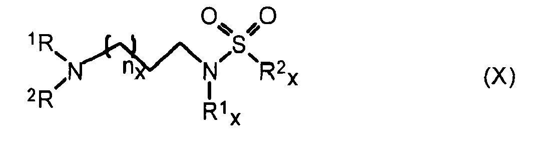 wherein:
- R¹ and R² are as defined with reference to formula (A) in claim 1;
- R¹ₓ is H or CH₃;
- R²x is selected from a phenyl optionally substituted with a halogen atom, preferably chlorine, a (C₁-C₄)alkyl, a (C₁-C₄)alkoxy, CF₃, OCF₃, NO₂, NH₂; or a CH₂-phenyl optionally substituted as above-specified;
- nₓ is from 0 to 3.

60. Use according to claim 59, of a compound which is 3-Chloro-N-(4-piperidinobutyl)-N-methyl-benzene sulphonamide.

61. Use according to claims 1 to 15, having the following formula (XI): 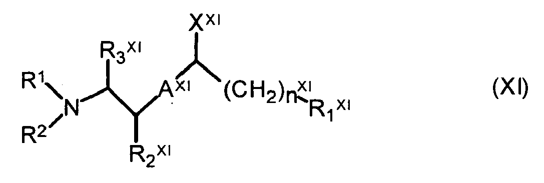 where R¹ and R² are as defined with reference to formula (A) in claim 1;
where A^{XI} is -NHCO-, -N(CH₃)-CO-, -NHCH₂-, -N(CH₃)-CH₂-, -CH=CH-, -COCH₂-, CH₂CH₂-, -CH(OH)CH₂-, or -C=C- ;
X^{XI} is H, CH₃, NH₂, NH(CH₃), N(CH₃)₂, OH, OCH₃, or SH;
R₂^{XI} is hydrogen or a methyl or ethyl group;
R₃^{XI} is hydrogen or a methyl or ethyl group;
n^{XI} is 0, 1, 2, 3, 4, 5 or 6; and
R₁^{XI} is selected from the group consisting of C₃ to C₈ cycloalkyl; phenyl or substituted phenyl; decahydronaphthalene and octahydroindene; or
R₁^{XI} and X^{XI} may be taken together to denote a 5,6- or 6,6-saturated bicyclic ring structure when X^{XI} is NH, O, S, or SO₂.

62. Use according to claim 61, **characterized in that** it is one of the following compounds :
- c*is-*1-(6-Cyclohexyl-3-hexen-1-yl)piperidine
- *trans*-1-(6-Cyclohexyl-3-hexen-1-yl)piperidine
- 1-(6-Cyclohexyl-3-hexin-1-yl)piperidine

63. Use according to claim 1 to 15, having the following formula (XII): 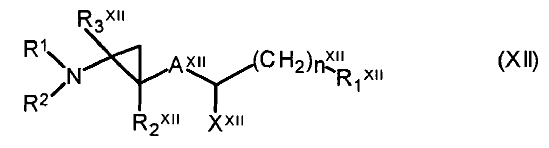 where R¹ and R² are as defined in reference to formula (A) in claim 1;
where R₂^{XII} is a hydrogen or a methyl or ethyl group;
R₃^{XII} is a hydrogen or a methyl or ethyl group;
n^{XII} is 0, 1, 2, 3, 4, 5, or 6; and
R₁^{XII} is selected from the group consisting of C₃ to C₈ cycloalkyl; phenyl substituted or not by one or more groups such as a halogen atom, a lower alkyl or cycloalkyl, a trifluoromethyl, aryl, alkoxy, α-alkyloxyalkyl, aryloxy, nitro, formyl, alkanoyl, aroyl, arylalkanoyl, amino, carboxamido, cyano, alkyloximino, alkylalkoximino, aryloximino, α-hydroxyalkyl, alkenyl, alkynyl, sulphamido, sulfamoyl, sulphonamido, carboxamide, carbocycloalkyl, alkylcarbnyloalkyl, carbonylalkoxy, arylalkyl or oxime group, or two substituants taken together with the carbon atoms of the phenyl ring to which it is fused form 5- or 6-membered saturated or unsaturated ring or a benzene ring or alkyl; heterocyclic; decahydronaphthalene; and octahydroindene; with the provisos that
when X^{XII} is H, A^{XII} can be -CH₂CH₂-, -COCH₂-, -CONH-, -CON(CH₃)-, -CH=CH-, -C=C-, -CH₂-NH-, -CH₂-N(CH₃)-, -CH(OH)CH₂-, -NH-CH₂-, -N(CH₃)-CH₂-, -CH₂O-, -CH₂S-, or -NHCOO-;
when X^{XII} is NH₂, NH(CH₃), N(CH₃)₂, OH, OCH₃, CH₃, SH or SCH₃; A^{XII} can be -NHCO-, -N(CH₃)-CO-, -NHCH₂-, -N(CH₃)-CH₂-, -CH=CH-, -COCH₂-, -CH₂CH₂-, -CH(OH)CH₂-, or -C≡C-; and
when R₁^{XII} and X^{XII} taken together denote a 5,6 or 6,6 saturated bicyclic ring structure X^{XII} can be NH, O, or S.

64. Use according to claim 63, **characterized in that**, A^{XII} is -CH=CH- or -C≡C-.

65. Use according to claims 63 to 64, **characterized in that** R₂^{XII}, R₃^{XII} are each hydrogen atom.

66. Use according to anyone of claims 63 to 65, **characterized in that** n_{XII} is an alkyl group.

67. Use according anyone of claims 63 to 66, of a compound which is 1-(2-(5, 5-Dimethyl-1-hexin-1-yl)cyclopropyl)piperidine.

68. Use according to anyone of claims 1 to 15 having the following formula (XIII): 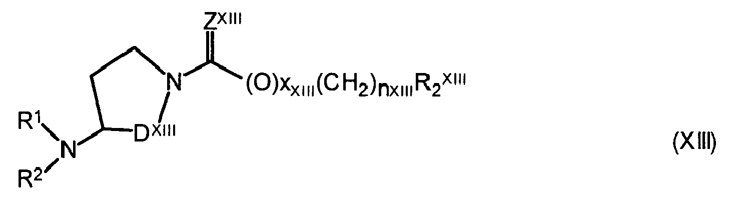 wherein R¹ and R² are as defined with reference to formula (A) in claim 1.
wherein D^{XIII} is CH₂ or CH₂-CH₂, Z^{XIII} represents sulfur (S) or oxygen (O), preferably O, X_{XIII} is 0 or 1, n_{XIII} is an integer from 0 to 6,
and R₂^{III} represents a substituted or unsubstituted linear chain or branched chain alkyl group of up to about 20 carbon atoms, a substituted or unsubstituted carbocyclic group of up to about 20 carbon atoms including mono and bicyclic moieties, and a substituted or an unsubstituted aryl group of up to about 20 carbon atoms, or any combination of above-mentioned groups, or salts thereof.

69. Use according to claim 68, of a compound which is N -heptanoyl-1,4'-bipiperidine or 1-(5-Cyclohexylpentanoyl)-1,4'-bipiperidine.

70. Use according to anyone of claims 1 to 15, having the following formula (XIV) 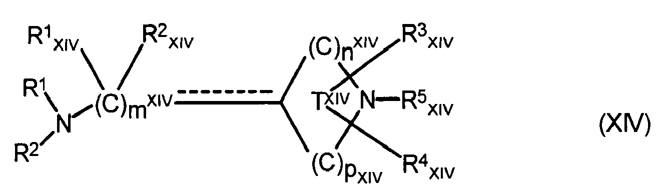 wherein R¹ and R² are as defined in reference of formula (A) in claim 1;
(A) m_{XIV} is an integer selected from the group consisting of: 1 and 2;
(B) n_{XIV} and p_{XIV} are intergers and are each independently selected from the group consisting of: 0, 1, 2, 3, and 4 such that the sum of n_{XIV} and p_{XIV} is 4 and T^{XIV} is a 6-membered ring;
(C) R³_{XIV} and R⁴_{XIV} are each independently bound to the same or different carbon atom of ring T^{XIV}, such that there is only one R³_{XIV} group and one R⁴_{XIV} group in ring T^{XIV}, and each R¹_{XIV}, R²_{XIV}, R³_{XIV} and R⁴ is independently selected from the group consisting of:
(1) H;
(2) C₁ to C₆ alkyl; and
(3) -(CH₂)_{qXIV}-R⁶_{XIV} wherein q_{XIV} is an integer of: 1 to 7, and R⁶_{XIV} is selected from the group consisting of: phenyl, substituted phenyl, -OR⁷_{XIV}, -C(O)OR⁷_{XIV}, -C(O)R⁷_{XIV}, -OC(O)R⁷_{XIV}, -C(O)NR⁷_{XIV}R⁸_{XIV}, CN and -SR⁷_{XIV} wherein R⁷_{XIV} and R⁸_{XIV} are as defined below, and wherein the substituents on said substituted phenyl are each independently selected from the group consisting of: -OH, -O-(C₁ to C₆)alkyl, halogen, C₁ to C₆ alkyl, -CF₃, -CN, and -NO₂, and wherein said substituted phenyl contains from 1 to 3 substituents;
(D) R⁵_{XIV} is selected from the group consisting of:
(1) H;
(2) C₁ to C₂₀ alkyl;
(3) C₃ to C₆ cycloalkyl;
(4) -C(O)OR^{7'}_{XIV} ; wherein R^{7'}_{XIV} is the same as R⁷_{XIV} defined below except that R^{7'}_{XIV} is not H;
(5) -C(O)R⁷_{XIV};
(6) -C(O)NR⁷_{XIV}R⁸_{XIV};
(7) allyl;
(8) propargyl; and
(9) -(CH₂)_{q}-R⁶_{XIV} wherein q_{XIV} and R⁶_{XIV} are as defined above, and when q_{XIV} is equal to 1, then R⁶_{XIV} is not OH or SH;
(E) R⁷_{XIV} and R⁸_{XIV} are each independently selected from the group consisting of: H, C₁ to C₆ alkyl, and C₃ to C₆ cycloalkyl;
(F) the dotted line (--------) represents a double bond that is optionally present when m_{XIV} is 1, and n_{XIV} is not 0, and p is not 0 (i.e., the nitrogen in the ring is not bound directly to the carbon atom bearing the double bond), and when said double bond is present then R²_{XIV} is absent; and
(G) when m_{XIV} is 2, each R¹_{XIV} is the same or different substituent for each m_{XIV}, and each R²_{XIV} is the same or different substituent for each m_{XIV}, and at least two of the substituents R¹_{XIV} and/or R²_{XIV} are H.

71. Use according to claim 70, of a compound which is selected from compounds having the following formula (XIVa), (XIVb) or (XIVc) 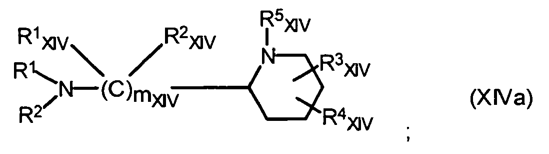 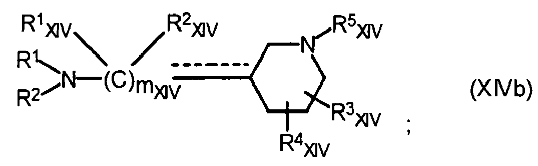 and 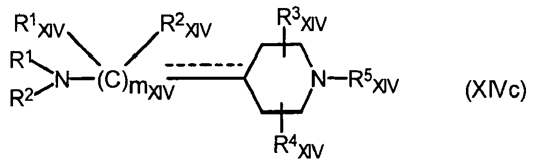 in which R⁵_{XIV} is preferably H or CH₃ and R³_{XIV} and R⁴_{XIV} are preferably each H.

72. Use according to anyone of claims 1 to 15, of a compound having the following formula (XV): 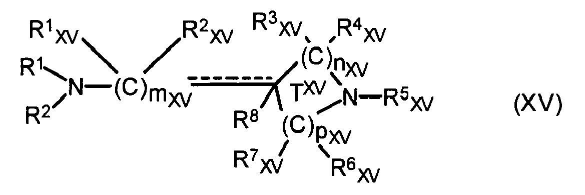 where R¹ and R² are as defined in reference to formula (A) in claim 1;
(A) m_{XV} is an integer selected from the group consisting of: 0,1, and 2;
(B) n_{XV} and pₓᵥ are intergers and are each independently selected from the group consisting of: 0, 1, 2, and 3 such that the sum of nₓᵥ and p_{XV} is 2 or 3 such that when the sum of nₓᵥ and pₓᵥ is 2, T^{xv} is a 4-membered ring and when the sum of n and pₓᵥ is 3, T^{xv} is a 5-membered ring;
(C) each R¹_{XV}, R²_{XV}, R³_{XV}, R⁴_{XV}, R⁶_{XV}, R⁷_{XV} and R⁸_{XV} is independently selected from the group consisting of:
(1) H;
(2) C₁ to C₆ alkyl;
(3) C₃ to C₆ cycloalkyl; and
(4) -(CH₂)_{qXV}-R⁹_{XV}, wherein q_{XV} is an integer of: 1 to 7, and R⁹_{XV} is selected from the group consisting of: phenyl, substituted phenyl, -OR¹⁰_{XV}, -C(O)OR¹⁰_{XV}, -C(O)R¹⁰_{XV}, -OC(O)R¹⁰_{XV}, -C(O)NR¹⁰_{XV}R¹¹_{XV}, CN and -SR¹⁰_{XV} wherein R¹⁰_{XV} and R¹¹_{XV} are as defined below, and wherein the substituents on said substituted phenyl are each independently selected from the group consisting of: -OH, -O-(C₁ to C₆) alkyl, halogen, C₁ to C₆ alkyl, -CF₃, -CN, and -NO₂, and wherein said substituted phenyl contains from 1 to 3 substituents; examples of -(CH₂)_{qXV-}R⁹_{XV} include benzyl, substituted benzyl and the like, wherein the substitutents on the substituted benzyl are as defined above for said substituted phenyl;
(D) R⁵_{XV} is selected from the group consisting of:
(1) H;
(2) C₁ to C₂₀ alkyl;
(3) C₃ to C₆ cycloalkyl;
(4) -C(O)OR^{10'}_{XV}; wherein R^{10'}_{XV} is the same as R¹⁰_{XV} defined below except that R^{10'}_{XV} is not H;
(5) -C(O)R¹⁰_{XV};
(6) -C(O)NR¹⁰_{XV}R¹¹_{XV};
(7) allyl;
(8) propargyl; and
(9) -(CH₂)_{qXV}-R⁹_{XV}, wherein q_{XV} and R⁹_{XV} are as defined above with the proviso that when q_{XV} is 1 then R⁹_{XV} is not -OH or -SH;
(E) R¹⁰_{XV} and R¹¹_{XV} are each independently selected from the group consisting of: H, C₁ to C₆ alkyl, and C₃ to C₆ cycloalkyl; and, for the substituent -C(O)NR¹⁰_{XV}R¹¹, R¹⁰_{XV} and R¹¹_{XV} together with the nitrogen to which they are bound, can form a ring having 5, 6, or 7 atoms;
(F) the dotted line (-----) represents a double bond that is optionally present when m_{XV} is 1, and T^{XV} is a 5-membered ring, and n_{XV} is not 0, and p_{XV} is not 0 (i.e., the nitrogen in the ring is not bound directly to the carbon atom bearing the double bond), and when said double bond is present then R²_{XV} and R⁸_{XV} are absent;
(G) when m_{XV} is 2, each R¹_{XV} is the same or different substituent for each m_{XV}, and each R²_{XV} is the same or different substituent for each m_{XV};
(H) when n_{XV} is 2 or 3, each R³_{XV} is the same or different substituent for each n_{XV}, and each R⁴_{XV} is the same or different substituent for each n_{XV}; and
(I) when p_{XV} is 2 or 3, each R⁶_{XV} is the same or different substituent for each p, and each R⁷_{XV} is the same or different substituent for each p_{XV}.

73. Use according to anyone of claims 1 to 15, of a compound having the following formula (XVI) 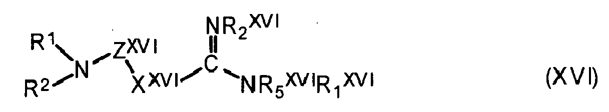 where R¹ and R² are as defined in reference to formula (A) in claim 1;
Z^{XVI} is a group of the formula (CH₂)_{mXVI} wherein m_{XVI} = 1-5 or a group of the formula: 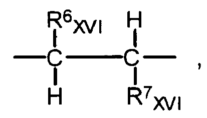 wherein R⁶_{XVI} = (C₁-C₃)alkyl R⁷_{XVI} = (C₁-C₃)alkyl ;
wherein Z^{XVI} may optionally comprise other substituents selected such that the activity of the derivative is not negatively affected,
X^{XVI} represents S, NH or CH₂
R¹_{XVI} represents hydrogen, (C₁-C₃)alkyl-, aryl(C₁-C₁₀)alkyl, wherein aryl may optionally be substituted, aryl, (C₅-C₇)cycloalkyl(C₁-C₁₀)alkyl-, or a group of the formula: 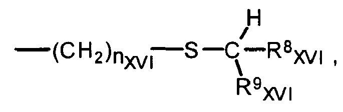
wherein n_{XVI} = 1-4, R⁸_{XVI} is aryl, aryl(C₁-C₁₀)alkyl-, (C₅-C₇)cycloalkyl- or (C₅-C₇) cycloalkyl(C₁-C₁₀)alkyl-, and R⁹_{XVI} is hydrogen, (C₁-C₁₀)alkyl-or aryl; R₂^{XVI} and R₅^{XVI} represent hydrogen, (C₁-C₃)alkyl-, aryl or arylalkyl-, wherein aryl may optionally be substituted; wherein aryl is phenyl, substituted phenyl, naphthyl, substituted napththyl, pyridyl or substituted pyridyl.

74. Use according to anyone of claims 1 to 15, of a compound having the following formula (XVII): 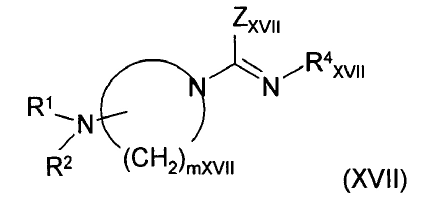 wherein m_{XVII} represents an integer of from 4 to 6.
R⁴_{XVII} represents a hydrogen atom, a linear or branched alkyl group, a cycloalkyl group, a cycloalkylalkyl group, a substituted or unsubstituted aryl group or a substituted or unsubstituted aralkyl group; and Z^{XVII} represents R⁵_{XVII} or A^{XVII}-R⁶_{XVII}, wherein A^{XVII} represents S or O, R₅^{XVII} represents a hydrogen atom, a lower alkyl group, a substituted or unsubstituted aryl group or a substituted or unsubstituted aralkyl group, and R₆^{XVII} represents a lower alkyl group, a lower alkenyl group, a lower alkynyl group or a substituted or unsubstituted aralkyl group.

75. Use according to anyone of claims 1 to 15, of a compound having the following formula (V) : 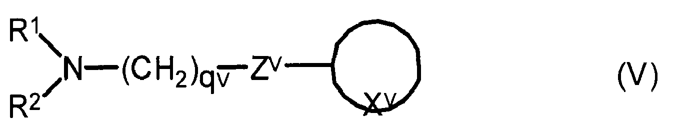 in which
- R¹ and R² are as defined with reference to formula (A) in claim 1;
- q^{v} is 2 to 5
- Z^{v} represents NH, O or S
- Xv represents a heterocycle, optionally condensed, containing one or more heteroatoms like nitrogen, oxygen or sulfur, unsubstituted or substituted by one or more groups like aryl or lower alkyl and halogen.

76. Use according to claim 75 wherein X^{V} means an heterocycle like : 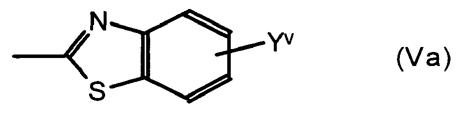 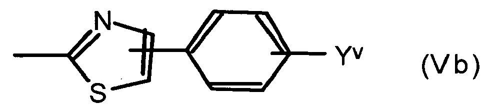 or 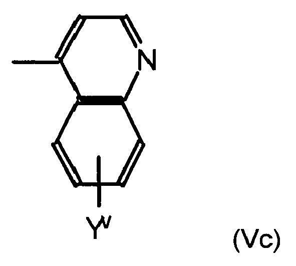 with Y^{V} being an hydrogen atom, a halogen or a lower alkyl.

77. Use according to claims 75 or 76 with one of the following compounds :
2-((2-Piperidinoethyl)amino)benzothiazole
2-(6-Piperidinohexylamino)benzothiazole
4-(6-Piperidinohexylamino)quinoline
2-Methyl 4-(3-piperidinopropylamino)quinoline
2-Methyl 4-(6-piperidinohexylamino)quinoline
7-Chloro-4-(3-piperidinopropylamino)quinoline
7-Chloro-4-(4-piperidinobutylamino)quinoline
7-Chloro-4-(8-piperidinooctylamino)quinoline
7-Chloro-4-(10-piperidinodecylamino)quinoline
7-Chloro-4-(12-piperidinododecylamino)quinoline
7-Chloro-4-(4-(3-piperidinopropoxy)phenylamino)quinoline
7-Chloro-4-(2-(4-(3-piperidinopropoxy) phenyl) ethylamino) quinoline

78. Use according to claim 1 with at least one of the following compounds :
1-(5-phenoxypentyl)-piperidine
1-(5-phenoxypentyl)-pyrrolidine
N-methyl-N-(5-phenoxypentyl)-ethylamine
1-(5-phenoxypentyl)-morpholine
N-(5-phenoxypentyl)-hexamethyleneimine
N-ethyl-N-(5-phenoxypentyl)-propylamine
1-(5-phenoxypentyl)-2-methyl-piperidine
1-(5-phenoxypentyl)-4-propyl-piperidine
1-(5-phenoxypentyl)-4-methyl-piperidine
1-(5-phenoxypentyl)-3-methyl-piperidine
1-acetyl-4-(5-phenoxypentyl)-piperazine
1-(5-phenoxypentyl)-3,5-trans-dimethyl-piperidine
1-(5-phenoxypentyl)-3,5-cis-dimethyl-piperidine
1-(5-phenoxypentyl)-2,6-cis-dimethyl-piperidine
4-carboethoxy-1-(5-phenoxypentyl)-piperidine
3-carboethoxy-1-(5-phenoxypentyl)-piperidine
1-[3-(4-cyclopropylcarbonylphenoxy) propyl]-piperidine
1-[3-(4-acetylphenoxy)-2-R-methylpropyl] piperidine
1-[3-(4-cyanophenoxy)propyl]-4-methylpiperidine
1-[3-(4-cyanophenoxy)propyl]-3-methylpiperidine
1-[3-(4-acetylphenoxy)-2-S-methylpropyl] piperidine
1-{3-[4-(3-oxobutyl)phenoxy] propyl}piperidine
1-[3-(4-cyano-3-ftuorophenoxy)propyl] piperidine
1-[3-(4-nitrophenoxy)propyl]-3-methylpiperidine
1-[3-(4-cyanophenoxy)propyl]-2-methylpiperidine
1-[3-(4-nitrophenoxy)propyl]-2-methylpiperidine
1-[3-(4-nitrophenoxy)propyl]-4-methylpiperidine
1-[3-(4-cyanophenoxy)propyl]-2,6-dimethylpiperidine
1-[3-(4-propionylphenoxy)propyl]-3-methylpiperidine
1-[3-(4-cyclobutylcarbonylphenoxy)propyl] piperidine
1-[3-(4-cyclopentylcarbonylphenoxy) propyl]piperidine
1-[3-(4-cyanophenoxy)propyl]-cis-2-methyl-5-ethylpiperidine
1-[3-(4-cyanophenoxy)propyl]-trans-2-methyl-5-ethylpiperidine
1-[3-(4-cyanophenoxy)propyl]-cis-3,5-dimethylpiperidine
1-[3-(4-propionylphenoxy)propyl]-4-methylpiperidine
1-[3-(4-propionylphenoxy)propyl]-2-methylpiperidine
1-{3-[4-(1-hydroxypropyl)phenoxy]propyl}-3-methylpiperidine
1-{3-[4-(1-hydroxypropyl)phenoxy]propyl}-4-methylpiperidine
1-[3-(4-propionylphenoxy)propyl]-2-methylpiperidine
1-[3-(4-propionylphenoxy)propyl]-4-methylpiperidine methoxime
1-[3-(4-cyanophenoxy)propyl]-trans-3,5-dimethylpiperidine
1-[3-(4-cyclopropylcarbonylphenoxy) propyl] -trans-3,5-dimethyl piperidine
1-[3-(4-cyclopropylcarbonylphenoxy) propyl] -cis-3,5-dimethyl piperidine
1-[3-(4-carbomethoxyphenoxy)propyl] piperidine
1-[3-(4-propenylphenoxy)propyl]-2-methyl piperidine
1-[3-(4-propionylphenoxy)propyl]-2-methylpiperidine
1-{3-[4-(1-ethoxypropyl)phenoxy]propyl}-2-methyl piperidine
1-[3-(4-propionylphenoxy)propyl]-4-methylpiperidine
1-[3-(4-bromophenoxy)propyl]piperidine
1-[3-(4-nitrophenoxy)propyl]piperidine
1-[3-(4-N,N-dimethylsulfonamidophenoxy) propyl]piperidine
1-[3-(4-isopropylphenoxy)propyl]piperidine
1-[3-(4-sec-butylphenoxy)propyl]piperidine
1-[3-(4-propylphenoxy)propyl]piperidine
1-[3-(4-ethylphenoxy)propyl]piperidine
1-(5-phenoxypentyl)-1,2,3,6-tetrahydropyridine
1-[5-(4-nitrophenoxy)-pentyl]-pyrrolidine
1-[5-(4-chlorophenoxy)-pentyl]-pyrrolidine
1-[5-(4-methoxyphenoxy)-pentyl]-pyrrolidine
1-[5-(4-methylphenoxy)-pentyl]-pyrrolidine
1-[5-(4-cyanophenoxy)-pentyl]-pyrrolidine
1-[5-(2-naphthyloxy)-pentyl]-pyrrolidine
1-[5-(1-naphthyloxy)-pentyl]-pyrrolidine
1-[5-(3-chlorophenoxy)-pentyl]-pyrrolidine
1-[5-(4-phenylphenoxy)-pentyl]-pyrrolidine
1-{5-[2-(5,6,7,8-tetrahydronaphthyl)-oxy]-pentyl}-pyrrolidine
1-[5-(3-phenylphenoxy)-pentyl]-pyrrolidine
1-(5-phenoxypentyl)-2,5-dihydropyrrole
1-{5-[1-(5,6,7,8-tetrahydronaphthyl)-oxy]-pentyl}-pyrrolidine
1-(4-phenoxybutyl)-pyrrolidine
1-(6-phenoxyhexyl)-pyrrolidine
1-(5-phenylthiopentyl)-pyrrolidine
1-(4-phenylthiobutyl)-pyrrolidine
1-(3-phenoxypropyl)-pyrrolidine
1-[5-(3-nitrophenoxy)-pentyl]-pyrrolidine
1-[5-(4-fluorophenoxy)-pentyl]-pyrrolidine
1-[5-(4-nitrophenoxy)-pentyl]-3-methyl-piperidine
1-[5-(4-acetylphenoxy)-pentyl]-pyrrolidine
1-[5-(4-aminophenoxy)-pentyl]-pyrrolidine
1-[5-(3-cyanophenoxy)-pentyl]-pyrrolidine
N-[3-(4-nitrophenoxy)-propyl]-diethylamine
N-[3-(4-cyanophenoxy)-propyl]-diethylamine
1-[5-(4-benzoylphenoxy)-pentyl]-pyrrolidine
1-{5-[4-(phenylacetyl)-phenoxy]-pentyl}-pyrrolidine
N-[3-(4-acetylphenoxy)-propyl]-diethylamine
1-[5-(4-acetamidophenoxy)-pentyl]-pyrrolidine
1-[5-(4-phenoxyphenoxy)-pentyl]-pyrrolidine
1-[5-(4-N-benzamidophenoxy)-pentyl]-pyrrolidine
1-{5-[4-(1-hydroxyethyl)-phenoxy]-pentyl}-pyrrolidine
1-[5-(4-cyanophenoxy)-pentyl]-diethylamine
1-[5-(4-cyanophenoxy)-pentyl]-piperidine
N-[5-(4-cyanophenoxy)-pentyl]-dimethylamine
N-[2-(4-cyanophenoxy)-ethyl]-diethylamine
N-[3-(4-cyanophenoxy)-propyl]-dimethylamine
N-[4-(4-cyanophenoxy)-butyl]-diethylamine
N-[5-(4-cyanophenoxy)-pentyl]-dipropylamine
1-[3-(4-cyanophenoxy)-propyl]-pyrrolidine
1-[3-(4-cyanophenoxy)-propyl]-piperidine
N-[3-(4-cyanophenoxy)-propyl]-hexamethyleneimine
N-[6-(4-cyanophenoxy)-hexyl]-diethylamine
N-[3-(4-cyanophenoxy)-propyl]-dipropylamine
N-3-[4-(1-hydroxyethyl)-phenoxy]-propyl-diethylamine
4-(3-diethylaminopropoxy)-acetophenone-oxime
1-[3-(4-acetylphenoxy)-propyl]-piperidine
1-[3-(4-acetylphenoxy)-propyl]-3-methyl-piperidine
1-[3-(4-acetylphenoxy)-propyl]-3,5-trans-dimethyl-piperidine
1-[3-(4-acetylphenoxy)-propyl]-4-methyl-piperidine
1-[3-(4-propionylphenoxy)-propyl]-piperidine
1-[3-(4-acetylphenoxy)-propyl]-3,5-cis-dimethyl-piperidine
1-[3-(4-formylphenoxy)-propyl]-piperidine
1-[3-(4-isobutyrylphenoxy)-propyl]-piperidine
N-[3-(4-propionylphenoxy)-propyl]-diethylamine
1-[3-(4-butyrylphenoxy)-propyl]-piperidine
1-[3-(4-acetylphenoxy)-propyl]-1,2,3,6-tetrahydropyridine
α-(4-Acetylphenoxy)-α-(4-methylpiperidino)p-xylol
α-(4-Acetylphenoxy)α'-(3,5-cis-dimethylpiperidino)p-xylol
α-(4-Acetylphenoxy)-α-(3,5-trans-dimethylpiperidino)p-xylol
α-(4-Acetylphenoxy)-α'-(2-methylpyrrolidino)p-xylol
α-(4-Cyclopropylcarbonylphenoxy)-α'-piperidino-p-xylol
α-(4-Cyclopropylcarbonylphenoxy)-α'-(4-methylpiperidino)p-xylol
α-(4-Cyclopropylcarbonylphenoxy)-α'-pyrrolidino-p-xylol
3-Phenylpropyl 3-(4-methylpiperidino)propyl ether
3-Phenylpropyl 3-(3,5-cis-dimethylpiperidino)propyl ether
3-Phenylpropyl 3-(3,5-trans-dimethylpiperidino)propyl ether
3-Phenylpropyl 3-(3-methylpiperidino)propyl ether
3-Phenylpropyl 3-pyrrolidinopropyl ether
3-(4-Chlorophenyl)propyl 3-(4-methylpiperidino)propyl ether
3-(4-Chlorophenyl)propyl 3-(3,5-cis-dimethylpiperidino)propyl ether
3-(4-Chlorophenyl)propyl3-(3,5-*trans*-dimethylpiperidino)propyl ether
4-(6-Piperidinohexylamino)quinoline
2-Methyl 4-(3-piperidinopropylamino)quinoline
2-Methyl 4-(6-piperidinohexylamino)quinoline
7-Chloro-4-(3-piperidinopropylamino)quinoline
7-Chloro-4-(4-piperidinobutylamino)quinoline
7-Chloro-4-(8-piperidinooctylamino)quinoline
7-Chloro-4-(10-piperidinodecylamino)quinoline
7-Chloro-4-(12-piperidinododecylamino)quinoline
7-Chloro-4-(4-(3-piperidinopropoxy)phenylamino)quinoline
7-Chloro-4-(2-(4-(3-piperidinopropoxy)phenyl)ethylamino)quinoline
4-(6-Piperidinohexanoyl)phenyl 3-piperidinopropyl ether
5-Nitro-2-(5-piperidinopentylamino)pyridine
3-Nitro-2-(6-piperidinopentylamino)pyridine
5-Amino-2-(6-piperidinopentylamino)pyridine
2-(6-Piperidinohexylamino)quinoline
*N*-(4-Chlorobenzyl)-*N*'-cyclohexyl-3-piperidinopropyl isothiourea
2-(6-Piperidinohexylamino)benzothiazole
10-Piperidinodecylamine
3-Phenylpropyl 3-(*N*,*N*-diethylamino)propyl ether
N-(3-(N,N-Diethylamino)propyl)N'-phenylurea
N-Cyclohexylmethyl-N'-(3-piperidinopropyl)guanidine
N-(4-Bromobenzyl)-N'-(4-piperidinobutyl)sulphamide
3-Chloro-N-(4-piperidinobutyl)-N-methyl-benzene sulphonamide
N-(4-Chlorobenzyl)-2-(4-piperidinomethyl) phenyl) ethan amidine
1-(5-Cyclohexylpentanoyl)-1,4-bipiperidine
cis-1-(6-Cyclohexyl-3-hexen-1-yl)piperidine
trans-1-(6-Cyclohexyl-3-hexen-1-yl)piperidine
1-(2-(5,5-Dimethyl-1-hexin-1-yl)cyclopropyl)piperidine

79. The use according to claim 1, wherein said compound is of formula (Ila): 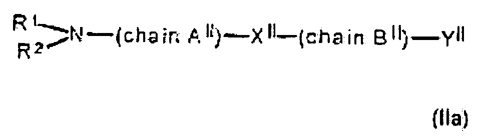 wherein:
R¹ and R² form together with the N atom to which they are attached a saturated nitrogen-containing ring 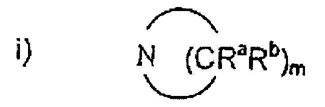
with m ranging from 2 to 8, or
R^{a-b} being independently a hydrogen atom or a linear or branched alkyl group containing 1 to 6 carbon atoms, and
the chain A^{II} selected from an unbranched alkyl group -(CH₂)_{nII}- where n_{II} is 3 ;
the group X" is -O- ;
the chain B^{II} is an unbranched alkyl comprising 3 carbon atoms; and
the group Y^{II} represents a phenyl group, unsubstituted or mono- or polysubstituted with one or more identical or different substituents selected from halogen atoms, OCF₃, CHO, CF₃, SO₂N(alkyl)₂, NO₂, S(aryl), SCH₂(phenyl), an unbranched or branched alkene, an unbranched or branched alkyne optionally substituted with a trialkylsilyl radical, -O(alkyl), -O(aryl), -CH₂CN, a ketone, an aldehyde, a sulphone, an acetal, an alcohol, a linear or branched alkyl group containing 1 to 6 carbon atoms, -CH=CH-CHO, -C(alkyl)=N-OH, -C(alkyl)=N-O(alkyl), -CH=NOH, -CH=NO(alkyl), , -C(alkyl)=NH-NH-CONH₂, an O-phenyl or -OCH₂(phenyl) group, -C(cycloalkyl)=NOH, -C(cycloalkyl)=N-O(alkyl);
or its pharmaceutically acceptable salts, hydrates, or hydrated salts, or the polymorphic crystalline structures of these compounds or their optical isomers, racemates, diastereoisomers or enantiomers.

80. The use according to claim 79 wherein -NR¹R² is a saturated nitrogen-containing ring: 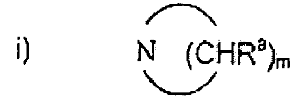 R^{a} and m being as defined in claim 79.

81. The use according to claim 79 or 80, wherein m is 4 or 5.

82. The use according to anyone of claims 79 to 81, wherein -NR¹R² is selected from the group consisting in piperidyl, pyrrolidinyl.

83. The use according to anyone of claims 79 to 82, wherein R^{a} is a hydrogen atom.

84. The use according to anyone of claims 79 to 83, wherein the nitrogen-containing ring i) is one of mono- and di-substituted.

85. The use according to anyone of claims 79 to 84, wherein the nitrogen-containing ring i) is mono-substituted with an alkyl group.

86. The use according to anyone of claims 79 to 85, wherein the nitrogen-containing ring is mono-substituted with a methyl group.

87. The use according to anyone of claims 79 to 88, wherein the substituent(s) is(are) in beta-position with respect to the nitrogen atom.

88. The use according to anyone of claims 79 to 87, wherein Y" represents a phenyl group at least mono-substituted with a halogen atom, keto-substituent which may include a linear or branched chain aliphatic ketone comprising from 1 to 8 carbon atoms and optionally bearing a hydroxyl group, a cycloalkylketone, an arylalkylketone or arylalkenylketone in which the aryl group is optionally substituted, or a heteroaryl ketone.

89. The use according to anyone of claims 79 to 88, wherein Y^{II} is a phenyl group at least mono-substituted with a halogen atom, -CHO, a ketone, an aldehyde, -CH=CH-CHO, -C(alkyl)=N-OH, -C(alkyl)=N-O(alkyl), -CH=N-OH, -CH=NO(alkyl), -C(cycloalkyl)=NOH, -C(cycloalkyl)=N-O(alkyl).

90. The use according to anyone of claims 79 to 89, wherein the compound is selected from:
- 3-Phenylpropyl 3-piperidinopropyl ether
- 3-(4-Chlorophenyl)propyl 3-piperidinopropyl ether
- 3-Phenylpropyl 3-(4-methylpiperidino)propyl ether
- 3-Phenylpropyl 3-(3,5-cis-dimethylpiperidino)propyl ether
- 3-Phenylpropyl 3-(3,5-trans-dimethylpiperidino)propyl ether
- 3-Phenylpropyl 3-(3-methylpiperidino)propyl ether
- 3-Phenylpropyl 3-pyrrolidinopropyl ether
- 3-(4-Chlorophenyl)propyl 3-(4-methylpiperidino)propyl ether
- 3-(4-Chlorophenyl) propyl 3-(3,5-cis-dimethyl piperidino)propyl ether
- 3-(4-Chlorophenyl) propyl 3-(3,5-trans-dimethyl piperidino)propyl ether.
or its pharmaceutically acceptable salts, hydrates, or hydrated salts, or the polymorphic crystalline structures of these compounds or their optical isomers, racemates, diastereoisomers or enantiomers.

91. The use according to anyone of claims 79 to 90, wherein the compound is selected from 3-(4-chlorophenyl)propyl-3-piperidinopropylether, or its pharmaceutically acceptable salts, hydrates, or hydrated salts, or the polymorphic crystalline structures of this compound or its optical isomers, racemates, diastereoisomers or enantiomers.

92. The use according to anyone of claims 79 to 91 wherein the compound is in the form of a pharmaceutically acceptable salt and said salt is chosen from the group consisting in hydrochloride, hydrobromide, hydrogen maleate or hydrogen oxalate.

93. The use according to anyone of the preceding claims, wherein the epilepsy is selected from the group consisting of absence epilepsy, pharmaco-resistant temporal lobe seizures, and photosensitive seizures.

94. Use of ligand of the H3 receptor for the preparation of a medicament intended for the treatment of absence epilepsy, pharmaco-resistant temporal lobe seizures, and photosensitive seizures.

95. Combination comprising an anti-epileptic drug and a ligand of the H3 receptor.

96. Use of a ligand of the H3 receptor for the preparation of a medicament for treating and/or preventing epilepsy in combination with an anti-epileptic drug.
